# EUROPEAN PATENT APPLICATION

(11) **EP 1 494 031 A2**
(43) Date of publication of application: **05.01.2005**
(21) Application number: 04253880.1
(22) Date of filing: 29.06.2004
(51) Int. Cl.: G01N 33/574, C12Q 1/68

(54) **Novel human genes and gene expression products**

(30) Priority: 30.06.2003 US 610049
(71) Applicant: Bayer Healthcare LLC, Tarrytown, New York 10591-5097 (US); MAYO FOUNDATION FOR MEDICAL EDUCATION AND RESEARCH, Rochester, MN 55905 (US)
(72) Inventor: Astle, Jon H., Taunton, MA 02780 (US); Thiagalingam, Arunthathi, Lexington, MA 02420 (US); Myerow, Susan H., Lexington, MA 02421 (US); Boardman, Lisa Allyn, Rochester, MN 55902 (US); Burgart, Lawrence J., Rochester, MN 55901 (US); Thibodeau, Stephen N., Rochester, MN 55906 (US); Burgess, Christopher C., Westwood, MA 02090 (US); Catino, Theodore J., Attleboro, MA 02702 (US); Dwivedi, Poornima, Alamo, CA 94507 (US); Molino, Gary A., Norfold, MA 02056 (US); Lewis, Marcia E., Cohasset, MA 02025 (US)
(74) Representative: Grant, Anne Rosemary

(57) **Abstract**

This invention relates to novel human genes, to proteins expressed by the genes, and to variants of the proteins. The invention also relates to diagnostic assays and therapeutic agents related to the genes and proteins, including probes, antisense constructs, and antibodies. The subject nucleic acids have been found to be differentially regulated in tumor cells, particularly in colon cancer tissue.

SEQUENCE LISTING

<110> Bayer Healthcare LLC
Mayo Foundation for Medical Education and Research

<120> Novel Human Genes and Gene Expression Products

<130> 28.14.84781

<140>

<150> US 10/610,049
<151> 2003-06-30

<160> 26

<170> PatentIn version 3.2

<210> 1
<211> 619
<212> DNA
<213> Homo sapiens

<220>
<221> misc_feature
<222> (492)..(492)
<223> n indicates that the identity of the corresponding nucleotide is unknown

<220>
<221> misc_feature
<222> (592)..(592)
<223> n indicates that the identity of the corresponding nucleotide is unknown

<220>
<221> misc_feature
<222> (594)..(594)
<223> n indicates that the identity of the corresponding nucleotide is unknown

<220>
<221> misc_feature
<222> (601)..(601)
<223> n indicates that the identity of the corresponding nucleotide is unknown

<220>
<221> misc_feature
<222> (604)..(604)
<223> n indicates that the identity of the corresponding nucleotide is unknown

<220>
<221> misc_feature

<222> (615)..(615)
<223> n indicates that the identity of the corresponding nucleotide is unknown

<220>
<221> misc_feature
<222> (619)..(619)
<223> n indicates that the identity of the corresponding nucleotide is unknown

<400> 1
accatgaaat atccagaaca tacttatatg taaagtatta tttatttgaa tccacaaaaa 60

acaacaaata atttttaaat ataaggattt tcctagatat tgcacgggag aatatacaaa 120

tagcaaaatt gaggccaagg gccaagagaa tatccgaact ttaatttcag gaattgaatg 180

ggtttgctag aatgtgatat ttgaagcatc acataaaaat gatgggacaa taaattttgc 240

cataaagtca aatttagctg gaaatcctgg atttttttct gttaaatctg gcaaccctag 300

tctgctagcc aggatccaca agtccttgtt ccactgtgcc ttggtttctc ctttatttct 360

aagtggaaaa agtattagcc accatcttac ctcacagtga tgttgtgagg acatgtggaa 420

gcactttaag ttttttcatc ataacataaa ttattttcaa gtgtaactta ttaacctatt 480

tattatttat gnatttattt aagcatcaaa tatttgtgca agaatttgga aaaatagaag 540

atgaatcatt gattgaatag tattaagatg tatagtaaat tatttatttt ananattaaa 600

ngangtttat taganaaan 619

<210> 2
<211> 633
<212> DNA
<213> Homo sapiens

<220>
<221> misc_feature
<222> (580)..(580)
<223> n indicates that the identity of the corresponding nucleotide is unknown

<220>
<221> misc_feature
<222> (617)..(617)
<223> n indicates that the identity of the corresponding nucleotide is unknown

<400> 2
ggtaccaatg gtgcctcctg gaatcaagta tctttacctt aggaataacc agattgacca 60

tattgatgaa aaggcctttg agaatgtaac tgatctgcag tggctcattc tagatcacaa 120

ccttctagaa aactccaaga taaaagggag agttttctct aaattgaaac aactgaagaa    180

gctgcatata aaccacaaca acctgacaga gtctgtgggc ccacttccca aatctctgga    240

ggatctgcag cttactcata acaagatcac aaagctgggc tcttttgaag gattggtaaa    300

cctgaccttc atccatctcc agcacaatcg gctgaaagag gatgctgttt cagctgcttt    360

taaaggtctt aaatcactcg aataccttga cttgagcttc aatcagatag ccagactgcc    420

ttctggtctc cctgtctctc ttctaactct ctacttagac aacaataaga tcagcaacat    480

ccctgatgaa gtatttcaag cgtttaatgc tttgcagtat ctgcgtttat ctcacaacga    540

actggctgat agtggaatac ctggaaattc tttcaatggn gccatcctgg gtgaacctgg    600

acttgcctat accagcntaa aacataccac cgg                                633

<210>  3
<211>  570
<212>  DNA
<213>  Homo sapiens

<220>
<221>  misc_feature
<222>  (372)..(372)
<223>  n indicates that the identity of the corresponding nucleotide is unknown

<220>
<221>  misc_feature
<222>  (382)..(382)
<223>  n indicates that the identity of the corresponding nucleotide is unknown

<220>
<221>  misc_feature
<222>  (405)..(406)
<223>  n indicates that the identity of the corresponding nucleotide is unknown

<220>
<221>  misc_feature
<222>  (408)..(409)
<223>  n indicates that the identity of the corresponding nucleotide is unknown

<220>
<221>  misc_feature
<222>  (417)..(417)
<223>  n indicates that the identity of the corresponding nucleotide is unknown

<220>

<221> misc_feature
<222> (428)..(428)
<223> n indicates that the identity of the corresponding nucleotide is unknown

<220>
<221> misc_feature
<222> (471)..(471)
<223> n indicates that the identity of the corresponding nucleotide is unknown

<220>
<221> misc_feature
<222> (475)..(475)
<223> n indicates that the identity of the corresponding nucleotide is unknown

<220>
<221> misc_feature
<222> (486)..(487)
<223> n indicates that the identity of the corresponding nucleotide is unknown

<220>
<221> misc_feature
<222> (490)..(490)
<223> n indicates that the identity of the corresponding nucleotide is unknown

<220>
<221> misc_feature
<222> (492)..(492)
<223> n indicates that the identity of the corresponding nucleotide is unknown

<220>
<221> misc_feature
<222> (497)..(497)
<223> n indicates that the identity of the corresponding nucleotide is unknown

<220>
<221> misc_feature
<222> (501)..(501)
<223> n indicates that the identity of the corresponding nucleotide is unknown

<220>
<221> misc_feature
<222> (506)..(506)
<223> n indicates that the identity of the corresponding nucleotide is unknown

<220>
<221> misc_feature
<222> (511)..(511)
<223> n indicates that the identity of the corresponding nucleotide is unknown

<220>
<221> misc_feature
<222> (518)..(518)

<223> n indicates that the identity of the corresponding nucleotide is unknown

<220>
<221> misc_feature
<222> (522)..(522)
<223> n indicates that the identity of the corresponding nucleotide is unknown

<220>
<221> misc_feature
<222> (527)..(527)
<223> n indicates that the identity of the corresponding nucleotide is unknown

<220>
<221> misc_feature
<222> (544)..(544)
<223> n indicates that the identity of the corresponding nucleotide is unknown

<220>
<221> misc_feature
<222> (556)..(556)
<223> n indicates that the identity of the corresponding nucleotide is unknown

<220>
<221> misc_feature
<222> (562)..(562)
<223> n indicates that the identity of the corresponding nucleotide is unknown

<220>
<221> misc_feature
<222> (565)..(565)
<223> n indicates that the identity of the corresponding nucleotide is unknown

<400> 3
ggtacttcta cacatctgcc taacttggga atgaatgtgg gagaaaatcg ctgctgctga 60

gatggactcc agaagaagaa actgtttctc caggcgactt tgaacccatt ttttggcagt 120

gttcatatta ttaaactagt caaaaatgct aaaataattt gggagaaaat attttttaag 180

tagtgttata gtttcatgtt tatcttttat tatgttttgt gaagttgtgt cttttcacta 240

attacctata ctatgccaat atttccttat atctatccat aacatttata ctacatttgt 300

aagagaatat gcacgtgaaa cttaacactt tataaggtaa aaatgaggtt tccaagattt 360

aataatctga tncagttctt gntatttccc aatagaatgg gactnngnnc tgttaanggc 420

ttaagganaa agggaagata agggttaaaa gttggttaat ggacccaacc ntttnaaaga 480

aatgcnntan anaatanttt natgantaaa naaaggtncc tngcccnggc cggccgtttt 540

aaangggcca atttcnagca cnctnggcgg 570

<210> 4
<211> 599
<212> DNA
<213> Homo sapiens

<220>
<221> misc_feature
<222> (292)..(292)
<223> n indicates that the identity of the corresponding nucleotide is unknown

<220>
<221> misc_feature
<222> (387)..(387)
<223> n indicates that the identity of the corresponding nucleotide is unknown

<220>
<221> misc_feature
<222> (453)..(453)
<223> n indicates that the identity of the corresponding nucleotide is unknown

<220>
<221> misc_feature
<222> (464)..(464)
<223> n indicates that the identity of the corresponding nucleotide is unknown

<220>
<221> misc_feature
<222> (476)..(476)
<223> n indicates that the identity of the corresponding nucleotide is unknown

<220>
<221> misc_feature
<222> (499)..(499)
<223> n indicates that the identity of the corresponding nucleotide is unknown

<220>
<221> misc_feature
<222> (510)..(510)
<223> n indicates that the identity of the corresponding nucleotide is unknown

<220>
<221> misc_feature
<222> (512)..(512)
<223> n indicates that the identity of the corresponding nucleotide is unknown

<220>
<221> misc_feature
<222> (532)..(532)
<223> n indicates that the identity of the corresponding nucleotide is unknown

<220>
<221> misc_feature
<222> (545)..(545)
<223> n indicates that the identity of the corresponding nucleotide is unknown

<220>
<221> misc_feature
<222> (559)..(559)
<223> n indicates that the identity of the corresponding nucleotide is unknown

<220>
<221> misc_feature
<222> (577)..(577)
<223> n indicates that the identity of the corresponding nucleotide is unknown

<400> 4
cgaggtactt tttccgcaca tgccttgtgc ctatctgagt attgatgcca tggatgtggc 60

cggagaacag cagctggatg tggaacacaa cctgttcaag caacgactag ataaagatgg 120

catccccgtg agctcagagg ctgagcggca tgagcttggg aaagtcgagg tgacggtgtt 180

tgaccctgac tccctggacc ctgatcgctg tgagagctgc tatggtgctg aggcagaaga 240

tatcaagtgc tgtaacacct gtgaagatgt gcgggaggca tatcgcccgt anaagctggg 300

ccttcaagaa cccagatact attgagcagt gccggcgaag agggcttcag ccagaagatg 360

caggaaccag aagaatgaag ctgccangtg tatggctttc ttggaaagtc aaataaggtg 420

gcccgaaact ttcactttgc ccttggggaa ganctttcca gcantcccat gtcacntcat 480

tgacttggca aactttggnc ttgacaaccn tnaccatgac ccactacatc ancacctgtc 540

atttngggga ggactttcna gccttgggaa acccctngac cccccaatgg taattggcc 599


<210> 5
<211> 563
<212> DNA
<213> Homo sapiens


<220>
<221> misc_feature
<222> (414)..(414)
<223> n indicates that the identity of the corresponding nucleotide is unknown

<220>
<221> misc_feature
<222> (469)..(469)
<223> n indicates that the identity of the corresponding nucleotide is unknown

<220>
<221> misc_feature
<222> (489)..(489)
<223> n indicates that the identity of the corresponding nucleotide is unknown

<220>
<221> misc_feature
<222> (517)..(517)
<223> n indicates that the identity of the corresponding nucleotide is unknown

<220>
<221> misc_feature
<222> (555)..(555)
<223> n indicates that the identity of the corresponding nucleotide is unknown

<400> 5
ggtacagctg tctgcattga aaattcatgc atggagaaag ggagtaagca agggagaaac 60

ggtgcgattc acatattccg cgagatcatc aagccagcag agaaatccct ccatgaaaag 120

ttaaaacaag ataagcgctt tagcaccttc ctcagcctac ttgaagctgc agacttgaaa 180

gagctcctga cacaacctgg agactggaca ttatttgtgc caaccaatga tgcttttaag 240

ggaatgacta gtgaagaaaa agaaattctg atcgggacaa aaatgctctt caaaacatca 300

ttctttatca cctgacacca ggagttttca ttggaaaagg atttgaacct ggtgttacta 360

acattttaaa gaccacacaa ggaaacaaaa tcttttcttg aaagaaagta aatngatcca 420

cttctggtga atgaatttga aattcaaagg aatctggcct tcatgccanc aaatgggggt 480

aattcatgnt ggagaataac ctcctttatc cagccgnaca cacctgttgg aaatggatcc 540

aactgctgga aattncttaa taa 563

<210> 6
<211> 629
<212> DNA
<213> Homo sapiens

<220>
<221> misc_feature
<222> (82)..(82)
<223> n indicates that the identity of the corresponding nucleotide is unknown

<220>
<221> misc_feature
<222> (98)..(98)

<223> n indicates that the identity of the corresponding nucleotide is unknown

<220>
<221> misc_feature
<222> (105)..(105)
<223> n indicates that the identity of the corresponding nucleotide is unknown

<220>
<221> misc_feature
<222> (140)..(140)
<223> n indicates that the identity of the corresponding nucleotide is unknown

<220>
<221> misc_feature
<222> (180)..(180)
<223> n indicates that the identity of the corresponding nucleotide is unknown

<220>
<221> misc_feature
<222> (189)..(189)
<223> n indicates that the identity of the corresponding nucleotide is unknown

<220>
<221> misc_feature
<222> (192)..(192)
<223> n indicates that the identity of the corresponding nucleotide is unknown

<220>
<221> misc_feature
<222> (201)..(201)
<223> n indicates that the identity of the corresponding nucleotide is unknown

<220>
<221> misc_feature
<222> (257)..(257)
<223> n indicates that the identity of the corresponding nucleotide is unknown

<220>
<221> misc_feature
<222> (366)..(366)
<223> n indicates that the identity of the corresponding nucleotide is unknown

<220>
<221> misc_feature
<222> (384)..(384)
<223> n indicates that the identity of the corresponding nucleotide is unknown

<220>
<221> misc_feature
<222> (390)..(390)
<223> n indicates that the identity of the corresponding nucleotide is unknown

<220>
<221> misc_feature
<222> (396)..(396)
<223> n indicates that the identity of the corresponding nucleotide is unknown

<220>
<221> misc_feature
<222> (405)..(405)
<223> n indicates that the identity of the corresponding nucleotide is unknown

<220>
<221> misc_feature
<222> (409)..(409)
<223> n indicates that the identity of the corresponding nucleotide is unknown

<220>
<221> misc_feature
<222> (413)..(413)
<223> n indicates that the identity of the corresponding nucleotide is unknown

<220>
<221> misc_feature
<222> (432)..(432)
<223> n indicates that the identity of the corresponding nucleotide is unknown

<220>
<221> misc_feature
<222> (436)..(436)
<223> n indicates that the identity of the corresponding nucleotide is unknown

<220>
<221> misc_feature
<222> (441)..(441)
<223> n indicates that the identity of the corresponding nucleotide is unknown

<220>
<221> misc_feature
<222> (450)..(451)
<223> n indicates that the identity of the corresponding nucleotide is unknown

<220>
<221> misc_feature
<222> (453)..(453)
<223> n indicates that the identity of the corresponding nucleotide is unknown

<220>
<221> misc_feature
<222> (456)..(457)
<223> n indicates that the identity of the corresponding nucleotide is unknown

<220>
<221> misc_feature

<222> (460)..(460)
<223> n indicates that the identity of the corresponding nucleotide is unknown

<220>
<221> misc_feature
<222> (464)..(465)
<223> n indicates that the identity of the corresponding nucleotide is unknown

<220>
<221> misc_feature
<222> (470)..(470)
<223> n indicates that the identity of the corresponding nucleotide is unknown

<220>
<221> misc_feature
<222> (472)..(473)
<223> n indicates that the identity of the corresponding nucleotide is unknown

<220>
<221> misc_feature
<222> (486)..(486)
<223> n indicates that the identity of the corresponding nucleotide is unknown

<220>
<221> misc_feature
<222> (490)..(490)
<223> n indicates that the identity of the corresponding nucleotide is unknown

<220>
<221> misc_feature
<222> (492)..(492)
<223> n indicates that the identity of the corresponding nucleotide is unknown

<220>
<221> misc_feature
<222> (500)..(502)
<223> n indicates that the identity of the corresponding nucleotide is unknown

<220>
<221> misc_feature
<222> (504)..(504)
<223> n indicates that the identity of the corresponding nucleotide is unknown

<220>
<221> misc_feature
<222> (511)..(511)
<223> n indicates that the identity of the corresponding nucleotide is unknown

<220>
<221> misc_feature
<222> (530)..(530)
<223> n indicates that the identity of the corresponding nucleotide is unknown

<220>
<221> misc_feature
<222> (538)..(538)
<223> n indicates that the identity of the corresponding nucleotide is unknown

<220>
<221> misc_feature
<222> (545)..(547)
<223> n indicates that the identity of the corresponding nucleotide is unknown

<220>
<221> misc_feature
<222> (554)..(555)
<223> n indicates that the identity of the corresponding nucleotide is unknown

<220>
<221> misc_feature
<222> (560)..(560)
<223> n indicates that the identity of the corresponding nucleotide is unknown

<220>
<221> misc_feature
<222> (573)..(573)
<223> n indicates that the identity of the corresponding nucleotide is unknown

<220>
<221> misc_feature
<222> (578)..(579)
<223> n indicates that the identity of the corresponding nucleotide is unknown

<220>
<221> misc_feature
<222> (585)..(586)
<223> n indicates that the identity of the corresponding nucleotide is unknown

<220>
<221> misc_feature
<222> (589)..(589)
<223> n indicates that the identity of the corresponding nucleotide is unknown

<220>
<221> misc_feature
<222> (603)..(603)
<223> n indicates that the identity of the corresponding nucleotide is unknown

<220>
<221> misc_feature
<222> (611)..(611)
<223> n indicates that the identity of the corresponding nucleotide is unknown

<220>

<221> misc_feature
<222> (619)..(619)
<223> n indicates that the identity of the corresponding nucleotide is unknown

<220>
<221> misc_feature
<222> (626)..(626)
<223> n indicates that the identity of the corresponding nucleotide is unknown

<400> 6
actttttttt tttttttttt ttttttgttt tttttttttt tttttttttt ttttttttgc 60

agtttctaag tcattacttt tnattttgaa agatttgnga aactnttcac atcatggtga 120

gagtttgtat gattaataan aagcagcttt ttcatgaaat gcttggaggt gaacgagttn 180

tcagcctgng anatccgacc ntcccattaa ctttgaagtt tctcttgatt aatagaagaa 240

aaaaggggag ggtgaanaaa aggaggaaca tgctaaaaac cttatgacaa tcatccaaat 300

gtgaggaaag aacaacccga ttcaccaact ccactttttc tattttacaa ctttctacat 360

ctcacncttg gattttggcc ttcntggctn aaacantcct ggcantccnt tanagcccct 420

gaaaaagagc cntggntttt ncaaaagacn ntnggnnggn gaannccttn annatgccct 480

gacccnttcn cnaagaactn nntntccggg ntcccaaaag tttgacccan cagcttantg 540

tgaannnaaa actnnccttn aaaggtaatg ggnggaanng gtgannaant gggttttttt 600

ganaagtctt ntttttctna aaaccnccg 629

<210> 7
<211> 607
<212> DNA
<213> Homo sapiens

<220>
<221> misc_feature
<222> (354)..(354)
<223> n indicates that the identity of the corresponding nucleotide is unknown

<220>
<221> misc_feature
<222> (378)..(378)
<223> n indicates that the identity of the corresponding nucleotide is unknown

<220>
<221> misc_feature
<222> (456)..(456)

<223> n indicates that the identity of the corresponding nucleotide is unknown

<220>
<221> misc_feature
<222> (487)..(487)
<223> n indicates that the identity of the corresponding nucleotide is unknown

<220>
<221> misc_feature
<222> (496)..(496)
<223> n indicates that the identity of the corresponding nucleotide is unknown

<220>
<221> misc_feature
<222> (514)..(514)
<223> n indicates that the identity of the corresponding nucleotide is unknown

<220>
<221> misc_feature
<222> (520)..(520)
<223> n indicates that the identity of the corresponding nucleotide is unknown

<220>
<221> misc_feature
<222> (525)..(525)
<223> n indicates that the identity of the corresponding nucleotide is unknown

<220>
<221> misc_feature
<222> (543)..(543)
<223> n indicates that the identity of the corresponding nucleotide is unknown

<220>
<221> misc_feature
<222> (551)..(551)
<223> n indicates that the identity of the corresponding nucleotide is unknown

<220>
<221> misc_feature
<222> (559)..(559)
<223> n indicates that the identity of the corresponding nucleotide is unknown

<220>
<221> misc_feature
<222> (572)..(572)
<223> n indicates that the identity of the corresponding nucleotide is unknown

<220>
<221> misc_feature
<222> (582)..(582)
<223> n indicates that the identity of the corresponding nucleotide is unknown

<220>
<221> misc_feature
<222> (589)..(589)
<223> n indicates that the identity of the corresponding nucleotide is unknown

<220>
<221> misc_feature
<222> (591)..(591)
<223> n indicates that the identity of the corresponding nucleotide is unknown

<220>
<221> misc_feature
<222> (601)..(602)
<223> n indicates that the identity of the corresponding nucleotide is unknown

<220>
<221> misc_feature
<222> (604)..(604)
<223> n indicates that the identity of the corresponding nucleotide is unknown

<220>
<221> misc_feature
<222> (607)..(607)
<223> n indicates that the identity of the corresponding nucleotide is unknown

<400> 7
acattaaagt gtgatacttg gttttgaaaa cattcaaaca gtctctgtgg aaatctgaga 60

gaaattggcg gagagctgcc gtggtgcatt cctcctgtag tgcttcaagc taatgcttca 120

tcctctctaa taacttttga tagacagggg ctagtcgcac agacctctgg gaagccctgg 180

aaaacgctga tgcttgtttg aagatctcaa gcgcagagtc tgcaagttca tcccctcttt 240

cctgaggtct gttggctgga ggctgcagaa cattggtgat gacatggacc acgccatttg 300

tggccatgat gtcaggctcg gcaacaggct ccttggtgac actcaccaca ttgnttttca 360

agctgacttt cagcttgnca ccttggagag actttaaccc ggaccaaggg cccgatgcct 420

tccgttaccc aggaatttca tcaccaatgg tggtanttca ggaatgttgg caagtttcct 480

tggcatnttc ccaaanagtt tgttcccgtt cttnttgggn ggcangggct tcggaaaggg 540

ttnattttgt ngggaaccna aaaactgggg tnaaactcct tnccggttna ngggtttccg 600

nnanccn 607

<210> 8
<211> 622
<212> DNA

<213> Homo sapiens

<220>
<221> misc_feature
<222> (576)..(576)
<223> n indicates that the identity of the corresponding nucleotide is unknown

<220>
<221> misc_feature
<222> (612)..(612)
<223> n indicates that the identity of the corresponding nucleotide is unknown

<220>
<221> misc_feature
<222> (617)..(617)
<223> n indicates that the identity of the corresponding nucleotide is unknown

<400> 8
acaatttaat ttttctgctt gcccaagaaa caaagcttct gtggaaccat ggaagaagat 60

gaaaatgaga ctggcaaaga acaaatgctg aatctgaaga agaggacaac tttgggcaaa 120

taatctgcat acttttaatt gggaataaga tggaaaatat gaatgctaaa tcaaattttt 180

taaaaaatac accacacgat acaactcaat acaggagtat ttcttctcaa attcttctag 240

caccatcaac attcttcaag tatctgaaat actattaatt aagcaccttt gtattatgaa 300

caaaacaaaa caaggacctc agttcatctc tgtctaggtc agcacctaac aatgtggatc 360

acactcatgg gaaagtgttt tgaggtagtt taaacctttt ggaaggttgg gttttaaact 420

tccctctgtg gaagatatca aaagccccaa gtggtgccaa atggttatgg ttttattttt 480

caattttaat ttgggtttct tccaaaggtg acatttccat acaaggggaa gggggtggaa 540

aaaaaatcaa attttggggg accagggagg ataatnaact gtttgcaatg cttgacaacc 600

tttttttttt gnccaantaa ca 622

<210> 9
<211> 1639
<212> DNA
<213> Homo sapiens

<400> 9
agcagagcac acaagcttct aggacaagag ccaggaagaa [?]ccaccggaa ggaaccatct 60

cactgtgtgt aaacatgact tccaagctgg ccgtggctct cttggcagcc ttcctgattt 120

ctgcagctct gtgtgaaggt gcagttttgc caaggagtgc taaagaactt agatgtcagt 180

gcataaagac atactccaaa cctttccacc ccaaatttat caaagaactg agagtgattg 240

agagtggacc acactgcgcc aacacagaaa ttattgtaaa gctttctgat ggaagagagc 300

tctgtctgga ccccaaggaa aactgggtgc agagggttgt ggagaagttt ttgaagaggg 360

ctgagaattc ataaaaaaat tcattctctg tggtatccaa gaatcagtga agatgccagt 420

gaaacttcaa gcaaatctac ttcaacactt catgtattgt gtgggtctgt tgtagggttg 480

ccagatgcaa tacaagattc ctggttaaat ttgaatttca gtaaacaatg aatagttttt 540

cattgtacca tgaaatatcc agaacatact tatatgtaaa gtattattta tttgaatcta 600

caaaaaacaa caaataattt ttaaatataa ggattttcct agatattgca cgggagaata 660

tacaaatagc aaaattgagc caagggccaa gagaatatcc gaactttaat ttcaggaatt 720

gaatgggttt gctagaatgt gatatttgaa gcatcacata aaaatgatgg gacaataaat 780

tttgccataa agtcaaattt agctggaaat cctggatttt tttctgttaa atctggcaac 840

cctagtctgc tagccaggat ccacaagtcc ttgttccact gtgccttggt ttctccttta 900

tttctaagtg gaaaaagtat tagccaccat cttacctcac agtgatgttg tgaggacatg 960

tggaagcact ttaagttttt tcatcataac ataaattatt ttcaagtgta acttattaac 1020

ctatttatta tttatgtatt tatttaagca tcaaatattt gtgcaagaat ttggaaaaat 1080

agaagatgaa tcattgattg aatagttata aagatgttat agtaaattta ttttatttta 1140

gatattaaat gatgttttat tagataaatt tcaatcaggg tttttagatt aaacaaagaa 1200

acaattgggt acccagttaa attttcattt cagataaaca acaaataatt ttttagtata 1260

agtacattat tgtttatctg aaagttttaa ttgaactaac aatcctagtt tgatactccc 1320

agtcttgtca ttgccagctg tgttggtagt gctgtgttga attacggaat aatgagttag 1380

aactattaaa acagccaaaa ctccacagtc aatattagta atttcttgct ggttgaaact 1440

tgtttattat gtacaaatag attcttataa tattatttaa atgactgcat ttttaaatac 1500

aaggctttat atttttaact ttaagatgtt tttatgtgct ctccaaattt tttttactgt 1560

ttctgattgt atggaaatat aaaagtaaat atgaaacatt taaaatataa tttgttgtca 1620

aagtaaaaaa aaaaaaaaa 1639

<210> 10
<211> 1804
<212> DNA
<213> Homo sapiens

<400> 10
gtatcactca gaatctggca gccagttccg tcctgacaga gttcacagca tatattggtg 60

gattcttgtc catagtgcat ctgctttaag aattaacgaa agcagtgtca agacagtaag 120

gattcaaacc atttgccaaa aatgagtcta agtgcattta ctctcttcct ggcattgatt 180

ggtggtacca gtggccagta ctatgattat gattttcccc tatcaattta tgggcaatca 240

tcaccaaact gtgcaccaga atgtaactgc cctgaaagct acccaagtgc catgtactgt 300

gatgagctga aattgaaaag tgtaccaatg gtgcctcctg gaatcaagta tctttacctt 360

aggaataacc agattgacca tattgatgaa aaggcctttg agaatgtaac tgatctgcag 420

tggctcattc tagatcacaa ccttctagaa aactccaaga taaaagggag agttttctct 480

aaattgaaac aactgaagaa gctgcatata aaccacaaca acctgacaga gtctgtgggc 540

ccacttccca aatctctgga ggatctgcag cttactcata acaagatcac aaagctgggc 600

tcttttgaag gattggtaaa cctgaccttc atccatctcc agcacaatcg gctgaaagag 660

gatgctgttt cagctgcttt taaaggtctt aaatcactcg aataccttga cttgagcttc 720

aatcagatag ccagactgcc ttctggtctc cctgtctctc ttctaactct ctacttagac 780

aacaataaga tcagcaacat ccctgatgag tatttcaagc gttttaatgc attgcagtat 840

ctgcgtttat ctcacaacga actggctgat agtggaatac ctggaaattc tttcaatgtg 900

tcatccctgg ttgagctgga tctgtcctat aacaagctta aaaacatacc aactgtcaat 960

gaaaaccttg aaaactatta cctggaggtc aatcaacttg agaagtttga cataaagagc 1020

ttctgcaaga tcctggggcc attatcctac tccaagatca agcatttgcg tttggatggc 1080

aatcgcatct cagaaaccag tcttccaccg gatatgtatg aatgtctacg tgttgctaac 1140

gaagtcactc ttaattaata tctgtatcct ggaacaatat tttatggtta tgtttttctg 1200

tgtgtcagtt ttcatagtat ccatatttta ttactgttta ttacttccat gaattttaaa 1260

atctgaggga aatgttttgt aaacatttat tttttttaaa gaaaagatga aaggcaggcc 1320

tatttcatca caagaacaca cacatataca cgaatagaca tcaaactcaa tgctttattt 1380

gtaaatttag tgttttttta tttctactgt caaatgatgt gcaaaacctt ttactggttg 1440

catggaaatc agccaagttt tataatcctt aaatcttaat gttcctcaaa gcttggatta 1500

aatacatatg gatgttactc tcttgcacca aattatcttg atacattcaa atttgtctgg 1560

ttaaaaaata ggtggtagat attgaggcca agaatattgc aaaatacatg aagcttcatg 1620

cacttaaaga agtattttta gaataagaat ttgcatactt acctagtgaa acttttctag 1680

aattattttt cactctaagt catgtatgtt tctctttgat tatttgcatg ttatgtttaa 1740

taagctacta gcaaaataaa acatagcaaa tgaaaaaaaa aaaaaaaaaa aaaaaaaaaa 1800

aaaa 1804

<210> 11
<211> 1098
<212> DNA
<213> Homo sapiens

<400> 11
cggcacgagg gtcccgcgcg ctcctccgac ccgctccgct ccgctccgct cggccccgcg 60

ccgcccgtca acatgatccg ctgcggcctg gcctgcgagc gctgccgctg gatcctgccc 120

ctgctcctac tcagcgccat cgccttcgac atcatcgcgc tggccggccg cggctggttg 180

cagtctagcg accacggcca gacgtcctcg ctgtggtgga aatgctccca agagggcggc 240

ggcagcgggt cctacgagga gggctgtcag agcctcatgg agtacgcgtg gggtagagca 300

gcggctgcca tgctcttctg tggcttcatc atcctggtga tctgtttcat cctctccttc 360

ttcgccctct gtggacccca gatgcttgtc ttcctgagag tgattggagg tctccttgcc 420

ttggctgctg tgttccagat catctccctg gtaatttacc ccgtgaagta cacccagacc 480

ttcacccttc atgccaaccc tgctgtcact tacatctata actgggccta cggctttggg 540

tgggcagcca cgattatcct gattggctgt gccttcttct tctgctgcct cctcaactac 600

gaagatgacc ttctgggcaa tgccaagcccaggtacttct acacatctgc ctaacttggg 660

aatgaatgtg ggagaaaatc gctgctgctg agatggactc cagaagaaga aactgtttct 720

ccaggcgact ttgaacccat tttttggcag tgttcatatt attaaactag tcaaaaatgc 780

taaaataatt tgggagaaaa tattttttaa gtagtgttat agtttcatgt ttatctttta 840

ttatgttttg tgaagttgtg tcttttcact aattacctat actatgccaa tatttcctta 900

tatctatcca taacatttat actacatttg taagagaata tgcacgtgaa acttaacact 960

ttataaggta aaaatgaggt ttccaagatt taataatctg atcaagttct tgttatttcc 1020

aaatagaatg gactcggtct gttaagggct aaggagaaga ggaagataag gttaaaagtt 1080

gttaatgacc aaacattc 1098

<210> 12
<211> 1337
<212> DNA
<213> Homo sapiens

<400> 12
   ctggcgtccc ctttccggcc ggtccccatg gaggcgctgg ggaagctgaa gcagttcgat 60
   gcctacccca agactttgga ggacttccgg gtcaagacct gcgggggcgc caccgtgacc 120
   attgtcagtg gccttctcat gctgctactg ttcctgtccg agctgcagta ttacctcacc 180
   acggaggtgc atcctgagct ctacgtggac aagtcgcggg gagataaact gaagatcaac 240
   atcgatgtac tttttccgca catgccttgt gcctatctga gtattgatgc catggatgtg 300
   gccggagaac agcagctgga tgtggaacac aacctgttca agcaacgact agataaagat 360
   ggcatccccg tgagctcaga ggctgagcgg catgagcttg ggaaagtcga ggtgacggtg 420
   tttgaccctg actccctgga ccctgatcgc tgtgagagct gctatggtgc tgaggcagaa 480
   gatatcaagt gctgtaacac ctgtgaagat gtgcgggagg catatcgccg tagaggctgg 540
   gccttcaaga acccagatac tattgagcag tgccggcgag agggcttcag ccagaagatg 600
   caggagcaga agaatgaagg ctgccaggtg tatggcttct tggaagtcaa taaggtggcc 660
   ggaaacttcc actttgcccc tgggaagagc ttccagcagt cccatgtgca cgtccatgac 720
   ttgcagagct ttggccttga caacatcaac atgacccact acatccagca cctgtcattt 780
   ggggaggact atccaggcat tgtgaacccc ctggaccaca ccaatgtcac tgcgccccaa 840
   gcctccatga tgttccagta ctttgtgaag gtggtgccca ctgtgtacat gaaggtggac 900
   ggagaggtac tgaggacaaa tcagttctct gtgaccagac atgagaaggt tgccaatggg 960
   ctgttgggcg accaaggcct tcccggagtc ttcgtcctct atgagctctc gcccatgatg 1020
   gtgaagctga cggagaagca caggtccttc acccacttcc tgacaggtgt gtgcgccatc 1080
   attgggggca tgttcacagt ggctggactc atcgattcgc tcatctacca ctcagcacga 1140
   gccatccaga agaaaattga tctagggaag acaacgtagt caccctcggt gcttcctctg 1200
   tctcctcttt ctccctggcc tgtggttgtc ccccagcctc tgccaccctc cacctcctcg 1260
   gtcagcccca gccccaggtt gataaatcta ttgattgatt gtgatagtaa aaaaaaaaaa 1320
   aaaaaaaaaa aaaaaaa 1337

<210> 13
<211> 3213
<212> DNA
<213> Homo sapiens

<400> 13
   agagactcaa gatgattccc tttttaccca tgttttctct actattgctg cttattgtta 60
   accctataaa cgccaacaat cattatgaca agatcttggc tcatagtcgt atcaggggtc 120
   gggaccaagg cccaaatgtc tgtgcccttc aacagatttt gggcaccaaa aagaaatact 180
   tcagcacttg taagaactgg tataaaaagt ccatctgtgg acagaaaacg actgttttat 240
   atgaatgttg ccctggttat atgagaatgg aaggaatgaa aggctgccca gcagttttgc 300
   ccattgacca tgtttatggc actctgggca tcgtgggagc caccacaacg cagcgctatt 360
   ctgacgcctc aaaactgagg gaggagatcg agggaaaggg atccttcact tactttgcac 420
   cgagtaatga ggcttgggac aacttggatt ctgatatccg tagaggtttg gagagcaacg 480
   tgaatgttga attactgaat gctttacata gtcacatgat taataagaga atgttgacca 540
   aggacttaaa aaatggcatg attattcctt caatgtataa caatttgggg cttttcatta 600
   accattatcc taatggggtt gtcactgtta attgtgctcg aatcatccat gggaaccaga 660
   ttgcaacaaa tggtgttgtc catgtcattg accgtgtgct tacacaaatt ggtacctcaa 720
   ttcaagactt cattgaagca gaagatgacc tttcatcttt tagagcagct gccatcacat 780
   cggacatatt ggaggccctt ggaagagacg gtcacttcac actctttgct cccaccaatg 840
   aggcttttga gaaacttcca cgaggtgtcc tagaaaggtt catgggagac aaagtggctt 900
   ccgaagctct tatgaagtac cacatcttaa atactctcca gtgttctgag tctattatgg 960
   gaggagcagt ctttgagacg ctggaaggaa atacaattga gataggatgt gacggtgaca 1020
   gtataacagt aaatggaatc aaaatggtga acaaaaagga tattgtgaca aataatggtg 1080
   tgatccattt gattgatcag gtcctaattc ctgattctgc caaacaagtt attgagctgg 1140
   ctggaaaaca gcaaaccacc ttcacggatc ttgtggccca attaggcttg gcatctgctc 1200
   tgaggccaga tggagaatac actttgctgg cacctgtgaa taatgcattt tctgatgata 1260
   ctctcagcat ggttcagcgc ctccttaaat taattctgca gaatcacata ttgaaagtaa 1320
   aagttggcct taatgagctt tacaacgggc aaatactgga aaccatcgga ggcaaacagc 1380
   tcagagtctt cgtatatcgt acagctgtct gcattgaaaa ttcatgcatg gagaaaggga 1440
   gtaagcaagg gagaaacggt gcgattcaca tattccgcga gatcatcaag ccagcagaga 1500
   aatccctcca tgaaaagtta aaacaagata agcgctttag caccttcctc agcctacttg 1560
   aagctgcaga cttgaaagag ctcctgacac aacctggaga ctggacatta tttgtgccaa 1620
   ccaatgatgc ttttaaggga atgactagtg aagaaaaaga aattctgata cgggacaaaa 1680
   atgctcttca aaacatcatt ctttatcacc tgacaccagg agttttcatt ggaaaaggat 1740
   ttgaacctgg tgttactaac attttaaaga ccacacaagg aagcaaaatc tttctgaaag 1800
   aagtaaatga tacacttctg gtgaatgaat tgaaatcaaa agaatctgac atcatgacaa 1860
   caaatggtgt aattcatgtt gtagataaac tcctctatcc agcagacaca cctgttggaa 1920
   atgatcaact gctggaaata cttaataaat taatcaaata catccaaatt aagtttgttc 1980
   gtggtagcac cttcaaagaa atccccgtga ctgtctatac aactaaaatt ataaccaaag 2040
   ttgtggaacc aaaaattaaa gtgattgaag gcagtcttca gcctattatc aaaactgaag 2100
   gacccacact aacaaaagtc aaaattgaag gtgaacctga attcagactg attaaagaag 2160
   gtgaaacaat aactgaagtg atccatggag agccaattat taaaaaatac accaaaatca 2220
   ttgatggagt gcctgtggaa ataactgaaa aagagacacg agaagaacga atcattacag 2280
   gtcctgaaat aaaatacact aggatttcta ctggaggtgg agaaacagaa gaaactctga 2340
   agaaattgtt acaagaagag gtcaccaagg tcaccaaatt cattgaaggt ggtgatggtc 2400
   atttatttga agatgaagaa attaaaagac tgcttcaggg agacacaccc gtgaggaagt 2460
   tgcaagccaa caaaaaagtt caaggttcta gaagacgatt aagggaaggt cgttctcagt 2520
   gaaaatccaa aaaccagaaa aaaatgttta tacaacccta agtcaataac ctgaccttag 2580
   aaaattgtga gagccaagtt gacttcagga actgaaacat cagcacaaag aagcaatcat 2640
   caaataattc tgaacacaaa tttaatattt ttttttctga atgagaaaca tgagggaaat 2700
   tgtggagtta gcctcctgtg gtaaaggaat tgaagaaaat ataacacctt acaccctttt 2760
   tcatcttgac attaaaagtt ctggctaact ttggaatcca ttagagaaaa atccttgtca 2820
   ccagattcat tacaattcaa atcgaagagt tgtgaactgt tatcccattg aaaagaccga 2880
   gccttgtatg tatgttatgg atacataaaa tgcacgcaag ccattatctc tccatgggaa 2940
   gctaagttat aaaaataggt gcttggtgta caaaactttt tatatcaaaa ggctttgcac 3000
   atttctatat gagtgggttt actggtaaat tatgttattt tttacaacta attttgtact 3060
   ctcagaatgt ttgtcatatg cttcttgcaa tgcatatttt ttaatctcaa acgtttcaat 3120
   aaaaccattt ttcagatata aagagaatta cttcaaattg agtaattcag aaaaactcaa 3180
   gatttaagtt aaaaagtggt ttggacttgg gaa 3213

<210> 14
<211> 2133
<212> DNA
<213> Homo sapiens

<400> 14
cgggagagcg cgctctgcct gccgcctgcc tgcctgccac tgagggttcc cagcaccatg 60

agggcctgga tcttctttct cctttgcctg gccgggaggg ccttggcagc ccctcagcaa 120

gaagccctgc ctgatgagac agaggtggtg gaagaaactg tggcagaggt gactgaggta 180

tctgtgggag ctaatcctgt ccaggtggaa gtaggagaat ttgatgatgg tgcagaggaa 240

accgaagagg aggtggtggc ggaaaatccc tgccagaacc accactgcaa acacggcaag 300

gtgtgcgagc tggatgagaa caacaccccc atgtgcgtgt gccaggaccc caccagctgc 360

ccagccccca ttggcgagtt tgagaaggtg tgcagcaatg acaacaagac cttcgactct 420

tcctgccact tctttgccac aaagtgcacc ctggagggca ccaagaaggg ccacaagctc 480

cacctggact acatcgggcc ttgcaaatac atcccccctt gcctggactc tgagctgacc 540

gaattccccc tgcgcatgcg ggactggctc aagaacgtcc tggtcaccct gtatgagagg 600

gatgaggaca acaaccttct gactgagaag cagaagctgc gggtgaagaa gatccatgag 660

aatgagaagc gcctggaggc aggagaccac cccgtggagc tgctggcccg ggacttcgag 720

aagaactata acatgtacat cttccctgta cactggcagt tcggccagct ggaccagcac 780

cccattgacg ggtacctctc ccacaccgag ctggctccac tgcgtgctcc cctcatcccc 840

atggagcatt gcaccacccg ctttttcgag acctgtgacc tggacaatga caagtacatc 900

gccctggatg agtgggccgg ctgcttcggc atcaagcaga aggatatcga caaggatctt 960

gtgatctaaa tccactcctt ccacagtacc ggattctctc tttaaccctc cccttcgtgt 1020

ttcccccaat gtttaaaatg tttggatggt ttgttgttct gcctggagac aaggtgctaa 1080

catagattta agtgaataca ttaacggtgc taaaaatgaa aattctaacc caagacatga 1140

cattcttagc tgtaacttaa ctattaaggc cttttccaca cgcattaata gtcccatttt 1200

tctcttgcca tttgtagctt tgcccattgt cttattggca catgggtgga cacggatctg 1260

ctgggctctg ccttaaacac acattgcagc ttcaactttt ctctttagtg ttctgtttga 1320

aactaatact taccgagtca gactttgtgt tcatttcatt tcagggtctt ggctgcctgt 1380

gggcttcccc aggtggcctg gaggtgggca aagggaagta acagacacac gatgttgtca 1440

aggatggttt tgggactaga ggctcagtgg tgggagagat ccctgcagaa tccaccaacc 1500

agaacgtggt ttgcctgagg ctgtaactga gagaaagatt ctggggctgt cttatgaaaa 1560

tatagacatt ctcacataag cccagttcat caccatttcc tcctttacct ttcagtgcag 1620

tttcttttca cattaggctg ttggttcaaa cttttgggag cacggactgt cagttctctg 1680

ggaagtggtc agcgcatcct gcagggcttc tcctcctctg tcttttggag aaccagggct 1740

cttctcaggg gctctaggga ctgccaggct gtttcagcca ggaaggccaa aatcaagagt 1800

gagatgtaga aagttgtaaa atagaaaaag tggagttggt gaatcggttg ttctttcctc 1860

acatttggat gattgtcata aggtttttag catgttcctc cttttcttca ccctcccctt 1920

tgttcttcta ttaatcaaga gaaacttcaa agttaatggg atggtcggat ctcacaggct 1980

gagaactcgt tcacctccaa gcatttcatg aaaaagctgc ttcttattaa tcatacaaac 2040

tctcaccatg atgtgaagag tttcacaaat ctttcaaaat aaaaagtaat gacttagaaa 2100

ctgaaaaaaa aaaaaaaaaa aaaaaaaaaa aaa 2133

<210> 15
<211> 2691

<212> DNA
<213> Homo sapiens

<400> 15
gcttgcccgt cggtcgctag ctcgctcggt gcgcgtcgtc ccgctccatg gcgctcttcg 60

tgcggctgct ggctctcgcc ctggctctgg ccctgggccc cgccgcgacc ctggcgggtc 120

ccgccaagtc gccctaccag ctggtgctgc agcacagcag gctccggggc cgccagcacg 180

gccccaacgt gtgtgctgtg cagaaggtta ttggcactaa taggaagtac ttcaccaact 240

gcaagcagtg gtaccaaagg aaaatctgtg gcaaatcaac agtcatcagc tacgagtgct 300

gtcctggata tgaaaaggtc cctggggaga agggctgtcc agcagcccta ccactctcaa 360

acctttacga gaccctggga gtcgttggat ccaccaccac tcagctgtac acggaccgca 420

cggagaagct gaggcctgag atggaggggc ccggcagctt caccatcttc gcccctagca 480

acgaggcctg ggcctccttg ccagctgaag tgctggactc cctggtcagc aatgtcaaca 540

ttgagctgct caatgccctc cgctaccata tggtgggcag gcgagtcctg actgatgagc 600

tgaaacacgg catgaccctc acctctatgt accagaattc caacatccag atccaccact 660

atcctaatgg gattgtaact gtgaactgtg cccggctcct gaaagccgac caccatgcaa 720

ccaacggggt ggtgcacctc atcgataagg tcatctccac catcaccaac aacatccagc 780

agatcattga gatcgaggac acctttgaga cccttcgggc tgctgtggct gcatcagggc 840

tcaacacgat gcttgaaggt aacggccagt acacgctttt ggccccgacc aatgaggcct 900

tcgagaagat ccctagtgag actttgaacc gtatcctggg cgacccagaa gccctgagag 960

acctgctgaa caaccacatc ttgaagtcag ctatgtgtgc tgaagccatc gttgcggggc 1020

tgtctgtaga gaccctggag ggcacgacac tggaggtggg ctgcagcggg gacatgctca 1080

ctatcaacgg gaaggcgatc atctccaata aagacatcct agccaccaac ggggtgatcc 1140

actacattga tgagctactc atcccagact cagccaagac actatttgaa ttggctgcag 1200

agtctgatgt gtccacagcc attgaccttt tcagacaagc cggcctcggc aatcatctct 1260

ctggaagtga gcggttgacc ctcctggctc ccctgaattc tgtattcaaa gatggaaccc 1320

ctccaattga tgcccataca aggaatttgc ttcggaacca cataattaaa gaccagctgg 1380

cctctaagta tctgtaccat ggacagaccc tggaaactct gggcggcaaa aaactgagag 1440

tttttgttta tcgtaatagc ctctgcattg agaacagctg catcgcggcc cacgacaaga 1500

gggggaggta cgggaccctg ttcacgatgg accgggtgct gaccccccca atggggactg 1560

tcatggatgt cctgaaggga gacaatcgct ttagcatgct ggtagctgcc atccagtctg 1620

caggactgac ggagaccctc aaccgggaag gagtctacac agtctttgct cccacaaatg 1680

aagccttccg agccctgcca ccaagagaac ggagcagact cttgggagat gccaaggaac 1740

ttgccaacat cctgaaatac cacattggtg atgaaatcct ggttagcgga ggcatcgggg 1800

ccctggtgcg gctaaagtct ctccaaggtg acaagctgga agtcagcttg aaaaacaatg 1860

tggtgagtgt caacaaggag cctgttgccg agcctgacat catggccaca aatggcgtgg 1920

tccatgtcat caccaatgtt ctgcagcctc cagccaacag acctcaggaa agaggggatg 1980

aacttgcaga ctctgcgctt gagatcttca aacaagcatc agcgttttcc agggcttccc 2040

agaggtctgt gcgactagcc cctgtctatc aaaagttatt agagaggatg aagcattagc 2100

ttgaagcact acaggaggaa tgcaccacgg cagctctccg ccaatttctc tcagatttcc 2160

acagagactg tttgaatgtt ttcaaaacca agtatcacac tttaatgtac atgggccgca 2220

ccataatgag atgtgagcct tgtgcatgtg ggggaggagg gagagagatg tactttttaa 2280

atcatgttcc ccctaaacat ggctgttaac ccactgcatg cagaaacttg gatgtcactg 2340

cctgacattc acttccagag aggacctatc ccaaatgtgg aattgactgc ctatgccaag 2400

tccctggaaa aggagcttca gtattgtggg gctcataaaa catgaatcaa gcaatccagc 2460

ctcatgggaa gtcctggcac agtttttgta aagcccttgc acagctggag aaatggcatc 2520

attataagct atgagttgaa atgttctgtc aaatgtgtct cacatctaca cgtggcttgg 2580

aggcttttat ggggccctgt ccaggtagaa aagaaatggt atgtagagct tagatttccc 2640

tattgtgaca gagccatggt gtgtttgtaa taataaaacc aaagaaacat a 2691

<210> 16
<211> 5084
<212> DNA
<213> Homo sapiens

<400> 16
agcaccacgg cagcaggagg tttcggctaa gttggaggta ctggccacga ctgcatgccc 60

gcgcccgcca ggtgatacct ccgccggtga cccaggggct ctgcgacaca aggagtctgc 120

atgtctaagt gctagacatg ctcagctttg tggatacgcg gactttgttg ctgcttgcag 180

taaccttatg cctagcaaca tgccaatctt tacaagagga aactgtaaga aagggcccag 240

ccggagatag aggaccacgt ggagaaaggg gtccaccagg ccccccaggc agagatggtg 300

aagatggtcc cacaggccct cctggtccac ctggtcctcc tggcccccct ggtctcggtg 360

ggaactttgc tgctcagtat gatggaaaag gagttggact tggccctgga ccaatgggct 420

taatgggacc tagaggccca cctggtgcag ctggagcccc aggccctcaa ggtttccaag 480

gacctgctgg tgagcctggt gaacctggtc aaactggtcc tgcaggtgct cgtggtccag 540

ctggccctcc tggcaaggct ggtgaagatg gtcaccctgg aaaacccgga cgacctggtg 600

agagaggagt tgttggacca cagggtgctc gtggtttccc tggaactcct ggacttcctg 660

gcttcaaagg cattagggga cacaatggtc tggatggatt gaagggacag cccggtgctc 720

ctggtgtgaa gggtgaacct ggtgcccctg gtgaaaatgg aactccaggt caaacaggag 780

cccgtgggct tcctggtgag agaggacgtg ttggtgcccc tggcccagct ggtgcccgtg 840

gcagtgatgg aagtgtgggt cccgtgggtc ctgctggtcc cattgggtct gctggccctc 900

caggcttccc aggtgcccct ggccccaagg gtgaaattgg agctgttggt aacgctggtc 960

ctgctggtcc cgccggtccc cgtggtgaag tgggtcttcc aggcctctcc ggccccgttg 1020

gacctcctgg taatcctgga gcaaacggcc ttactggtgc caagggtgct gctggccttc 1080

ccggcgttgc tggggctccc ggcctccctg gaccccgcgg tattcctggc cctgttggtg 1140

ctgccggtgc tactggtgcc agaggacttg ttggtgagcc tggtccagct ggctccaaag 1200

gagagagcgg taacaagggt gagcccggct ctgctgggcc ccaaggtcct cctggtccca 1260

gtggtgaaga aggaaagaga ggccctaatg gggaagctgg atctgccggc cctccaggac 1320

ctcctgggct gagaggtagt cctggttctc gtggtcttcc tggagctgat ggcagagctg 1380

gcgtcatggg ccctcctggt agtcgtggtg caagtggccc tgctggagtc cgaggaccta 1440

atggagatgc tggtcgccct ggggagcctg gtctcatggg acccagaggt cttcctggtt 1500

cccctggaaa tatcggcccc gctggaaaag aaggtcctgt cggcctccct ggcatcgacg 1560

gcaggcctgg cccaattggc ccagctggag caagaggaga gcctggcaac attggattcc 1620

ctggacccaa aggccccact ggtgatcctg gcaaaaacgg tgataaaggt catgctggtc 1680

ttgctggtgc tcggggtgct ccaggtcctg atggaaacaa tggtgctcag ggacctcctg 1740

gaccacaggg tgttcaaggt ggaaaaggtg aacagggtcc cgctggtcct ccaggcttcc 1800

agggtctgcc tggcccctca ggtcccgctg gtgaagttgg caaaccagga gaaaggggtc 1860

tccatggtga gtttggtctc cctggtcctg ctggtccaag aggggaacgc ggtcccccag 1920

gtgagagtgg tgctgccggt cctactggtc ctattggaag ccgaggtcct tctggacccc 1980

cagggcctga tggaaacaag ggtgaacctg gtgtggttgg tgctgtgggc actgctggtc 2040

catctggtcc tagtggactc ccaggagaga ggggtgctgc tggcatacct ggaggcaagg 2100

gagaaaaggg tgaacctggt ctcagaggtg aaattggtaa ccctggcaga gatggtgctc 2160

gtggtgctca tggtgctgta ggtgcccctg gtcctgctgg agccacaggt gaccggggcg 2220

aagctggggc tgctggtcct gctggtcctg ctggtcctcg gggaagccct ggtgaacgtg 2280

gcgaggtcgg tcctgctggc cccaacggat ttgctggtcc ggctggtgct gctggtcaac 2340

cgggtgctaa aggagaaaga ggagccaaag ggcctaaggg tgaaaacggt gttgttggtc 2400

ccacaggccc cgttggagct gctggcccag ctggtccaaa tggtcccccc ggtcctgctg 2460

gaagtcgtgg tgatggaggc ccccctggta tgactggttt ccctggtgct gctggacgga 2520

ctggtccccc aggaccctct ggtatttctg gccctcctgg tccccctggt cctgctggga 2580

aagaagggct tcgtggtcct cgtggtgacc aaggtccagt tggccgaact ggagaagtag 2640

gtgcagttgg tccccctggc ttcgctggtg agaagggtcc ctctggagag gctggtactg 2700

ctggacctcc tggcactcca ggtcctcagg gtcttcttgg tgctcctggt attctgggtc 2760

tccctggctc gagaggtgaa cgtggtctac ctggtgttgc tggtgctgtg ggtgaacctg 2820

gtcctcttgg cattgccggc cctcctgggg cccgtggtcc tcctggtgct gtgggtagtc 2880

ctggagtcaa cggtgctcct ggtgaagctg gtcgtgatgg caaccctggg aacgatggtc 2940

ccccaggtcg cgatggtcaa cccggacaca agggagagcg cggttaccct ggcaatattg 3000

gtcccgttgg tgctgcaggt gcacctggtc ctcatggccc cgtgggtcct gctggcaaac 3060

atggaaaccg tggtgaaact ggtccttctg gtcctgttgg tcctgctggt gctgttggcc 3120

caagaggtcc tagtggccca caaggcattc gtggcgataa gggagagccc ggtgaaaagg 3180

ggcccagagg tcttcctggc ttaaagggac acaatggatt gcaaggtctg cctggtatcg 3240

ctggtcacca tggtgatcaa ggtgctcctg gctccgtggg tcctgctggt cctaggggcc 3300

ctgctggtcc ttctggccct gctggaaaag atggtcgcac tggacatcct ggtacggttg 3360

gacctgctgg cattcgaggc cctcagggtc accaaggccc tgctggcccc cctggtcccc 3420

ctggccctcc tggacctcca ggtgtaagcg gtggtggtta tgactttggt tacgatggag 3480

acttctacag ggctgaccag cctcgctcag caccttctct cagacccaag gactatgaag 3540

ttgatgctac tctgaagtct ctcaacaacc agattgagac ccttcttact cctgaaggct 3600

ctagaaagaa cccagctcgc acatgccgtg acttgagact cagccaccca gagtggagca 3660

gtggttacta ctggattgac cctaaccaag gatgcactat ggatgctatc aaagtatact 3720

gtgatttctc tactggcgaa acctgtatcc gggcccaacc tgaaaacatc ccagccaaga 3780

actggtatag gagctccaag gacaagaaac acgtctggct aggagaaact atcaatgctg 3840

gcagccagtt tgaatataat gtagaaggag tgacttccaa ggaaatggct acccaacttg 3900

ccttcatgcg cctgctggcc aactatgcct ctcagaacat cacctaccac tgcaagaaca 3960

gcattgcata catggatgag gagactggca acctgaaaaa ggctgtcatt ctacagggct 4020

ctaatgatgt tgaacttgtt gctgagggca acagcaggtt cacttacact gttcttgtag 4080

atggctgctc taaaaagaca aatgaatggg gaaagacaat cattgaatac aaaacaaata 4140

agccatcacg cctgcccttc cttgatattg cacctttgga catcggtggt gctgaccatg 4200

aattctttgt ggacattggc ccagtctgtt tcaaataaat gaactcaatc taaattaaaa 4260

aagaaagaaa tttgaaaaaa ctttctcttt gccatttctt cttcttcttt tttaactgaa 4320

agctgaatcc ttccatttct tctgcacatc tacttgctta aattgtgggc aaaagagaaa 4380

aagaaggatt gatcagagca ttgtgcaata cagtttcatt aactccttcc cccgctcccc 4440

caaaaatttg aatttttttt tcaacactct tacacctgtt atggaaaatg tcaacctttg 4500

taagaaaacc aaaataaaaa ttgaaaaata aaaaccataa acatttgcac cacttgtggc 4560

ttttgaatat cttccacaga gggaagttta aaacccaaac ttccaaaggt ttaaactacc 4620

tcaaaacact ttcccatgag tgtgatccac attgttaggt gctgacctag acagagatga 4680

actgaggtcc ttgttttgtt ttgttcataa tacaaaggtg ctaattaata gtatttcaga 4740

tacttgaaga atgttgatgg tgctagaaga atttgagaag aaatactcct gtattgagtt 4800

gtatcgtgtg gtgtattttt taaaaaattt gatttagcat tcatattttc catcttattc 4860

ccaattaaaa gtatgcagat tatttgccca aagttgtcct cttcttcaga ttcagcattt 4920

gttctttgcc agtctcattt tcatcttctt ccatggttcc acagaagctt tgtttcttgg 4980

gcaagcagaa aaattaaatt gtacctattt tgtatatgtg agatgtttaa ataaattgtg 5040

aaaaaaatga aataaagcat gtttggtttt ccaaaagaac atat 5084

<210> 17
<211> 99
<212> PRT
<213> Homo sapiens

<400> 17

Met Thr Ser Lys Leu Ala Val Ala Leu Leu Ala Ala Phe Leu Ile Ser
1               5                   10                  15

Ala Ala Leu Cys Glu Gly Ala Val Leu Pro Arg Ser Ala Lys Glu Leu
            20                  25                  30

Arg Cys Gln Cys Ile Lys Thr Tyr Ser Lys Pro Phe His Pro Lys Phe
        35                  40                  45

Ile Lys Glu Leu Arg Val Ile Glu Ser Gly Pro His Cys Ala Asn Thr
    50                  55                  60

Glu Ile Ile Val Lys Leu Ser Asp Gly Arg Glu Leu Cys Leu Asp Pro
65                  70                  75                  80

Lys Glu Asn Trp Val Gln Arg Val Val Glu Lys Phe Leu Lys Arg Ala
                85                  90                  95

Glu Asn Ser

<210> 18
<211> 338
<212> PRT
<213> Homo sapiens

<400> 18

Met Ser Leu Ser Ala Phe Thr Leu Phe Leu Ala Leu Ile Gly Gly Thr
1               5                   10                  15

Ser Gly Gln Tyr Tyr Asp Tyr Asp Phe Pro Leu Ser Ile Tyr Gly Gln
            20                  25                  30

Ser Ser Pro Asn Cys Ala Pro Glu Cys Asn Cys Pro Glu Ser Tyr Pro
        35                  40                  45

Ser Ala Met Tyr Cys Asp Glu Leu Lys Leu Lys Ser Val Pro Met Val
    50                  55                  60

Pro Pro Gly Ile Lys Tyr Leu Tyr Leu Arg Asn Asn Gln Ile Asp His
65                  70                  75                  80

Ile Asp Glu Lys Ala Phe Glu Asn Val Thr Asp Leu Gln Trp Leu Ile
                85                  90                  95

Leu Asp His Asn Leu Leu Glu Asn Ser Lys Ile Lys Gly Arg Val Phe
            100                 105                 110

Ser Lys Leu Lys Gln Leu Lys Lys Leu His Ile Asn His Asn Asn Leu
        115                 120                 125

Thr Glu Ser Val Gly Pro Leu Pro Lys Ser Leu Glu Asp Leu Gln Leu
    130                 135                 140

Thr His Asn Lys Ile Thr Lys Leu Gly Ser Phe Glu Gly Leu Val Asn
145                 150                 155                 160

Leu Thr Phe Ile His Leu Gln His Asn Arg Leu Lys Glu Asp Ala Val
                165                 170                 175

Ser Ala Ala Phe Lys Gly Leu Lys Ser Leu Glu Tyr Leu Asp Leu Ser
            180                 185                 190

Phe Asn Gln Ile Ala Arg Leu Pro Ser Gly Leu Pro Val Ser Leu Leu
        195                 200                 205

Thr Leu Tyr Leu Asp Asn Asn Lys Ile Ser Asn Ile Pro Asp Glu Tyr
210                215                220

Phe Lys Arg Phe Asn Ala Leu Gln Tyr Leu Arg Leu Ser His Asn Glu
225                230                235                240

Leu Ala Asp Ser Gly Ile Pro Gly Asn Ser Phe Asn Val Ser Ser Leu
                245                250                255

Val Glu Leu Asp Leu Ser Tyr Asn Lys Leu Lys Asn Ile Pro Thr Val
            260                265                270

Asn Glu Asn Leu Glu Asn Tyr Tyr Leu Glu Val Asn Gln Leu Glu Lys
        275                280                285

Phe Asp Ile Lys Ser Phe Cys Lys Ile Leu Gly Pro Leu Ser Tyr Ser
    290                295                300

Lys Ile Lys His Leu Arg Leu Asp Gly Asn Arg Ile Ser Glu Thr Ser
305                310                315                320

Leu Pro Pro Asp Met Tyr Glu Cys Leu Arg Val Ala Asn Glu Val Thr
                325                330                335

Leu Asn

<210> 19
<211> 193
<212> PRT
<213> Homo sapiens

<400> 19

Met Ile Arg Cys Gly Leu Ala Cys Glu Arg Cys Arg Trp Ile Leu Pro
1               5                   10                  15

Leu Leu Leu Leu Ser Ala Ile Ala Phe Asp Ile Ile Ala Leu Ala Gly
            20                  25                  30

Arg Gly Trp Leu Gln Ser Ser Asp His Gly Gln Thr Ser Ser Leu Trp
        35                  40                  45

Trp Lys Cys Ser Gln Glu Gly Gly Gly Ser Gly Ser Tyr Glu Glu Gly
    50                  55                  60

Cys Gln Ser Leu Met Glu Tyr Ala Trp Gly Arg Ala Ala Ala Ala Met
65                  70                  75                  80

Leu Phe Cys Gly Phe Ile Ile Leu Val Ile Cys Phe Ile Leu Ser Phe
                85                  90                  95

Phe Ala Leu Cys Gly Pro Gln Met Leu Val Phe Leu Arg Val Ile Gly
            100                 105                 110

Gly Leu Leu Ala Leu Ala Ala Val Phe Gln Ile Ile Ser Leu Val Ile
        115                 120                 125

Tyr Pro Val Lys Tyr Thr Gln Thr Phe Thr Leu His Ala Asn Pro Ala
    130                 135                 140

Val Thr Tyr Ile Tyr Asn Trp Ala Tyr Gly Phe Gly Trp Ala Ala Thr
145                 150                 155                 160

Ile Ile Leu Ile Gly Cys Ala Phe Phe Phe Cys Cys LeuLeu Asn Tyr
165 170 175

Glu Asp Asp Leu Leu Gly Asn Ala Lys Pro Arg Tyr Phe Tyr Thr Ser
180 185 190

Ala

<210> 20
<211> 383
<212> PRT
<213> Homo sapiens

<400> 20

Met Glu Ala Leu Gly Lys Leu Lys Gln Phe Asp Ala Tyr Pro Lys Thr
1               5                   10                  15

Leu Glu Asp Phe Arg Val Lys Thr Cys Gly Gly Ala Thr Val Thr Ile
            20                  25                  30

Val Ser Gly Leu Leu Met Leu Leu Leu Phe Leu Ser Glu Leu Gln Tyr
        35                  40                  45

Tyr Leu Thr Thr Glu Val His Pro Glu Leu Tyr Val Asp Lys Ser Arg
    50                  55                  60

Gly Asp Lys Leu Lys Ile Asn Ile Asp Val Leu Phe Pro His Met Pro
65                  70                  75                  80

Cys Ala Tyr Leu Ser Ile Asp Ala Met Asp Val Ala Gly Glu Gln Gln
                85                  90                  95

Leu Asp Val Glu His Asn Leu Phe Lys Gln Arg Leu Asp Lys Asp Gly
            100                 105                 110

Ile Pro Val Ser Ser Glu Ala Glu Arg His Glu Leu Gly Lys Val Glu
        115                 120                 125

Val Thr Val Phe Asp Pro Asp Ser Leu Asp Pro Asp Arg Cys Glu Ser
    130                 135                 140

Cys Tyr Gly Ala Glu Ala Glu Asp Ile Lys Cys Cys Asn Thr Cys Glu
145                 150                 155                 160

Asp Val Arg Glu Ala Tyr Arg Arg Arg Gly Trp Ala Phe Lys Asn Pro
                165                 170                 175

Asp Thr Ile Glu Gln Cys Arg Arg Glu Gly Phe Ser Gln Lys Met Gln
            180                 185                 190

Glu Gln Lys Asn Glu Gly Cys Gln Val Tyr Gly Phe Leu Glu Val Asn
        195                 200                 205

Lys Val Ala Gly Asn Phe His Phe Ala Pro Gly Lys Ser Phe Gln Gln
    210                 215                 220

Ser His Val His Val His Asp Leu Gln Ser Phe Gly Leu Asp Asn Ile
225                 230                 235                 240

Asn Met Thr His Tyr Ile Gln His Leu Ser Phe Gly Glu Asp Tyr Pro
                245                 250                 255

Gly Ile Val Asn Pro Leu Asp His Thr Asn Val Thr Ala Pro Gln Ala
            260                 265                 270

Ser Met Met Phe Gln Tyr Phe Val Lys Val Val Pro Thr Val Tyr Met
        275                 280                 285

Lys Val Asp Gly Glu Val Leu Arg Thr Asn Gln Phe Ser Val Thr Arg
    290                 295                 300

His Glu Lys Val Ala Asn Gly Leu Leu Gly Asp Gln Gly Leu Pro Gly
305                 310                 315                 320

Val Phe Val Leu Tyr Glu Leu Ser Pro Met Met Val Lys Leu Thr Glu
                325                 330                 335

Lys His Arg Ser Phe Thr His Phe Leu Thr Gly Val Cys Ala Ile Ile
            340                 345                 350

Gly Gly Met Phe Thr Val Ala Gly Leu Ile Asp Ser Leu Ile Tyr His
        355                 360                 365

Ser Ala Arg Ala Ile Gln Lys Lys Ile Asp Leu Gly Lys Thr Thr
    370                 375                 380

<210> 21
<211> 836
<212> PRT
<213> Homo sapiens

<400> 21

Met Ile Pro Phe Leu Pro Met Phe Ser Leu Leu Leu Leu Leu Ile Val

1               5                   10                  15

Asn Pro Ile Asn Ala Asn Asn His Tyr Asp Lys Ile Leu Ala His Ser
            20                  25                  30

Arg Ile Arg Gly Arg Asp Gln Gly Pro Asn Val Cys Ala Leu Gln Gln
        35                  40                  45

Ile Leu Gly Thr Lys Lys Lys Tyr Phe Ser Thr Cys Lys Asn Trp Tyr
    50                  55                  60

Lys Lys Ser Ile Cys Gly Gln Lys Thr Thr Val Leu Tyr Glu Cys Cys
65                  70                  75                  80

Pro Gly Tyr Met Arg Met Glu Gly Met Lys Gly Cys Pro Ala Val Leu
                85                  90                  95

Pro Ile Asp His Val Tyr Gly Thr Leu Gly Ile Val Gly Ala Thr Thr
            100                 105                 110

Thr Gln Arg Tyr Ser Asp Ala Ser Lys Leu Arg Glu Glu Ile Glu Gly
        115                 120                 125

Lys Gly Ser Phe Thr Tyr Phe Ala Pro Ser Asn Glu Ala Trp Asp Asn
    130                 135                 140

Leu Asp Ser Asp Ile Arg Arg Gly Leu Glu Ser Asn Val Asn Val Glu
145                 150                 155                 160

Leu Leu Asn Ala Leu His Ser His Met Ile Asn Lys Arg Met Leu Thr
                165                 170                 175

Lys Asp Leu Lys Asn Gly Met Ile Ile Pro Ser Met Tyr Asn Asn Leu
            180                 185                 190

Gly Leu Phe Ile Asn His Tyr Pro Asn Gly Val Val Thr Val Asn Cys
        195                 200                 205

Ala Arg Ile Ile His Gly Asn Gln Ile Ala Thr Asn Gly Val Val His

    210                 215                 220

Val Ile Asp Arg Val Leu Thr Gln Ile Gly Thr Ser Ile Gln Asp Phe
225                 230                 235                 240

Ile Glu Ala Glu Asp Asp Leu Ser Ser Phe Arg Ala Ala Ala Ile Thr
                245                 250                 255

Ser Asp Ile Leu Glu Ala Leu Gly Arg Asp Gly His Phe Thr Leu Phe
            260                 265                 270

Ala Pro Thr Asn Glu Ala Phe Glu Lys Leu Pro Arg Gly Val Leu Glu
        275                 280                 285

Arg Phe Met Gly Asp Lys Val Ala Ser Glu Ala Leu Met Lys Tyr His
    290                 295                 300

Ile Leu Asn Thr Leu Gln Cys Ser Glu Ser Ile Met Gly Gly Ala Val
305                 310                 315                 320

Phe Glu Thr Leu Glu Gly Asn Thr Ile Glu Ile Gly Cys Asp Gly Asp
                325                 330                 335

Ser Ile Thr Val Asn Gly Ile Lys Met Val Asn Lys Lys Asp Ile Val
            340                 345                 350

Thr Asn Asn Gly Val Ile His Leu Ile Asp Gln Val Leu Ile Pro Asp
        355                 360                 365

Ser Ala Lys Gln Val Ile Glu Leu Ala Gly Lys Gln Gln Thr Thr Phe
    370                 375                 380

Thr Asp Leu Val Ala Gln Leu Gly Leu Ala Ser Ala Leu Arg Pro Asp
385                 390                 395                 400

Gly Glu Tyr Thr Leu Leu Ala Pro Val Asn Asn Ala Phe Ser Asp Asp
                405                 410                 415

Thr Leu Ser Met Val Gln Arg Leu Leu Lys Leu Ile Leu Gln Asn His

            420                 425                 430

Ile Leu Lys Val Lys Val Gly Leu Asn Glu Leu Tyr Asn Gly Gln Ile
        435                 440                 445

Leu Glu Thr Ile Gly Gly Lys Gln Leu Arg Val Phe Val Tyr Arg Thr
    450                 455                 460

Ala Val Cys Ile Glu Asn Ser Cys Met Glu Lys Gly Ser Lys Gln Gly
465                 470                 475                 480

Arg Asn Gly Ala Ile His Ile Phe Arg Glu Ile Ile Lys Pro Ala Glu
                485                 490                 495

Lys Ser Leu His Glu Lys Leu Lys Gln Asp Lys Arg Phe Ser Thr Phe
            500                 505                 510

Leu Ser Leu Leu Glu Ala Ala Asp Leu Lys Glu Leu Leu Thr Gln Pro
        515                 520                 525

Gly Asp Trp Thr Leu Phe Val Pro Thr Asn Asp Ala Phe Lys Gly Met
    530                 535                 540

Thr Ser Glu Glu Lys Glu Ile Leu Ile Arg Asp Lys Asn Ala Leu Gln
545                 550                 555                 560

Asn Ile Ile Leu Tyr His Leu Thr Pro Gly Val Phe Ile Gly Lys Gly
                565                 570                 575

Phe Glu Pro Gly Val Thr Asn Ile Leu Lys Thr Thr Gln Gly Ser Lys
            580                 585                 590

Ile Phe Leu Lys Glu Val Asn Asp Thr Leu Leu Val Asn Glu Leu Lys
        595                 600                 605

Ser Lys Glu Ser Asp Ile Met Thr Thr Asn Gly Val Ile His Val Val
    610                 615                 620

Asp Lys Leu Leu Tyr Pro Ala Asp Thr Pro Val Gly Asn Asp Gln Leu

625                 630                 635                 640

Leu Glu Ile Leu Asn Lys Leu Ile Lys Tyr Ile Gln Ile Lys Phe Val
            645                 650                 655

Arg Gly Ser Thr Phe Lys Glu Ile Pro Val Thr Val Tyr Thr Thr Lys
        660                 665                 670

Ile Ile Thr Lys Val Val Glu Pro Lys Ile Lys Val Ile Glu Gly Ser
        675                 680                 685

Leu Gln Pro Ile Ile Lys Thr Glu Gly Pro Thr Leu Thr Lys Val Lys
    690                 695                 700

Ile Glu Gly Glu Pro Glu Phe Arg Leu Ile Lys Glu Gly Glu Thr Ile
705                 710                 715                 720

Thr Glu Val Ile His Gly Glu Pro Ile Ile Lys Lys Tyr Thr Lys Ile
            725                 730                 735

Ile Asp Gly Val Pro Val Glu Ile Thr Glu Lys Glu Thr Arg Glu Glu
        740                 745                 750

Arg Ile Ile Thr Gly Pro Glu Ile Lys Tyr Thr Arg Ile Ser Thr Gly
        755                 760                 765

Gly Gly Glu Thr Glu Glu Thr Leu Lys Lys Leu Leu Gln Glu Glu Val
    770                 775                 780

Thr Lys Val Thr Lys Phe Ile Glu Gly Gly Asp Gly His Leu Phe Glu
785                 790                 795                 800

Asp Glu Glu Ile Lys Arg Leu Leu Gln Gly Asp Thr Pro Val Arg Lys
            805                 810                 815

Leu Gln Ala Asn Lys Lys Val Gln Gly Ser Arg Arg Arg Leu Arg Glu
        820                 825                 830

Gly Arg Ser Gln

        835

<210> 22
<211> 303
<212> PRT
<213> Homo sapiens

<400> 22

Met Arg Ala Trp Ile Phe Phe Leu Leu Cys Leu Ala Gly Arg Ala Leu
1               5                   10                  15

Ala Ala Pro Gln Gln Glu Ala Leu Pro Asp Glu Thr Glu Val Val Glu
            20                  25                  30

Glu Thr Val Ala Glu Val Thr Glu Val Ser Val Gly Ala Asn Pro Val
        35                  40                  45

Gln Val Glu Val Gly Glu Phe Asp Asp Gly Ala Glu Glu Thr Glu Glu
    50                  55                  60

Glu Val Val Ala Glu Asn Pro Cys Gln Asn His His Cys Lys His Gly
65                  70                  75                  80

Lys Val Cys Glu Leu Asp Glu Asn Asn Thr Pro Met Cys Val Cys Gln
                85                  90                  95

Asp Pro Thr Ser Cys Pro Ala Pro Ile Gly Glu Phe Glu Lys Val Cys
            100                 105                 110

Ser Asn Asp Asn Lys Thr Phe Asp Ser Ser Cys His Phe Phe Ala Thr
        115                 120                 125

Lys Cys Thr Leu Glu Gly Thr Lys Lys Gly His Lys Leu His Leu Asp
    130                 135                 140

Tyr Ile Gly Pro Cys Lys Tyr Ile Pro Pro Cys Leu Asp Ser Glu Leu
145                 150                 155                 160

Thr Glu Phe Pro Leu Arg Met Arg Asp Trp Leu Lys Asn Val Leu Val
                165                 170                 175

Thr Leu Tyr Glu Arg Asp Glu Asp Asn Asn Leu Leu Thr Glu Lys Gln
            180                 185                 190

Lys Leu Arg Val Lys Lys Ile His Glu Asn Glu Lys Arg Leu Glu Ala
        195                 200                 205

Gly Asp His Pro Val Glu Leu Leu Ala Arg Asp Phe Glu Lys Asn Tyr
    210                 215                 220

Asn Met Tyr Ile Phe Pro Val His Trp Gln Phe Gly Gln Leu Asp Gln
225                 230                 235                 240

His Pro Ile Asp Gly Tyr Leu Ser His Thr Glu Leu Ala Pro Leu Arg
                245                 250                 255

Ala Pro Leu Ile Pro Met Glu His Cys Thr Thr Arg Phe Phe Glu Thr
            260                 265                 270

Cys Asp Leu Asp Asn Asp Lys Tyr Ile Ala Leu Asp Glu Trp Ala Gly
        275                 280                 285

Cys Phe Gly Ile Lys Gln Lys Asp Ile Asp Lys Asp Leu Val Ile
    290                 295                 300

<210> 23
<211> 683
<212> PRT
<213> Homo sapiens

<400> 23

Met Ala Leu Phe Val Arg Leu Leu Ala Leu Ala Leu Ala Leu Ala Leu
1               5                   10                  15

Gly Pro Ala Ala Thr Leu Ala Gly Pro Ala Lys Ser Pro Tyr Gln Leu
            20                  25                  30

Val Leu Gln His Ser Arg Leu Arg Gly Arg Gln His Gly Pro Asn Val
        35                  40                  45

Cys Ala Val Gln Lys Val Ile Gly Thr Asn Arg Lys Tyr Phe Thr Asn
    50                  55                  60

Cys Lys Gln Trp Tyr Gln Arg Lys Ile Cys Gly Lys Ser Thr Val Ile
65                  70                  75                  80

Ser Tyr Glu Cys Cys Pro Gly Tyr Glu Lys Val Pro Gly Glu Lys Gly
                85                  90                  95

Cys Pro Ala Ala Leu Pro Leu Ser Asn Leu Tyr Glu Thr Leu Gly Val
            100                 105                 110

Val Gly Ser Thr Thr Thr Gln Leu Tyr Thr Asp Arg Thr Glu Lys Leu
        115                 120                 125

Arg Pro Glu Met Glu Gly Pro Gly Ser Phe Thr Ile Phe Ala Pro Ser
    130                 135                 140

Asn Glu Ala Trp Ala Ser Leu Pro Ala Glu Val Leu Asp Ser Leu Val
145                 150                 155                 160

Ser Asn Val Asn Ile Glu Leu Leu Asn Ala Leu Arg Tyr His Met Val
                165                 170                 175

Gly Arg Arg Val Leu Thr Asp Glu Leu Lys His Gly Met Thr Leu Thr
            180                 185                 190

Ser Met Tyr Gln Asn Ser Asn Ile Gln Ile His His Tyr Pro Asn Gly
        195                 200                 205

Ile Val Thr Val Asn Cys Ala Arg Leu Leu Lys Ala Asp His His Ala
    210                 215                 220

Thr Asn Gly Val Val His Leu Ile Asp Lys Val Ile Ser Thr Ile Thr
225                 230                 235                 240

Asn Asn Ile Gln Gln Ile Ile Glu Ile Glu Asp Thr Phe Glu Thr Leu
                245                 250                 255

Arg Ala Ala Val Ala Ala Ser Gly Leu Asn Thr Met Leu Glu Gly Asn
            260                 265                 270

Gly Gln Tyr Thr Leu Leu Ala Pro Thr Asn Glu Ala Phe Glu Lys Ile
        275                 280                 285

Pro Ser Glu Thr Leu Asn Arg Ile Leu Gly Asp Pro Glu Ala Leu Arg
    290                 295                 300

Asp Leu Leu Asn Asn His Ile Leu Lys Ser Ala Met Cys Ala Glu Ala
305                 310                 315                 320

Ile Val Ala Gly Leu Ser Val Glu Thr Leu Glu Gly Thr Thr Leu Glu
                325                 330                 335

Val Gly Cys Ser Gly Asp Met Leu Thr Ile Asn Gly Lys Ala Ile Ile
            340                 345                 350

Ser Asn Lys Asp Ile Leu Ala Thr Asn Gly Val Ile His Tyr Ile Asp
        355                 360                 365

Glu Leu Leu Ile Pro Asp Ser Ala Lys Thr Leu PheGlu Leu Ala Ala
370 375 380

Glu Ser Asp Val Ser Thr Ala Ile Asp Leu Phe Arg Gln Ala Gly Leu
385 390 395 400

Gly Asn His Leu Ser Gly Ser Glu Arg Leu Thr Leu Leu Ala Pro Leu
405 410 415

Asn Ser Val Phe Lys Asp Gly Thr Pro Pro Ile Asp Ala His Thr Arg
420 425 430

Asn Leu Leu Arg Asn His Ile Ile Lys Asp Gln Leu Ala Ser Lys Tyr
435 440 445

Leu Tyr His Gly Gln Thr Leu Glu Thr Leu Gly Gly Lys Lys Leu Arg
450 455 460

Val Phe Val Tyr Arg Asn Ser Leu Cys Ile Glu Asn Ser Cys Ile Ala
465                 470                 475                 480

Ala His Asp Lys Arg Gly Arg Tyr Gly Thr Leu Phe Thr Met Asp Arg
                485                 490                 495

Val Leu Thr Pro Pro Met Gly Thr Val Met Asp Val Leu Lys Gly Asp
            500                 505                 510

Asn Arg Phe Ser Met Leu Val Ala Ala Ile Gln Ser Ala Gly Leu Thr
        515                 520                 525

Glu Thr Leu Asn Arg Glu Gly Val Tyr Thr Val Phe Ala Pro Thr Asn
    530                 535                 540

Glu Ala Phe Arg Ala Leu Pro Pro Arg Glu Arg Ser Arg Leu Leu Gly
545                 550                 555                 560

Asp Ala Lys Glu Leu Ala Asn Ile Leu Lys Tyr His Ile Gly Asp Glu
                565                 570                 575

Ile Leu Val Ser Gly Gly Ile Gly Ala Leu Val Arg Leu Lys Ser Leu
            580                 585                 590

Gln Gly Asp Lys Leu Glu Val Ser Leu Lys Asn Asn Val Val Ser Val
        595                 600                 605


    610                 615                 620


625                 630                 635                 640


                645                 650                 655


            660                 665                 670


675 680

<210> 24
<211> 1366
<212> PRT
<213> Homo sapiens

<400> 24

Met Leu Ser Phe Val Asp Thr Arg Thr Leu Leu Leu Leu Ala Val Thr
1               5                   10                  15

Leu Cys Leu Ala Thr Cys Gln Ser Leu Gln Glu Glu Thr Val Arg Lys
            20                  25                  30

Gly Pro Ala Gly Asp Arg Gly Pro Arg Gly Glu Arg Gly Pro Pro Gly
        35                  40                  45

Pro Pro Gly Arg Asp Gly Glu Asp Gly Pro Thr Gly Pro Pro Gly Pro
    50                  55                  60

Pro Gly Pro Pro Gly Pro Pro Gly Leu Gly Gly Asn Phe Ala Ala Gln
65                  70                  75                  80

Tyr Asp Gly Lys Gly Val Gly Leu Gly Pro Gly Pro Met Gly Leu Met
                85                  90                  95

Gly Pro Arg Gly Pro Pro Gly Ala Ala Gly Ala Pro Gly Pro Gln Gly
            100                 105                 110

Phe Gln Gly Pro Ala Gly Glu Pro Gly Glu Pro Gly Gln Thr Gly Pro
        115                 120                 125

Ala Gly Ala Arg Gly Pro Ala Gly Pro Pro Gly Lys Ala Gly Glu Asp
    130                 135                 140

Gly His Pro Gly Lys Pro Gly Arg Pro Gly Glu Arg Gly Val Val Gly
145                 150                 155                 160

Pro Gln Gly Ala Arg Gly Phe Pro Gly Thr Pro Gly Leu Pro Gly Phe
                165                 170                 175

Lys Gly Ile Arg Gly His Asn Gly Leu Asp Gly Leu Lys Gly Gln Pro
            180                 185                 190

Gly Ala Pro Gly Val Lys Gly Glu Pro Gly Ala Pro Gly Glu Asn Gly
        195                 200                 205

Thr Pro Gly Gln Thr Gly Ala Arg Gly Leu Pro Gly Glu Arg Gly Arg
    210                 215                 220

Val Gly Ala Pro Gly Pro Ala Gly Ala Arg Gly Ser Asp Gly Ser Val
225                 230                 235                 240

Gly Pro Val Gly Pro Ala Gly Pro Ile Gly Ser Ala Gly Pro Pro Gly
                245                 250                 255

Phe Pro Gly Ala Pro Gly Pro Lys Gly Glu Ile Gly Ala Val Gly Asn
            260                 265                 270

Ala Gly Pro Ala Gly Pro Ala Gly Pro Arg Gly Glu Val Gly Leu Pro
        275                 280                 285

Gly Leu Ser Gly Pro Val Gly Pro Pro Gly Asn Pro Gly Ala Asn Gly
    290                 295                 300

Leu Thr Gly Ala Lys Gly Ala Ala Gly Leu Pro Gly Val Ala Gly Ala
305                 310                 315                 320

Pro Gly Leu Pro Gly Pro Arg Gly Ile Pro Gly Pro Val Gly Ala Ala
                325                 330                 335

Gly Ala Thr Gly Ala Arg Gly Leu Val Gly Glu Pro Gly Pro Ala Gly
            340                 345                 350

Ser Lys Gly Glu Ser Gly Asn Lys Gly Glu Pro Gly Ser Ala Gly Pro
        355                 360                 365

Gln Gly Pro Pro Gly Pro Ser Gly Glu Glu Gly Lys Arg Gly Pro Asn
    370                 375                 380

Gly Glu Ala Gly Ser Ala Gly Pro Pro Gly Pro Pro Gly Leu Arg Gly
385                 390                 395                 400

Ser Pro Gly Ser Arg Gly Leu Pro Gly AlaAsp Gly Arg Ala Gly Val
405 410 415

Met Gly Pro Pro Gly Ser Arg Gly Ala Ser Gly Pro Ala Gly Val Arg
420 425 430

Gly Pro Asn Gly Asp Ala Gly Arg Pro Gly Glu Pro Gly Leu Met Gly
435 440 445

Pro Arg Gly Leu Pro Gly Ser Pro Gly Asn Ile Gly Pro Ala Gly Lys
450 455 460

Glu Gly Pro Val Gly Leu Pro Gly Ile Asp Gly Arg Pro Gly Pro Ile
465 470 475 480

Gly Pro Ala Gly Ala Arg Gly Glu Pro Gly Asn Ile Gly Phe Pro Gly
485 490 495

Pro Lys Gly Pro Thr Gly Asp Pro Gly Lys Asn Gly Asp Lys Gly His
500 505 510

Ala Gly Leu Ala Gly Ala Arg Gly Ala Pro Gly Pro Asp Gly Asn Asn
515 520 525

Gly Ala Gln Gly Pro Pro Gly Pro Gln Gly Val Gln Gly Gly Lys Gly
530 535 540

Glu Gln Gly Pro Ala Gly Pro Pro Gly Phe Gln Gly Leu Pro Gly Pro
545 550 555 560

Ser Gly Pro Ala Gly Glu Val Gly Lys Pro Gly Glu Arg Gly Leu His
565 570 575

Gly Glu Phe Gly Leu Pro Gly Pro Ala Gly Pro Arg Gly Glu Arg Gly
580 585 590

Pro Pro Gly Glu Ser Gly Ala Ala Gly Pro Thr Gly Pro Ile Gly Ser
595 600 605

Arg Gly Pro Ser Gly Pro Pro Gly Pro Asp Gly Asn Lys Gly Glu Pro
610 615 620

Gly Val Val Gly Ala Val Gly Thr Ala Gly Pro Ser Gly Pro Ser Gly
625 630 635 640

Leu Pro Gly Glu Arg Gly Ala Ala Gly Ile Pro Gly Gly Lys Gly Glu
645 650 655

Lys Gly Glu Pro Gly Leu Arg Gly Glu Ile Gly Asn Pro Gly Arg Asp
660 665 670

Gly Ala Arg Gly Ala His Gly Ala Val Gly Ala Pro Gly Pro Ala Gly
675 680 685

Ala Thr Gly Asp Arg Gly Glu Ala Gly Ala Ala Gly Pro Ala Gly Pro
690 695 700

Ala Gly Pro Arg Gly Ser Pro Gly Glu Arg Gly Glu Val Gly Pro Ala
705 710 715 720

Gly Pro Asn Gly Phe Ala Gly Pro Ala Gly Ala Ala Gly Gln Pro Gly
725 730 735

Ala Lys Gly Glu Arg Gly Ala Lys Gly Pro Lys Gly Glu Asn Gly Val
740 745 750

Val Gly Pro Thr Gly Pro Val Gly Ala Ala Gly Pro Ala Gly Pro Asn
755 760 765

Gly Pro Pro Gly Pro Ala Gly Ser Arg Gly Asp Gly Gly Pro Pro Gly
770 775 780

Met Thr Gly Phe Pro Gly Ala Ala Gly Arg Thr Gly Pro Pro Gly Pro
785                 790                 795                 800

Ser Gly Ile Ser Gly Pro Pro Gly Pro Pro Gly Pro Ala Gly Lys Glu
            805                 810                 815

Gly Leu Arg Gly Pro Arg Gly Asp Gln Gly Pro Val Gly Arg Thr Gly
        820                 825                 830

Glu Val Gly Ala Val Gly Pro Pro Gly Phe Ala Gly Glu Lys Gly Pro
        835                 840                 845

Ser Gly Glu Ala Gly Thr Ala Gly Pro Pro Gly Thr Pro Gly Pro Gln
    850                 855                 860

Gly Leu Leu Gly Ala Pro Gly Ile Leu Gly Leu Pro Gly Ser Arg Gly
865                 870                 875                 880

Glu Arg Gly Leu Pro Gly Val Ala Gly Ala Val Gly Glu ProGly Pro
            885                 890                 895

Leu Gly Ile Ala Gly Pro Pro Gly Ala Arg Gly Pro Pro Gly Ala Val
        900                 905                 910

Gly Ser Pro Gly Val Asn Gly Ala Pro Gly Glu Ala Gly Arg Asp Gly
        915                 920                 925

Asn Pro Gly Asn Asp Gly Pro Pro Gly Arg Asp Gly Gln Pro Gly His
    930                 935                 940

Lys Gly Glu Arg Gly Tyr Pro Gly Asn Ile Gly Pro Val Gly Ala Ala
945                 950                 955                 960

Gly Ala Pro Gly Pro His Gly Pro Val Gly Pro Ala Gly Lys His Gly
            965                 970                 975

Asn Arg Gly Glu Thr Gly Pro Ser Gly Pro Val Gly Pro Ala Gly Ala
        980                 985                 990

Val Gly Pro Arg Gly Pro Ser Gly  Pro Gln Gly Ile Arg  Gly Asp Lys
      995                1000               1005

Gly Glu  Pro Gly Glu Lys Gly  Pro Arg Gly Leu Pro  Gly Leu Lys
   1010               1015               1020

Gly His  Asn Gly Leu Gln Gly  Leu Pro Gly Ile Ala  Gly His His
   1025               1030               1035

Gly Asp  Gln Gly Ala Pro Gly  Ser Val Gly Pro Ala  Gly Pro Arg
   1040               1045               1050

Gly Pro  Ala Gly Pro Ser Gly  Pro Ala Gly Lys Asp  Gly Arg Thr
   1055               1060               1065

Gly His  Pro Gly Thr Val Gly  Pro Ala Gly Ile Arg  Gly Pro Gln
   1070               1075               1080

Gly His  Gln Gly Pro Ala Gly  Pro Pro Gly Pro Pro  Gly Pro Pro
   1085               1090               1095

Gly Pro  Pro Gly Val Ser Gly  Gly Gly Tyr Asp Phe  Gly Tyr Asp
   1100               1105               1110

Gly Asp  Phe Tyr Arg Ala Asp  Gln Pro Arg Ser Ala  Pro Ser Leu
   1115               1120               1125

Arg Pro  Lys Asp Tyr Glu Val  Asp Ala Thr Leu Lys  Ser Leu Asn
   1130               1135               1140

Asn Gln  Ile Glu Thr Leu Leu  Thr Pro Glu Gly Ser  Arg Lys Asn
   1145               1150               1155

Pro Ala  Arg Thr Cys Arg Asp  Leu Arg Leu Ser His  Pro Glu Trp
   1160               1165               1170

Ser Ser  Gly Tyr Tyr Trp Ile  Asp Pro Asn Gln Gly  Cys Thr Met
   1175               1180               1185

Asp Ala Ile Lys Val Tyr Cys Asp Phe Ser Thr Gly Glu Thr Cys
1190 1195 1200

Ile Arg Ala Gln Pro Glu Asn Ile ProAla Lys Asn Trp Tyr Arg
1205 1210 1215

Ser Ser Lys Asp Lys Lys His Val Trp Leu Gly Glu Thr Ile Asn
1220 1225 1230

Ala Gly Ser Gln Phe Glu Tyr Asn Val Glu Gly Val Thr Ser Lys
1235 1240 1245

Glu Met Ala Thr Gln Leu Ala Phe Met Arg Leu Leu Ala Asn Tyr
1250 1255 1260

Ala Ser Gln Asn Ile Thr Tyr His Cys Lys Asn Ser Ile Ala Tyr
1265 1270 1275

Met Asp Glu Glu Thr Gly Asn Leu Lys Lys Ala Val Ile Leu Gln
1280 1285 1290

Gly Ser Asn Asp Val Glu Leu Val Ala Glu Gly Asn Ser Arg Phe
1295 1300 1305

Thr Tyr Thr Val Leu Val Asp Gly Cys Ser Lys Lys Thr Asn Glu
1310 1315 1320

Trp Gly Lys Thr Ile Ile Glu Tyr Lys Thr Asn Lys Pro Ser Arg
1325 1330 1335

Leu Pro Phe Leu Asp Ile Ala Pro Leu Asp Ile Gly Gly Ala Asp
1340 1345 1350

His Glu Phe Phe Val Asp Ile Gly Pro Val Cys Phe Lys
1355 1360 1365

<210> 25
<211> 20
<212> PRT
<213> Artificial Sequence

<220>
<223>  Query Sequence

<400>  25

Ala Ser Asn Pro Glu Arg Thr Met Ile Pro Val Thr Arg Val Gly Leu
1               5                   10                  15

Ile Arg Tyr Met
            20

<210>  26
<211>  19
<212>  PRT
<213>  Artificial Sequence

<220>
<223>  Individual Sequence

<400>  26

Tyr Met Met Thr Glu Tyr Leu Ala Ile Pro Val Arg Val Gly Leu Pro
1               5                   10                  15

Arg Tyr Met

## Description

The present invention provides nucleic acid sequences and proteins encoded thereby, as well as probes derived from the nucleic acid sequences, antibodies directed to the encoded proteins, and diagnostic methods for detecting cancerous cells, especially colon cancer cells.

Colorectal carcinoma is a malignant neoplastic disease. There is a high incidence of colorectal carcinoma in the Western world, particularly in the United States. Tumors of this type often metastasize through lymphatic and vascular channels. Many patients with colorectal carcinoma eventually die from this disease. In fact, it is estimated that 62,000 persons in the United States alone die of colorectal carcinoma annually.

However, if diagnosed early, colon cancer may be treated effectively by surgical removal of the cancerous tissue. Colorectal cancers originate in the colorectal epithelium and typically are not extensively vascularized (and therefore not invasive) during the early stages of development. Colorectal cancer is thought to result from the clonal expansion of a single mutant cell in the epithelial lining of the colon or rectum. The transition to a highly vascularized, invasive and ultimately metastatic cancer which spreads throughout the body commonly takes ten years or longer. If the cancer is detected prior to invasion, surgical removal of the cancerous tissue is an effective cure. However, colorectal cancer is often detected only upon manifestation of clinical symptoms, such as pain and black tarry stool. Generally, such symptoms are present only when the disease is well established, often after metastasis has occurred, and the prognosis for the patient is poor, even after surgical resection of the cancerous tissue. Early detection of colorectal cancer therefore is important in that detection may significantly reduce its morbidity.

Invasive diagnostic methods such as endoscopic examination allow for direct visual identification, removal, and biopsy of potentially cancerous growths such as polyps. Endoscopy is expensive, uncomfortable, inherently risky, and therefore not a practical tool for screening populations to identify those with colorectal cancer. Non-invasive analysis of stool samples for characteristics indicative of the presence of colorectal cancer or precancer is a preferred alternative for early diagnosis, but no known diagnostic method is available which reliably achieves this goal. A reliable, non-invasive, and accurate technique for diagnosing colon cancer at an early stage would help save many lives.

The present invention provides nucleic acid sequences and proteins encoded thereby, as well as probes derived from the nucleic acid sequences, antibodies directed to the encoded proteins, and diagnostic and prognostic methods for detecting and monitoring cancer, especially colon cancer. The sequences disclosure herein have been found to be differentially expressed in samples obtained from colon cancer cell lines and/or colon cancer tissue.

In one embodiment the invention provides a method of detecting the presence of colon cancer in an individual comprising obtaining a sample from said individual, and detecting the presence of at least one nucleic acid sequence comprising a nucleic acid sequence selected from the group consisting of SEQ ID Nos 1-8.

In one aspect, the step of detecting comprises: contacting the sample with a polynucleotide probe comprising at least 12 contiguous nucleotides of a sequence selected from the group consisting of a sequence comprising SEQ ID Nos 1-8 which is capable of hybridizing to a sequence selected from the group of a sequence comprising SEQ ID Nos 1-8; and detecting the hybridization of the polynucleotide probe to the sequence selected from the group of a sequence comprising SEQ ID Nos 1-8, wherein detection of hybridization is indicative of the presence of a nucleic acid sequence comprising a nucleic acid sequence selected from the group consisting of SEQ ID Nos 1-8.

The present invention also provides a method of detecting colon cancer in an individual comprising obtaining a sample from said individual, and detecting the presence of a nucleic acid sequence consisting of a nucleic acid sequence selected from the group consisting of SEQ ID Nos 9-16.

The step of detecting may comprise, for example, contacting the sample with a polynucleotide probe comprising at least 12 contiguous nucleotides of a sequence selected from the group consisting of a sequence comprising SEQ ID Nos 9-16 which is capable of hybridizing to a sequence selected from the group of a sequence comprising SEQ ID Nos 9-16; and detecting the hybridization of the polynucleotide probe to the sequence selected from the group of a sequence comprising SEQ ID Nos 9-16, wherein detection of hybridization is indicative of the presence of a nucleic acid sequence comprising a nucleic acid sequence selected from the group consisting of SEQ ID Nos 9-16.

In a further embodiment, the invention provides a method of detecting colon cancer in an individual comprising obtaining a sample from the individual, and detecting the presence in the sample of a polypeptide sequence comprising a polypeptide sequence selected from the group consisting of SEQ ID Nos 17-24.

The step of detecting may comprise, for example, contacting the patient sample with a polypeptide ligand which is capable of binding to one or more of SEQ ID Nos 17-24; and detecting the binding of said polypeptide ligand to one or more of SEQ ID Nos 17-24, wherein detection of binding is indicative of the presence of a polypeptide sequence comprising a polypeptide sequence selected from the group consisting of SEQ ID Nos 17-24 in said sample.

In a preferred embodiment, the polypeptide ligand is an antibody, or antibody fragment, and preferably includes a detectable label.

The methods of the invention may be carried out *in vitro.*

The invention further provides a nucleic acid comprising a nucleotide sequence comprising the sequence of SEQ ID Nos. 1-8 and 9-16, or a sequence complementary thereto. The sequence further may comprise a transcriptional regulatory sequence operably linked to the nucleotide sequence to render the nucleotide sequence suitable for use as an expression vector. The nucleic acid may be included in an expression vector capable of replicating in a prokaryotic or eukaryotic cell. The invention provides a host cell transfected with the expression vector.

The invention also provides a nucleic acid comprising a nucleotide sequence which hybridizes under stringent conditions to a sequence of SEQ ID Nos. 1-8 and 9-16, or a sequence complementary thereto. The sequence further may comprise a transcriptional regulatory sequence operably linked to the nucleotide sequence to render the nucleotide sequence suitable for use as an expression vector. The nucleic acid may be included in an expression vector capable of replicating in a prokaryotic or eukaryotic cell. In a related embodiment, the invention provides a host cell transfected with the expression vector.

The invention also provides a transgenic animal having a transgene of a nucleic acid comprising a nucleotide sequence comprising the sequence of SEQ ID Nos. 1-8 or 9-16, or a sequence complementary thereto, incorporated in cells thereof. The invention provides a transgenic animal having a transgene of a nucleic acid comprising a nucleic acid sequence which hybridizes under stringent conditions to a sequence comprising the sequence of SEQ ID Nos 1-8 or 9-16. The transgene modifies the level of expression of the nucleic acid, the stability of a mRNA transcript of the nucleic acid, or the activity of the encoded product of the nucleic acid.

The invention also provides an isolated substantially pure nucleic acid corresponding to at least about 8, at least about 12, at least about 15, at least about 25, or at least about 40 consecutive nucleotides up to the full length of one of SEQ ID Nos. 1-8 or 9-16, or a sequence complementary thereto or up to the full length of the gene of which said sequence is a fragment. In another aspect, the invention provides a substantially pure nucleic acid which hybridizes under stringent conditions to a nucleic acid probe corresponding to at least about 8, at least about 12, at least about 15, at least about 25, or at least about 40 consecutive nucleotides up to the full length of one of SEQ ID Nos. 1-8 or 9-16, or a sequence complementary thereto or up to the full length of the gene of which said sequence is a fragment. The invention also provides an antisense oligonucleotide analog which comprises at least about 8, at least about 12, at least about 25, or at least about 40 consecutive nucleotides of one of SEQ ID Nos. 1-8 or 9-16 or a sequence complementary thereto, and which is resistant to cleavage by a nuclease, preferably an endogenous endonuclease or exonuclease. The invention also provides an antisense oligonucleotide analog which hybridizes under stringent conditions to at least about 8, at least about 12, at least about 25, or at least about 40 consecutive nucleotides of one of SEQ ID Nos. 1-8 or 9-16 or a sequence complementary thereto, and which is resistant to cleavage by a nuclease, preferably an endogenous endonuclease or exonuclease.

The invention further provides a probe/primer comprising a substantially purified oligonucleotide, said oligonucleotide comprising a region of a nucleic acid sequence of SEQ ID Nos. 1-8 or 9-16 sufficient to hybridize with a nucleic acid substantially complementary to the sequence of SEQ ID Nos. 1-8 or 9-16, respectively, and containing a region of nucleotide sequence which comprises at least about 8 at least about 12, at least about 15, at least about 25, or at least about 40 consecutive nucleotides of sense or antisense sequence selected from SEQ ID Nos. 1-8 or 9-16 up to the full length of one of SEQ ID Nos. 1-8 or 9 -16 or a sequence complementary thereto. The probe/primer may comprise a substantially purified oligonucleotide, wherein the oligonucleotide comprises a region of nucleic acid sequence complementary to the sequence of SEQ ID Nos. 1-8 or 9-16 sufficient to hybridize with a sequence of SEQ ID Nos 1-8 or 9-16, respectively. In preferred embodiments, the probe selectively hybridizes with a target nucleic acid. In another embodiment, the probe may include a label group attached thereto and able to be detected. The label group may be selected from radioisotopes, fluorescent compounds, enzymes, and enzyme co-factors. The invention further provides arrays of at least about 10, at least about 25, at least about 50, or at least about 100 different probes as described above attached to a solid support.

As used herein, "sufficient to hybridize" refers to conditions, including the degree of complementarity between two nucleic acid sequences, which are sufficient to permit annealing of the two sequences under stringent hybridization conditions. Stringent hybridization conditions are well known to those of skill in the art and may be found in numerous scientific texts and laboratory manuals (see, for example, Maniatis et al., 1982 Molecular Cloning: A Laboratory Manual, Cold Spring Harbor Press, Inc.). Other conditions which may affect whether two sequences are sufficient to hybridize include G/C content, melting temperature and sequence length. Preferably, sequence which are "sufficient to hybridize" are at least 8 nucleotides in length, and have a G/C content no greater than about 50%.

The invention also pertains to a method of determining the phenotype of a cell, comprising detecting the differential expression, relative to a normal cell, of at least one nucleic acid comprising SEQ ID Nos. 1-8, wherein the nucleic acid is differentially expressed by at least about 1.5 fold, at least about 2 fold, at least about 10 fold, or at least up to or greater than about 50 fold. "Differential expression" as used herein refers to an increase or decrease in expression of a nucleic acid sequence of at least about 1.5 fold, at least about 2 fold, at least about 10 fold, or at least up to or greater than about 50 fold. In a preferred embodiment a sequence of one or more of SEQ ID Nos 1-8 or 9-16 is differentially expressed if it is over or under expressed by an average of at least about 1.5 fold as measured in at least two individuals with colon cancer relative to the average expression level in at least two normal individuals. The method of determining the phenotype of a cell may comprise detecting the differential expression, relative to a normal cell, of at least one nucleic acid which hybridizes under stringent conditions one of SEQ ID Nos. 1-8, wherein the nucleic acid is differentially expressed by at least at least about 1.5 fold, at least about 2 fold, at least about 10 fold, or at least up to or greater than about 50 fold. Alternatively, the method of determining the phenotype of a cell may comprise detecting the differential expression, relative to a normal cell, of at least one nucleic acid comprising SEQ ID Nos 9-16.

The present invention provides polypeptides encoded by the subject nucleic acids. The polypeptides may comprise the sequence of SEQ ID Nos. 17-24, or a fragment thereof. In another embodiment, the invention pertains to a polypeptide including an amino acid sequence encoded by a nucleic acid comprising a nucleotide sequence of SEQ ID Nos. 9-16 or a sequence complementary thereto, or a fragment thereof. The fragments may comprise, for example, at least about 10, at least about 20, at least about 30, or at least about 40 amino acids of the present polypeptides.

Further provided are antibodies which specifically bind to a polypeptide comprising the sequence of SEQ ID Nos. 17-24, or a fragment thereof. The invention also pertains to antibodies which bind to a polypeptide comprising an amino acid sequence encoded by a nucleic acid comprising a nucleotide sequence of SEQ ID Nos. 9-16, or a sequence complementary thereto, or a fragment thereof. The fragments bound by the antibodies of the invention may comprise, for example, at least about 10, at least about 20, at least about 30, or at least about 40 amino acids of the present polypeptides.

The invention provides diagnostic and prognostic methods. In one embodiment, the invention pertains to a method for determining the phenotype of cells from a patient by providing a nucleic acid probe comprising a nucleotide sequence having at least about 8, at least about 12, at least about 15, at least about 25, or at least about 40 consecutive nucleotides represented in a sequence of SEQ ID Nos. 1-8 or 9-16 or a sequence complementary thereto or up to the full length of the gene of which said sequence is a fragment, obtaining a sample of cells from a patient, providing a second sample of cells substantially all of which are non-cancerous, contacting the nucleic acid probe under stringent conditions with mRNA of each of said first and second cell samples, and comparing (a) the amount of hybridization of the probe with mRNA of the first cell sample, with (b) the amount of hybridization of the probe with mRNA of the second cell sample, wherein a difference of at least about 0.5 fold, at least about 1 fold, at least about 2 fold, or at least up to about 50 fold in the amount of hybridization with the mRNA of the first cell sample as compared to the amount of hybridization with the mRNA of the second cell sample is indicative of the phenotype of cells in the first cell sample. Determining the phenotype includes determining the genotype, as the term is used herein.

The present invention provides a test kit for identifying transformed (i.e., malignant) cells, comprising a probe/primer as described above, for measuring a level of a nucleic acid comprising a nucleic acid of SEQ ID Nos. 1-8 or 9-16, or a sequence complementary thereto in a sample of cells isolated from a patient. The kit may further include instructions for using the kit, compositions for suspending or fixing the cells, detectable tags or labels, compositions for rendering a nucleic acid susceptible to hybridization, compositions for lysing cells, or compositions for the purification of nucleic acids.

The invention also provides a method of determining the phenotype of a cell, comprising detecting the differential expression, relative to a normal cell, of at least one protein of SEQ ID Nos. 17-24, wherein the protein is differentially expressed by at least about 1.5 fold, at least about 2 fold, at least about 10 fold, at least about 20 fold, or at least or more than about 50 fold. "Differential expression" as used herein refers to an increase or decrease in expression of an amino acid sequence of at least about 1.5 fold, at least about 2 fold, at least about 10 fold, or at least up to or greater than about 50 fold. In a preferred embodiment a sequence of one or more of SEQ ID Nos 17-24 is differentially expressed if it is over or under expressed by an average of at least about 1.5 fold as measured in at least two individuals with colon cancer relative to the average expression level in at least two normal individuals. In another embodiment, the invention provides a method of determining the phenotype of a cell, comprising detecting the differential expression, relative to a normal cell, of at least one protein of SEQ ID Nos. 17-24, wherein the protein is differentially expressed by at least about 0.5 fold, at least about 2 fold, at least about 5 fold, at least about 20 fold, or at least about 50 fold. In one embodiment, the level of the protein is detected in an immunoassay.

The invention further pertains to a method for determining the presence or absence of a nucleic acid comprising one of SEQ ID Nos. 1-8 or 9-16, or a sequence complementary thereto in a cell, comprising contacting the cell with a probe as described above. The invention further provides a method for determining the presence or absence of a subject polypeptide of SEQ ID Nos. 17-24 in a cell, comprising contacting the cell with an antibody as described above. In yet another embodiment, the invention provides a method for determining the presence of an aberrant mutation (e.g., deletion, insertion, or substitution of nucleic acids) or aberrant methylation in a gene comprising a sequence of SEQ ID Nos. 1-8 or 9-16 or a sequence complementary thereto, comprising collecting a sample of cells from a patient, isolating nucleic acid from the cells of the sample, contacting the nucleic acid sample with one or more primers which specifically hybridize to a nucleic acid sequence of SEQ ID Nos. 1-8 or 9-16 under conditions such that hybridization and amplification of the nucleic acid occurs, and comparing the presence, absence, or size of an amplification product to the amplification product of a normal cell.

The invention provides a test kit for identifying transformed (i.e., malignant) cells, comprising an antibody specific for a protein encoded by a nucleic acid comprising any one of SEQ Nos. 1-8 or 9-16, or a sequence complementary thereto. The kit may further include instructions for using the kit, compositions for suspending or fixing the cells, detectable tags or labels, compositions for rendering a polypeptide susceptible to the binding of an antibody, compositions for lysing cells, or compositions for the purification of polypeptides.

In yet another aspect, the invention provides pharmaceutical compositions including the subject nucleic acids. An agent which alters the level of expression in a cell of a nucleic acid comprising one of SEQ ID Nos. 1-8 or 9 -16, or a sequence complementary thereto, is identified by providing a cell, treating the cell with a test agent, determining the level of expression in the cell of a nucleic acid of SEQ ID Nos. 1-8 or 9-16, or a sequence complementary thereto, and comparing the level of expression of the nucleic acid in the treated cell with the level of expression of the nucleic acid in an untreated cell, wherein a change in the level of expression of the nucleic acid in the treated cell relative to the level of expression of the nucleic acid in the untreated cell is indicative of an agent which alters the level of expression of the nucleic acid in a cell. The invention further provides a pharmaceutical composition comprising an agent identified by this method.

The invention also provides a pharmaceutical composition which includes a polypeptide either encoded by a nucleic acid having a nucleotide sequence comprising one of SEQ ID Nos. 1-8 or 9-16, or a sequence complementary thereto, or having the sequence of SEQ ID Nos. 17-24. In one embodiment, the invention pertains to a pharmaceutical composition comprising a nucleic acid including a sequence which hybridizes under stringent conditions to one of SEQ ID Nos. 1-8 or 9-16 or a sequence complementary thereto. Pharmaceutical compositions, useful in the present invention may further include fusion proteins comprising the amino acid sequence of SEQ ID Nos 17-24, or a fragment thereof, antibodies, or antibody fragments.

### Brief Description of the Figures

Figure 1 shows the nucleic acid sequence of SEQ ID Nos: 1-8 and 9-16.
Figure 2 shows the amino acid sequence of SEQ ID Nos: 17-24.
Figure 3 shows the differential expression of TGFβi in patient colon cancer samples.

The invention relates to nucleic acids comprising the sequence of SEQ ID Nos 1-8 and having the full length cDNA sequence of SEQ ID Nos. 9-16, respectively, or a sequence complementary thereto, and genes corresponding to these sequences, and to polypeptides and proteins encoded by these nucleic acids and genes, and portions thereof.

The invention further relates to the polypeptide sequences encoded by the mRNA sequence complementary to the cDNA sequence of SEQ ID Nos. 9-16, respectively including, but not limited to polypeptides having the amino acid sequence of SEQ ID Nos. 17-24.

Polypeptides and proteins that are variants of the polypeptides and proteins of SEQ ID Nos. 17-24 also are within the scope of the invention. The variants may differ from the wild-type protein in having one or more amino acid substitutions that enhance, add, or diminish a biological activity of the wild-type protein. Once the amino acid change is selected, a nucleic acid encoding that variant is constructed according to the invention. Such nucleic acids, which are variants of SEQ ID Nos 9-16, are encompassed by the invention.

The following detailed description discloses how to obtain or make cDNA and human genes corresponding to the present nucleic acids, how to express the nucleic acids and related genes, how to identify structural motifs of the genes, how to identify the function of a protein encoded by a gene corresponding to an nucleic acid, how to use nucleic acids as probes in mapping and in tissue profiling, how to use the corresponding polypeptides and proteins to raise antibodies, and how to use the nucleic acids, polypeptides, and proteins for therapeutic and diagnostic purposes.

Accordingly, certain aspects of the present invention relate to nucleic acids differentially expressed in tumor tissue, especially colon cancer cell lines, polypeptides encoded by such nucleic acids, antibodies immunoreactive with these polypeptides, and preparations of such compositions. Moreover, the present invention provides diagnostic and therapeutic assays, reagents, and compositions for detecting and treating disorders involving, for example, aberrant expression of the subject nucleic acids.

This invention relates in part to novel methods for identifying and/or classifying cancerous cells present in a human tumors, particularly in solid tumors, e.g., carcinomas and sarcomas, such as, for example, breast or colon cancers. The method uses genes that are differentially expressed in cancer cell lines and/or cancer tissue compared with related normal cells, such as normal colon cells, and thereby identifies or classifies tumor cells by the upregulation and/or downregulation of expression of particular genes, an event which is implicated in tumorigenesis.

Upregulation or increased expression of certain genes such as oncogenes, act to promote malignant growth. Downregulation or decreased expression of genes such as tumor suppressor genes also promotes malignant growth. Thus, alteration in the expression of either type of gene is a potential diagnostic indicator for determining whether a subject is at risk of developing or has cancer, e.g., colon cancer.

Accordingly, in one aspect, the invention also provides biomarkers, such as nucleic acid markers, for human tumor cells, e.g., for colon cancer cells. The invention also provides proteins encoded by these nucleic acid markers. The invention also features methods for identifying drugs useful for treatment of such cancer cells, and for treatment of a cancerous condition, such as colon cancer. Unlike prior methods, the invention provides a means for identifying cancer cells at an early stage of development, so that premalignant cells can be identified prior to their spreading throughout the human body. This allows early detection of potentially cancerous conditions, and treatment of those cancerous conditions prior to spread of the cancerous cells throughout the body, or prior to development of an irreversible cancerous condition.

For convenience, the meaning of certain terms and phrases used in the specification, examples, and appended claims, are provided below.

The term "an aberrant expression", as applied to a nucleic acid of the present invention, refers to level of expression of that nucleic acid which differs from the level of expression of that nucleic acid in healthy tissue, or which differs from the activity of the polypeptide present in a healthy subject. An activity of a polypeptide can be aberrant because it is stronger than the activity of its native counterpart. Alternatively, an activity can be aberrant because it is weaker or absent relative to the activity of its native counterpart. An aberrant activity can also be a change in the activity; for example, an aberrant polypeptide can interact with a different target peptide. A cell can have an aberrant expression level of a gene due to overexpression or underexpression of that gene.

The term "agonist", as used herein, is meant to refer to an agent that mimics or upregulates (e.g., potentiates or supplements) the bioactivity of a protein. An agonist can be a wild-type protein or derivative thereof having at least one bioactivity of the wild-type protein. An agonist can also be a compound that upregulates expression of a gene or which increases at least one bioactivity of a protein. An agonist can also be a compound which increases the interaction of a polypeptide with another molecule, e.g., a target peptide or nucleic acid.

The term "allele", which is used interchangeably herein with "allelic variant", refers to alternative forms of a gene or portions thereof. Alleles occupy the same locus or position on homologous chromosomes. When a subject has two identical alleles of a gene, the subject is said to be homozygous for that gene or allele. When a subject has two different alleles of a gene, the subject is said to be heterozygous for the gene. Alleles of a specific gene can differ from each other in a single nucleotide, or several nucleotides, and can include substitutions, deletions, and/or insertions of nucleotides. An allele of a gene can also be a form of a gene containing mutations.

The term "allelic variant of a polymorphic region of a gene" refers to a region of a gene having one of several nucleotide sequences found in that region of the gene in other individuals.

"Antagonist" as used herein is meant to refer to an agent that downregulates (e.g., suppresses or inhibits) at least one bioactivity of a protein. An antagonist can be a compound which inhibits or decreases the interaction between a protein and another molecule, e.g., a target peptide or enzyme substrate. An antagonist can also be a compound that downregulates expression of a gene or which reduces the amount of expressed protein present.

The term "antibody" as used herein is intended to include whole antibodies, e.g., of any isotype (IgG, IgA, IgM, IgE, etc), and includes fragments thereof, and single-chain antibodies, which also are specifically reactive with a vertebrate, e.g., mammalian, protein. Antibodies can be fragmented using conventional techniques and the fragments screened for utility in the same manner as described above for whole antibodies. Thus, the term includes segments of proteolytically-cleaved or recombinantly-prepared portions of an antibody molecule that are capable of selectively reacting with a certain protein. Nonlimiting examples of such proteolytic and/or recombinant fragments include Fab, F(ab')2, Fab' , Fv, and single chain antibodies (scFv) containing a V[L] and/or V[H] domain joined by a peptide linker. The scFv's may be covalently or non-covalently linked to form antibodies having two or more binding sites. The subject invention includes polyclonal, monoclonal, or other purified preparations of antibodies and recombinant antibodies.

The phenomenon of "apoptosis" is well known, and can be described as a programmed death of cells. As is known, apoptosis is contrasted with "necrosis", a phenomenon when cells die as a result of being killed by a toxic material, or other external effect. Apoptosis involves chromatic condensation, membrane blebbing, and fragmentation of DNA, all of which are generally visible upon microscopic examination.

A disease, disorder, or condition "associated with" or "characterized by" an aberrant expression of a nucleic acid refers to a disease, disorder, or condition in a subject which is caused by, contributed to by, or causative of an aberrant level of expression of a nucleic acid.

As used herein the term "bioactive fragment of a polypeptide" refers to a fragment of a full-length polypeptide, wherein the fragment specifically agonizes (mimics) or antagonizes (inhibits) the activity of a wild-type polypeptide. The bioactive fragment preferably is a fragment capable of interacting with at least one other molecule, e.g., protein, small molecule, or DNA, which a full length protein can bind.

"Biological activity" or "bioactivity" or "activity" or "biological function", which are used interchangeably, herein mean an effector or antigenic function that is directly or indirectly performed by a polypeptide (whether in its native or denatured conformation), or by any subsequence thereof. Biological activities include binding to polypeptides, binding to other proteins or molecules, activity as a DNA binding protein, as a transcription regulator, ability to bind damaged DNA, etc. A bioactivity can be modulated by directly affecting the subject polypeptide. Alternatively, a bioactivity can be altered by modulating the level of the polypeptide, such as by modulating expression of the corresponding gene.

The term "biomarker" refers a biological molecule, e.g., a nucleic acid, peptide, hormone, etc., whose presence or concentration can be detected and correlated with a known condition, such as a disease state.

"Cells," "host cells", or "recombinant host cells" are terms used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A "chimeric polypeptide" or "fusion polypeptide" is a fusion of a first amino acid sequence encoding one of the subject polypeptides with a second amino acid sequence defining a domain (e.g., polypeptide portion) foreign to and not substantially homologous with any domain of the subject polypeptide. A chimeric polypeptide may present a foreign domain which is found (albeit in a different polypeptide) in an organism which also expresses the first polypeptide, or it may be an "interspecies," "intergenic," etc., fusion of polypeptide structures expressed by different kinds of organisms. In general, a fusion polypeptide can be represented by the general formula (X)ₙ-(Y)ₘ-(Z)ₙ, wherein Y represents a portion of the subject polypeptide, and X and Z are each independently absent or represent amino acid sequences which are not related to the native sequence found in an organism, or which are not found as a polypeptide chain contiguous with the subject sequence, where m is an integer greater than or equal to one, and each occurrence of n is, independently, 0 or an integer greater than or equal to 1 (n and m are preferably no greater than 5 or 10).

A "delivery complex" shall mean a targeting means (e.g., a molecule that results in higher affinity binding of a nucleic acid, protein, polypeptide or peptide to a target cell surface and/or increased cellular or nuclear uptake by a target cell). Examples of targeting means include: sterols (e.g., cholesterol), lipids (e.g., a cationic lipid, virosome or liposome), viruses (e.g., adenovirus, adeno-associated virus, and retrovirus), or target cell-specific binding agents (e.g., ligands recognized by target cell specific receptors). Preferred complexes are sufficiently stable in vivo to prevent significant uncoupling prior to internalization by the target cell.
However, the complex is cleavable under appropriate conditions within the cell so that the nucleic acid, protein, polypeptide or peptide is released in a functional form.

As is well known, genes or a particular polypeptide may exist in single or multiple copies within the genome of an individual. Such duplicate genes may be identical or may have certain modifications, including nucleotide substitutions, additions or deletions, which all still code for polypeptides having substantially the same activity. The term "DNA sequence encoding a polypeptide" may thus refer to one or more genes within a particular individual. Moreover, certain differences in nucleotide sequences may exist between individual organisms, which are called alleles. Such allelic differences may or may not result in differences in amino acid sequence of the encoded polypeptide yet still encode a polypeptide with the same biological activity.

The term "equivalent" is understood to include nucleotide sequences encoding functionally equivalent polypeptides. Equivalent nucleotide sequences will include sequences that differ by one or more nucleotide substitutions, additions or deletions, such as allelic variants; and will, therefore, include sequences that differ from the nucleotide sequence of the nucleic acids shown in SEQ ID NOs: 1-8 or 9-16 due to the degeneracy of the genetic code.

As used herein, the terms "gene", "recombinant gene", and "gene construct" refer to a nucleic acid of the present invention associated with an open reading frame, including both exon and (optionally) intron sequences.

A "recombinant gene" refers to nucleic acid encoding a polypeptide and comprising exon sequences, though it may optionally include intron sequences which are derived from, for example, a related or unrelated chromosomal gene. The term "intron" refers to a DNA sequence present in a given gene which is not translated into protein and is generally found between exons.

The term "growth" or "growth state" of a cell refers to the proliferative state of a cell as well as to its differentiative state. Accordingly, the term refers to the phase of the cell cycle in which the cell is, e.g., G0, G1, G2, prophase, metaphase, or telophase, as well as to its state of differentiation, e.g., undifferentiated, partially differentiated, or fully differentiated. Without wanting to be limited, differentiation of a cell is usually accompanied by a decrease in the proliferative rate of a cell.

"Homology" or "identity" or "similarity" refers to sequence similarity between two peptides or between two nucleic acid molecules, with identity being a more strict comparison. Homology and identity can each be determined by comparing a position in each sequence which may be aligned for purposes of comparison. When a position in the compared sequence is occupied by the same base or amino acid, then the molecules are identical at that position. A degree of homology or similarity or identity between nucleic acid sequences is a function of the number of identical or matching nucleotides at positions shared by the nucleic acid sequences. A degree of identity of amino acid sequences is a function of the number of identical amino acids at positions shared by the amino acid sequences. A degree of homology or similarity of amino acid sequences is a function of the number of amino acids, i.e., structurally related, at positions shared by the amino acid sequences. An "unrelated" or "non-homologous" sequence shares less than 40% identity, though preferably less than 25% identity, with one of the sequences of the present invention.

The term "percent identical" refers to sequence identity between two amino acid sequences or between two nucleotide sequences. Identity can each be determined by comparing a position in each sequence which may be aligned for purposes of comparison. When an equivalent position in the compared sequences is occupied by the same base or amino acid, then the molecules are identical at that position; when the equivalent site occupied by the same or a similar amino acid residue (e.g., similar in steric and/or electronic nature), then the molecules can be referred to as homologous (similar) at that position. Expression as a percentage of homology, similarity, or identity refers to a function of the number of identical or similar amino acids at positions shared by the compared sequences. Various alignment algorithms and/or programs may be used, including FASTA, BLAST, or ENTREZ. FASTA and BLAST are available as a part of the GCG sequence analysis package (University of Wisconsin, Madison, Wis.), and can be used with, e.g., default settings. ENTREZ is available through the National Center for Biotechnology Information, National Library of Medicine, National Institutes of Health, Bethesda, Md. In one embodiment, the percent identity of two sequences can be determined by the GCG program with a gap weight of 1, e.g., each amino acid gap is weighted as if it were a single amino acid or nucleotide mismatch between the two sequences.

Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, California, USA. Preferably, an alignment program that permits gaps in the sequence is utilized to align the sequences. The Smith-Waterman is one type of algorithm that permits gaps in sequence alignments. See Meth. Mol. 70-187 (1997). Also, the GAP program using the Needleman and Wunsch alignment method can be utilized to align sequences. An alternative search strategy uses MPSRCH software, which runs on a MASPAR computer. MPSRCH uses a Smith-Waterman algorithm to score sequences on a massively parallel computer. This approach improves ability to pick up distantly related matches, and is especially tolerant of small gaps and nucleotide sequence errors. Nucleic acid-encoded amino acid sequences can be used to search both protein and DNA databases.

Databases with individual sequences are described in Methods in Enzymology. ed. Doolittle, supra. Databases include Genbank, EMBL, and DNA Database of Japan (DDBJ).

Preferred nucleic acids have a sequence at least about 70%, and more preferably at least about 80% identical and more preferably at least about 90% and even more preferably at least about 95% identical to an nucleic acid sequence of a sequence shown in one of SEQ ID NOS: 1-8 or 9-16. Nucleic acids at least about 90%, more preferably about 95%, and most preferably at least about 98 -99% identical with a nucleic sequence represented in one of SEQ ID NOS: 1-8 or 9 -16 are of course also within the scope of the invention. In preferred embodiments, the nucleic acid is of mammalian origin.

The term "interact" as used herein is meant to include detectable interactions (e.g., biochemical interactions) between molecules, such as interaction between protein-protein, protein-nucleic acid, nucleic acid-nucleic acid, and protein-small molecule or nucleic acid-small molecule in nature.

The term "isolated" as used herein with respect to nucleic acids, such as DNA or RNA, refers to molecules separated from other DNAS, or RNAs, respectively, that are present in the natural source of the macromolecule. The term isolated as used herein also refers to a nucleic acid or peptide that is substantially pure, i.e., free of cellular material, viral material, or culture medium when produced by recombinant DNA techniques, or chemical precursors or other chemicals when chemically synthesized. Moreover, an "isolated nucleic acid" is meant to include substantially pure and/or purified nucleic acid fragments which are not naturally occurring as fragments and would not be found in the natural state. The term "isolated" is also used herein to refer to polypeptides which are isolated from other cellular proteins and is meant to encompass both substantially purified and recombinant polypeptides.

The terms "modulated" and "differentially regulated" as used herein refer to both upregulation (i.e., activation or stimulation (e.g., by agonizing or potentiating) and downregulation (i.e., inhibition or suppression (e.g., by antagonizing, decreasing or inhibiting)).

The term "mutated gene" refers to an allelic form of a gene, which is capable of altering the phenotype of a subject having the mutated gene relative to a subject which does not have the mutated gene. If a subject must be homozygous for this mutation to have an altered phenotype, the mutation is said to be recessive. If one copy of the mutated gene is sufficient to alter the genotype of the subject, the mutation is said to be dominant. If a subject has one copy of the mutated gene and has a phenotype that is intermediate between that of a homozygous and that of a heterozygous subject (for that gene), the mutation is said to be co-dominant.

The designation "N", where it appears in the accompanying Sequence Listing, indicates that the identity of the corresponding nucleotide is unknown. "N" should therefore not necessarily be interpreted as permitting substitution with any nucleotide, e.g., A, T, C, or G, but rather as holding the place of a nucleotide whose identity has not been conclusively determined.

The "non-human animals" of the invention include mammalians such as rodents, non-human primates, sheep, dog, cow, chickens, amphibians, reptiles, etc. Preferred non-human animals are selected from the rodent family including rat and mouse, most preferably mouse, though transgenic amphibians, such as members of the Xenopus genus, and transgenic chickens can also provide important tools for understanding and identifying agents which can affect, for example, embryogenesis and tissue formation. The term "chimeric animal" is used herein to refer to animals in which the recombinant gene is found, or in which the recombinant gene is expressed in some but not all cells of the animal. The term "tissue-specific chimeric animal" indicates that one of the recombinant genes is present and/or expressed or disrupted in some tissues but not others.

As used herein, the term "nucleic acid" refers to polynucleotides such as deoxyribonucleic acid (DNA), and, where appropriate, ribonucleic acid (RNA). The term should also be understood to include, as equivalents, analogs of either RNA or DNA made from nucleotide analogs, and, as applicable to the embodiment being described, single (sense or antisense) and double-stranded polynucleotides. ESTs, chromosomes, cDNAs, mRNAs, and rRNAs are representative examples of molecules that may be referred to as nucleic acids.

The term "nucleotide sequence complementary to the nucleotide sequence of SEQ ID NO. x" refers to the nucleotide sequence of the complementary strand of a nucleic acid strand having SEQ ID NO. x. The term "complementary strand" is used herein interchangeably with the term "complement". The complement of a nucleic acid strand can be the complement of a coding strand or the complement of a non-coding strand.

The term "polymorphism" refers to the coexistence of more than one form of a gene or portion (e.g., allelic variant) thereof. A portion of a gene of which there are at least two different forms, i.e., two different nucleotide sequences, is referred to as a "polymorphic region of a gene". A polymorphic region can be a single nucleotide, the identity of which differs in different alleles. A polymorphic region can also be several nucleotides long.

A "polymorphic gene" refers to a gene having at least one polymorphic region.

As used herein, the term "promoter" means a DNA sequence that regulates expression of a selected DNA sequence operably linked to the promoter, and which effects expression of the selected DNA sequence in cells. The term encompasses "tissue specific" promoters, i.e., promoters which effect expression of the selected DNA sequence only in specific cells (e.g., cells of a specific tissue). The term also covers so-called "leaky" promoters, which regulate expression of a selected DNA primarily in one tissue, but cause expression in other tissues as well. The term also encompasses non-tissue specific promoters and promoters that constitutively expressed or that are inducible (i.e., expression levels can be controlled).

The terms "protein", "polypeptide", and "peptide" are used interchangeably herein when referring to a gene product.

The term "recombinant protein" refers to a polypeptide of the present invention which is produced by recombinant DNA techniques, wherein generally, DNA encoding a polypeptide is inserted into a suitable expression vector which is in turn used to transform a host cell to produce the heterologous protein. Moreover, the phrase "derived from", with respect to a recombinant gene, is meant to include within the meaning of "recombinant protein" those proteins having an amino acid sequence of a native polypeptide, or an amino acid sequence similar thereto which is generated by mutations including substitutions and deletions (including truncation) of a naturally occurring form of the polypeptide.

"Small molecule" as used herein, is meant to refer to a composition, which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

As used herein, the term "specifically hybridizes" or "specifically detects" refers to the ability of a nucleic acid molecule of the invention to hybridize to at least a portion of, for example approximately 6, 12, 15, 20, 30, 50, 100, 150, 200, 300, 350, 400, 500, 750, or 1000 contiguous nucleotides of a nucleic acid designated in any one of SEQ ID Nos 1, 3, 5, or 7, or a sequence complementary thereto, or naturally occurring mutants thereof, such that it has less than about 15%, preferably less than about 10%, and more preferably less than about 5% background hybridization to a cellular nucleic acid (e.g., mRNA or genomic DNA) encoding a different protein. In preferred embodiments, the oligonucleotide probe detects only a specific nucleic acid, e.g., it does not substantially hybridize to similar or related nucleic acids, or complements thereof.

"Transcriptional regulatory sequence" is a generic term used throughout the specification to refer to DNA sequences, such as initiation signals, enhancers, and promoters, which induce or control transcription of protein coding sequences with which they are operably linked. In preferred embodiments, transcription of one of the genes is under the control of a promoter sequence (or other transcriptional regulatory sequence) which controls the expression of the recombinant gene in a cell-type in which expression is intended. It will also be understood that the recombinant gene can be under the control of transcriptional regulatory sequences which are the same or which are different from those sequences which control transcription of the naturally occurring forms of the polypeptide.

As used herein, the term "transfection" means the introduction of a nucleic acid, e.g., via an expression vector, into a recipient cell by nucleic acid-mediated gene transfer. "Transformation", as used herein, refers to a process in which a cell's genotype is changed as a result of the cellular uptake of exogenous DNA or RNA, and, for example, the transformed cell expresses a recombinant form of a polypeptide or, in the case of anti-sense expression from the transferred gene, the expression of the target gene is disrupted.

As used herein, the term "transgene" means a nucleic acid sequence (or an antisense transcript thereto) which has been introduced into a cell. A transgene could be partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can also be present in a cell in the form of an episome. A transgene can include one or more transcriptional regulatory sequences and any other nucleic acid, such as introns, that may be necessary for optimal expression of a selected nucleic acid.

A "transgenic animal" refers to any animal, preferably a non-human mammal, bird or an amphibian, in which one or more of the cells of the animal contain heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical crossbreeding, or in vitro fertilization, but rather is directed to the introduction of a recombinant DNA molecule. This molecule may be integrated within a chromosome, or it may be extra-chromosomally replicating DNA. In the typical transgenic animals described herein, the transgene causes cells to express a recombinant form of one of the subject polypeptide, e.g. either agonistic or antagonistic forms. However, transgenic animals in which the recombinant gene is silent are also contemplated, as for example, the FLP or CRE recombinase dependent constructs described below. Moreover, "transgenic animal" also includes those recombinant animals in which gene disruption of one or more genes is caused by human intervention, including both recombination and antisense techniques.

The term "treating" as used herein is intended to encompass curing as well as ameliorating at least one symptom of the condition or disease.

The term "vector" refers to a nucleic acid molecule capable of transporting another nucleic acid to which it has been linked. One type of preferred vector is an episome, i.e., a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer generally to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

The term "wild-type allele" refers to an allele of a gene which, when present in two copies in a subject results in a wild-type phenotype. There can be several different wild-type alleles of a specific gene, since certain nucleotide changes in a gene may not affect the phenotype of a subject having two copies of the gene with the nucleotide changes.

As described below, one aspect of the invention pertains to isolated nucleic acids, variants, and/or equivalents of such nucleic acids.

Nucleic acids of the present invention have been identified as differentially expressed in tumor cells and/or tissue, e.g., colon cancer-derived cell lines (relative to the expression levels in normal tissue, e.g., normal colon tissue and/or normal non-colon tissue). The method used, in one embodiment of the invention, to identify differentially express nucleic acid sequences relies on a process in which full length mRNA molecules are digested into smaller pieces for analysis by subtractive hybridization and selective amplification. That is, the sequences of SEQ ID Nos 1-8, which have been identified as being differentially expressed according to the present invention, and are a truncated form of the full length sequences of the genes represented by SEQ ID Nos 9-16 (full length sequences), respectively. Thus, the full length sequences were obtained prior to the shorter sequences and, in fact, used to generate the shorter sequences for differential expression analysis. The process for differential expression analysis was performed using the PCR-Select™ subtraction methods described by BD Biosciences Clontech. Briefly, the SMART™ system (BD Biosciences) may be used to generate full length mRNA from total cellular RNA sample, which is then used to generate a full length cDNA library. Subsequently, the full length cDNA is digested with a restriction enzyme such as *Rsa I* to generate short fragments useful for subractive hybridization (see PCR-Select™ manual; BD Biosciences Clontech). According to this method, the present invention has identified the sequences of SEQ ID Nos 1-8, which are the *RsaI* digested products of the full length sequences (SEQ ID Nos 9-16) as being differentially expressed in colon cancer. Table 1 below describes the identity of each of SEQ ID Nos 1-8 or 9-16.

**Table 1**

| **Gene Name** | **SEQ ID NO of truncated sequence** | **Corresponding SEQ ID NO from copending application USSN 09/871,161** | **SEQ ID NO of full length cDNA sequence** | **SEQ ID NO of amino acid sequence** | **Clone ID** |
|---|---|---|---|---|---|
| Interleukin 8 (IL 8) | 1 | 211 | 9 | 17 | DE0098T7 |
| Lumican (LUM) | 2 | 206 | 10 | 18 | DE0087T7 |
| p53-induced protein PIGPC1 | 3 | 418 | 11 | 19 | DE0789T7 |
| Serologically defined breast cancer antigen 84 (SDBCAG84) | 4 | 74 | 12 | 20 | DE0202T7 |
| Osteoblast specific factor 2 (OSF2) | 5 | 434 | 13 | 21 | DE0824T7 |
| Secreted protein, acidic, cysteine-rich (osteonectin; SPARC) | 6 | 389 | 14 | 22 | DE0715T7 |
| Transforming growth factor, beta induced (TGFβi) | 7 | 272 | 15 | 23 | DE0213T7 |
| Alpha 2 type collagen (COL1A2) | 8 | 189 | 16 | 24 | DE0042T7 |

Accordingly, the invention provides the sequences of SEQ ID Nos. 1-8 or 9-16, a sequence complementary thereto, or a sequence which specifically hybridizes to a sequence of SEQ ID No. 1-8 or 9-16. In certain embodiments, the subject nucleic acids are differentially expressed by at least about 0.5 fold, at least about 2 fold, at least about 5 fold, at least about 20 fold, or at least about 50 fold. Preferred nucleic acids include sequences identified as differentially expressed both in colon cancer cell tissue and colon cancer cell lines. In preferred embodiments, nucleic acids of the present invention are upregulated in tumor cells, especially colon cancer tissue and/or colon cancer-derived cell lines. In another embodiment, nucleic acids of the present invention are downregulated in tumor cells, especially colon cancer tissue and/or colon cancer-derived cell lines.

Genes which are upregulated, such as oncogenes, or downregulated, such as tumor suppressors, in aberrantly proliferating cells may be targets for diagnostic or therapeutic techniques. For example, upregulation of the *cdc2* gene induces mitosis. Overexpression of the *myt1* gene, a mitotic deactivator, negatively regulates the activity of *cdc2*. Aberrant proliferation may thus be induced either by upregulating *cdc2* or by downregulating *myt1*. Similarly, downregulation of tumor suppressors such as p53 and Rb have been implicated in tumorigenesis.

Still other preferred nucleic acids of the present invention encode a polypeptide comprising at least a portion of a polypeptide encoded by one of SEQ ID Nos. 9-16. For example, preferred nucleic acid molecules for use as probes/primers or antisense molecules (i.e., noncoding nucleic acid molecules) can comprise a region of a nucleic acid sequence of SEQ ID Nos 1-8 or 9-16 sufficient to hybridize with a nucleic acid substantially complementary to the sequence of SEQ ID Nos 1-8 or 9-16. Preferred nucleic acid molecules for use as probes/primers can further comprise a region of nucleic acid sequence substantially complementary to the sequence of SEQ ID Nos. 1-8 or 9-16 sufficient to hybridize with the sequence of SEQ ID Nos. 1-8 or 9-16. The nucleic acid sequences of the present invention for use as probes/primers are preferably at least about 12, 20, 30, 50, 60, 70, 80, 90, or 100 base pairs in length up to the length of the complete gene. Coding nucleic acid molecules can comprise, for example, from about 50, 60,70,80,90, or 100 base pairs up to the length of the complete gene.

Another aspect of the invention provides a nucleic acid which hybridizes under low, medium, or high stringency conditions to a nucleic acid sequence represented by one of SEQ ID Nos. 1-8 or 9-16, or a sequence complementary thereto. Appropriate stringency conditions which promote DNA hybridization, for example, 6.0 x sodium chloride/sodium citrate (SSC) at about 45 °C, followed by a wash of 2.0 x SSC at 50°C, are known to those skilled in the art or can be found in Current Protocols in Molecular Biology, John Wiley & Sons, N.Y. (1989), 6.3.1-12.3.6. For example, the salt concentration in the wash step can be selected from a low stringency of about 2.0 x SSC at 50°C to a high stringency of about 0.2 x SSC at 50°C. In addition, the temperature in the wash step can be increased from low stringency conditions at room temperature, about 22 °C, to high stringency conditions at about 65 °C. Both temperature and salt may be varied, or temperature or salt concentration may be held constant while the other variable is changed. In a preferred embodiment, a nucleic acid of the present invention will bind to one of SEQ ID Nos. 1-8 or 9-16, or a sequence complementary thereto, under moderately stringent conditions, for example at about 2.0 x SSC and about 40°C. In a particularly preferred embodiment, a nucleic acid of the present invention will bind to one of SEQ ID Nos. 1-8 or 9-16, or a sequence complementary thereto, under high stringency conditions.

In one embodiment, the invention provides nucleic acids which hybridize under low stringency conditions of 6 x SSC at room temperature followed by a wash at 2 x SSC at room temperature.

In another embodiment, the invention provides nucleic acids which hybridize under high stringency conditions of 2 x SSC at about 65 °C followed by a wash at 0.2 x SSC at about 65 °C.

Nucleic acids having a sequence that differs from the nucleotide sequences shown in one of SEQ ID Nos. 1-8 or 9-16, or a sequence complementary thereto, due to degeneracy in the genetic code, are also within the scope of the invention. Such nucleic acids encode functionally equivalent peptides (i.e., a peptide having equivalent or similar biological activity) but differ in sequence from the sequence shown in the sequence listing due to degeneracy in the genetic code. For example, a number of amino acids are designated by more than one triplet. Codons that specify the same amino acid, or synonyms (for example, CAU and CAC each encode histidine) may result in "silent" mutations which do not affect the amino acid sequence of a polypeptide. However, it is expected that DNA sequence polymorphisms that do lead to changes in the amino acid sequences of the subject polypeptides will exist among mammals. One skilled in the art will appreciate that these variations in one or more nucleotides (e.g., up to about 3-5% of the nucleotides) of the nucleic acids encoding polypeptides having an activity of a polypeptide may exist among individuals of a given species due to natural allelic variation.

Also within the scope of the invention are nucleic acids encoding splicing variants of proteins encoded by a nucleic acid of SEQ ID Nos. 1-8 or 9-16, or a sequence complementary thereto, or natural homologs of such proteins. Such homologs can be cloned by hybridization or PCR, as further described herein.

The polynucleotide sequence may also encode for a leader sequence, e.g., the natural leader sequence or a heterologous leader sequence, for a subject polypeptide. For example, the desired DNA sequence may be fused in the same reading frame to a DNA sequence which aids in expression and secretion of the polypeptide from the host cell, for example, a leader sequence which functions as a secretory sequence for controlling transport of the polypeptide from the cell. The protein having a leader sequence is a preprotein and may have the leader sequence cleaved by the host cell to form the mature form of the protein.

The polynucleotide of the present invention may also be fused in frame to a marker sequence, also referred to herein as "Tag sequence" encoding a "Tag peptide", which allows for marking and/or purification of the present invention. In a preferred embodiment, the market sequence is a hexahistidine tag, e g, supplied by a PQE-9 vector. Numerous other Tag peptides are available commercially Other frequently used Tags include myc-epitopes (e g, see Ellison et al. (1991) J Biol hem 266:21150-2 1157) which includes a 10-residue sequence from c-myc, the pFLAG system (International Biotechnologies, Inc.), the pEZZ-protein A system (Pharmacia, NJ), and a 16 amino acid portion of the Haemophilus influenza hemagglutinin protein. Furthermore, any polypeptide can be used as a Tag so long as a reagent, e.g., an antibody interacting specifically with the Tag polypeptide is available or can be prepared or identified.

As indicated by the examples set out below, nucleic acids can be obtained from mRNA present in any of a number of eukaryotic cells, e.g., and are preferably obtained from metazoan cells, more preferably from vertebrate cells, and even more preferably from mammalian cells. It should also be possible to obtain nucleic acids of the present invention from genomic DNA from both adults and embryos. For example, a gene can be cloned from either a cDNA or a genomic library in accordance with protocols generally known to persons skilled in the art. cDNA can be obtained by isolating total mRNA from a cell, e.g., a vertebrate cell, a mammalian cell, or a human cell, including embryonic cells. Double stranded cDNAs can then be prepared from the total mRNA, and subsequently inserted into a suitable plasmid or bacteriophage vector using any one of a number of known techniques. The gene can also be cloned using established polymerase chain reaction techniques in accordance with the nucleotide sequence information provided by the invention.

The invention includes within its scope a polynucleotide having the nucleotide sequence of nucleic acid obtained from this biological material, wherein the nucleic acid hybridizes under stringent conditions (at least about 4 x SSC at 65 °C, or at least about 4 x SSC at 42 °C; see, for example, U.S. Patent No. 5,707,829, incorporated herein by reference) with at least 15 contiguous nucleotides of at least one of SEQ ID Nos. 1-8 or 9-16. By this is intended that when at least 15 contiguous nucleotides of one of SEQ ID Nos. 1-8 or 9-16 is used as a probe, the probe will preferentially hybridize with a gene or mRNA (of the biological material) comprising the complementary sequence, allowing the identification and retrieval of the nucleic acids of the biological material that uniquely hybridize to the selected probe. Probes from more than one of SEQ ID Nos. 1-8 or 9-16 will hybridize with the same gene or mRNA if the cDNA from which they were derived corresponds to one mRNA. Probes of more than 15 nucleotides can be used, but 15 nucleotides represents enough sequence for unique identification.

In another embodiment the nucleic acids are isolated from libraries prepared from normal colon specific tissue. Techniques for producing and probing nucleic acid sequence libraries are described, for example, in Sambrook et al., "Molecular Cloning: A Laboratory Manual" (New York, Cold Spring Harbor Laboratory, 1989). The cDNA can be prepared by using primers based on a sequence from SEQ ID Nos. 1-8 or 9-16. In one embodiment, the cDNA library can be made from only poly-adenylated mRNA. Thus, poly-T primers can be used to prepare cDNA from the mRNA. Alignment of SEQ ID Nos. 1-8 or 9-16 can result in identification of a related polypeptide or polynucleotide. Some of the polynucleotides disclosed herein contains repetitive regions that were subject to masking during the search procedures. The information about the repetitive regions is discussed below.

Constructs of polynucleotides having sequences of SEQ ID Nos. 1-8 or 9-16, or sequences complementary thereto can be generated synthetically. Alternatively, single-step assembly of a gene and entire plasmid from large numbers of oligodeoxyribonucleotides is described by Stemmer et at, Gene (Amsterdam) (1995) 164(i):49-53. In this method, assembly PCR (the synthesis of long DNA sequences from large numbers of oligodeoxyribonucleotides (oligos)) is described. The method is derived from DNA shuffling (Stemmer, Nature (1994) 370:389-391), and does not rely on DNA ligase, but instead relies on DNA polymerase to build increasingly longer DNA fragments during the assembly process. For example, a 1.1-kb fragment containing the TEM-1 beta-lactamase-encoding gene (bla) can be assembled in a single reaction from a total of 56 oligos, each 40 nucleotides (nt) in length. The synthetic gene can be PCR amplified and cloned in a vector containing the tetracycline-resistance gene (Tc-R) as the sole selectable marker. Without relying on ampicillin (Ap) selection, 76% of the Tc-R colonies were Ap-R, making this approach a general method for the rapid and cost-effective synthesis of any gene.

Identification of Functional and Structural Motifs of Novel Genes Using Art-Recognized Methods

Translations of the nucleotide sequence of the nucleic acids, cDNAs, or full genes can be aligned with individual known sequences. Similarity with individual sequences can be used to determine the activity of the polypeptides encoded by the polynucleotides of the invention. For example, sequences that show similarity with a chemokine sequence may exhibit chemokine activities. Also, sequences exhibiting similarity with more than one individual sequence may exhibit activities that are characteristic of either or both individual sequences.

The full length sequences and fragments of the polynucleotide sequences of the nearest neighbors can be used as probes and primers to identify and isolate the full length sequence of the nucleic acid. The nearest neighbors can indicate a tissue or cell type to be used to construct a library for the full-length sequences of the nucleic acid.

Typically, the nucleic acids are translated in all six frames to determine the best alignment with the individual sequences. The sequences disclosed herein in the Sequence Listing are in a 5' to 3' orientation and translation in three frames can be sufficient (with a few specific exceptions as described in the Examples). These amino acid sequences are referred to, generally, as query sequences, which will be aligned with the individual sequences.

Nucleic acid sequences can be compared with known genes by any of the methods disclosed above. Results of individual and query sequence alignments can be divided into three categories: high similarity, weak similarity, and no similarity. Individual alignment results ranging from high similarity to weak similarity provide a basis for determining polypeptide activity and/or structure.

Parameters for categorizing individual results include: percentage of the alignment region length where the strongest alignment is found, percent sequence identity, and p value.

The percentage of the alignment region length is calculated by counting the number of residues of the individual sequence found in the region of strongest alignment. This number is divided by the total residue length of the query sequence to find a percentage. An example is shown below:

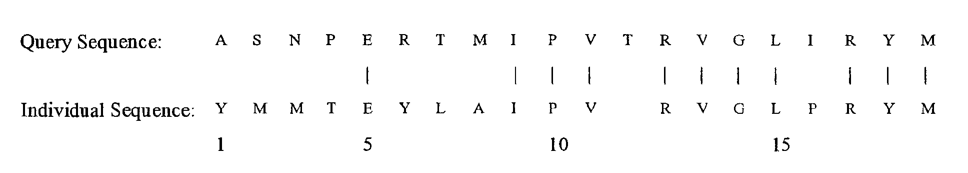

The region of alignment begins at amino acid 9 and ends at amino acid 19. The total length of the query sequence is 20 amino acids. The percent of the alignment region length is 11/20 or 55%.

Percent sequence identity is calculated by counting the number of amino acid matches between the query and individual sequence and dividing total number of matches by the number of residues of the individual sequence found in the region of strongest alignment. For the example above, the percent identity would be 10 matches divided by 11 amino acids, or approximately 90.9%.

P value is the probability that the alignment was produced by chance. For a single alignment, the p value can be calculated according to Karlin et al., Proc. Natl. Acad. Sci. 87: 2264 (1990) and Karlin et al., Proc. Natl. Acad. Sci. 90: (1993). The p value of multiple alignments using the same query sequence can be calculated using an heuristic approach described in Altschul et al., Genet. 6:119(1994). Alignment programs such as BLAST program can calculate the p value.

The boundaries of the region where the sequences align can be determined according to Doolittle, Methods in Enzymology, supra; BLAST or FASTA programs; or by determining the area where the sequence identity is highest.

Another factor to consider for determining identity or similarity is the location of the similarity or identity. Strong local alignment can indicate similarity even if the length of alignment is short. Sequence identity scattered throughout the length of the query sequence also can indicate a similarity between the query and profile sequences.

### High Similarity

For the alignment results to be considered high similarity, the percent of the alignment region length, typically, is at least about 55% of total length query sequence; more typically, at least about 58%; even more typically; at least about 60% of the total residue length of the query sequence. Usually, percent length of the alignment region can be as much as about 62%; more usually, as much as about 64%; even more usually, as much as about 66%.

Further, for high similarity, the region of alignment, typically, exhibits at least about 75% of sequence identity; more typically, at least about 78%; even more typically; at least about 80% sequence identity. Usually, percent sequence identity can be as much as about 82%; more usually, as much as about 84%; even more usually, as much as about 86%.

The p value is used in conjunction with these methods. If high similarity is found, the query sequence is considered to have high similarity with a profile sequence when the p value is less than or equal to about 10⁻²; more usually; less than or equal to about 10⁻³ even more usually; less than or equal to about 10⁻⁴. More typically, the p value is no more than about 10⁻⁵ more typically; no more than or equal to about 10⁻¹⁰; even more typically; no more than or equal to about 10⁻¹⁵ for the query sequence to be considered high similarity.

### Weak Similarity

For the alignment results to be considered weak there is no minimum percent length of the alignment region no minimum length of alignment. A better showing of weak similarity is considered when the region of alignment is, typically, at least about 15 amino acid residues in length; more typically, at least about 20; even more typically; at least about 25 amino acid residues in length. Usually, length of the alignment region can be as much as about 30 amino acid residues; more usually, as much as about 40; even more usually, as much as about 60 amino acid residues.

Further, for weak similarity, the region of alignment, typically, exhibits at least about 35% of sequence identity; more typically, at least about 40%; even more typically; at least about 45% sequence identity. Usually, percent sequence identity can be as much as about 50%; more usually, as much as about 55%; even more usually, as much as about 60%.

If low similarity is found, the query sequence is considered to have weak similarity with a profile sequence when the p value is usually less than or equal to about 10⁻²; more usually; less than or equal to about 10⁻³ even more usually; less than or equal to about 10⁻⁴. More typically, the p value is no more than about 10⁻⁵ more usually; no more than or equal to about 10⁻¹⁰; even more usually; no more than or equal to about 10⁻¹⁵ for the query sequence to be considered weak similarity.

### Similarity Determined by Sequence Identity

Sequence identity alone can be used to determine similarity of a query sequence to an individual sequence and can indicate the activity of the sequence. Such an alignment, preferably, permits gaps to align sequences. Typically, the query sequence is related to the profile sequence if the sequence identity over the entire query sequence is at least about 15%; more typically, at least about 20%; even more typically, at least about 25%; even more typically, at least about 50%. Sequence identity alone as a measure of similarity is most useful when the query sequence is usually, at least 80 residues in length; more usually, 90 residues; even more usually, at least 95 amino acid residues in length. More typically, similarity can be concluded based on sequence identity alone when the query sequence is preferably 100 residues in length; more preferably, 120 residues in length; even more preferably, 150 amino acid residues in length.

### Determining Activity from Alignments with Profile and Multiple Aligned Sequences

Translations of the nucleic acids can be aligned with amino acid profiles that define either protein families or common motifs. Also, translations of the nucleic acids can be aligned to multiple sequence alignments (MSA) comprising the polypeptide sequences of members of protein families or motifs. Similarity or identity with profile sequences or MSAs can be used to determine the activity of the polypeptides encoded by nucleic acids or corresponding cDNA or genes. For example, sequences that show an identity or similarity with a chemokine profile or MSA can exhibit chemokine activities.

Profiles can designed manually by (1) creating a MSA, which is an alignment of the amino acid sequence of members that belong to the family and (2) constructing a statistical representation of the alignment. Such methods are described, for example, in Bimey et al., Nucl. Acid Res. 25(14): 2730-2739 (1996).

MSAs of some protein families and motifs are publicly available. For example, these include MSAs of 547 different families and motifs. These MSAs are described also in Sonnhammer et al., Proteins 28: 405-420 (1997). Other sources are also available in the world wide web. A brief description of these MSAs is reported in Pascarella et al., Prot. Eng. 9(3): 249-251 (1996).

Techniques for building profiles from MSAs are described in Sonnhammer et al., supra; Birney et al., supra; and Methods in Enzymology, vol. 266: "Computer Methods for Macromolecular Sequence Analysis," 1996, ed. Doolittle, Academic Press, Inc., a division of Harcourt Brace & Co., San Diego, California, USA.

Similarity between a query sequence and a protein family or motif can be determined by (a) comparing the query sequence against the profile and/or (b) aligning the query sequence with the members of the family or motif.

Typically, a program such as Searchwise can be used to compare the query sequence to the statistical representation of the multiple alignment, also known as a profile. The program is described in Birney et al., supra. Other techniques to compare the sequence and profile are described in Sonnhammer et al., supra and Doolittle, supra.

Next, methods described by Feng et al., J. Mol. Evol. 25:351-360 (1987) and Higgins et al., CABIOS 5:151-153 (1989) can be used align the query sequence with the members of a family or motif, also known as a MSA. Computer programs, such as PILEUP, can be used. See Feng et al., infra.

The following factors are used to determine if a similarity between a query sequence and a profile or MSA exists: (1) number of conserved residues found in the query sequence, (2) percentage of conserved residues found in the query sequence, (3) number of frameshifts, and (4) spacing between conserved residues.

Some alignment programs that both translate and align sequences can make any number of frameshifts when translating the nucleotide sequence to produce the best alignment. The fewer frameshifts needed to produce an alignment, the stronger the similarity or identity between the query and profile or MSAs. For example, a weak similarity resulting from no frameshifts can be a better indication of activity or structure of a query sequence, than a strong similarity resulting from two frameshifts.

Preferably, three or fewer frameshifts are found in an alignment; more preferably two or fewer frameshifts; even more preferably, one or fewer frameshifts; even more preferably, no frameshifts are found in an alignment of query and profile or MSAs.

Conserved residues are those amino acids that are found at a particular position in all or some of the family or motif members. For example, most known chemokines contain four conserved cysteines. Alternatively, a position is considered conserved if only a certain class of amino acids is found in a particular position in all or some of the family members. For example, the N-terminal position may contain a positively charged amino acid, such as lysine, arginine, or histidine.

Typically, a residue of a polypeptide is conserved when a class of amino acids or a single amino acid is found at a particular position in at least about 40% of all class members; more typically, at least about 50%; even more typically, at least about 60% of the members. Usually, a residue is conserved when a class or single amino acid is found in at least about 70% of the members of a family or motif; more usually, at least about 80%; even more usually, at least about 90%; even more usually, at least about 95%.

A residue is considered conserved when three unrelated amino acids are found at a particular position in the some or all of the members; more usually, two unrelated amino acids. These residues are conserved when the unrelated amino acids are found at particular positions in at least about 40% of all class member, more typically, at least about 50%; even more typically, at least about 60% of the members. Usually, a residue is conserved when a class or single amino acid is found in at least about 70% of the members of a family or motif more usually, at least about 80%; even more usually, at least about 90%; even more usually, at least about 95%.

A query sequence has similarity to a profile or MSA when the query sequence comprises at least about 25% of the conserved residues of the profile or MSA; more usually, at least about 30%; even more usually; at least about 40%. Typically, the query sequence has a stronger similarity to a profile sequence or MSA when the query sequence comprises at least about 45% of the conserved residues of the profile or MSA more typically, at least about 50%; even more typically; at least about 55%.

The nucleotide sequences determined from the cloning of genes from tumor cells, especially colon cancer cell lines and tissues will further allow for the generation of probes and primers designed for identifying and/or cloning homologs in other cell types, e.g., from other tissues, as well as homologs from other mammalian organisms. Nucleotide sequences useful as probes/primers may include all or a portion of the sequences listed in SEQ ID Nos. 1-8 or 9 -16 or sequences complementary thereto, or sequences which hybridize under stringent conditions to all or a portion of SEQ ID Nos. 1-8 or 9-16. For instance, the present invention also provides a probe/primer comprising a substantially purified oligonucleotide, which oligonucleotide comprising a nucleotide sequence that hybridizes under stringent conditions to at least approximately 8, preferably about 12, preferably about 15, preferably about 25, more preferably about 40 consecutive nucleotides up to the full length of the sense or anti-sense sequence selected from the group consisting of SEQ ID Nos. 1-8 or 9-16, or a sequence complementary thereto, or naturally occurring mutants thereof. For instance, primers based on a nucleic acid represented in SEQ ID Nos. 1-8 or 9-16, or a sequence complementary thereto, can be used in PCR reactions to clone homologs of that sequence.

In yet another embodiment, the invention provides probes/primers comprising a nucleotide sequence that hybridizes under moderately stringent conditions to at least approximately 8, preferably about 12, preferably about 15, preferably about 25, more preferably about 40 consecutive nucleotides up to the full length of the sense or antisense sequence selected from the group consisting of SEQ ID Nos. 1-8 or 9-16, or naturally occurring mutants thereof.

In particular, these probes are useful because they provide a method for detecting mutations in wild-type genes of the present invention. Nucleic acid probes which are complementary to a wild-type gene of the present invention and can form mismatches with mutant genes are provided, allowing for detection by enzymatic or chemical cleavage or by shifts in electrophoretic mobility. Likewise, probes based on the subject sequences can be used to detect transcripts or genomic sequences encoding the same or homologous proteins, for use, for example, in prognostic or diagnostic assays. In preferred embodiments, the probe further comprises a label group attached thereto and able to be detected, e.g., the label group is selected from radioisotopes, fluorescent compounds, chemiluminescent compounds, enzymes, and enzyme co-factors.

Full-length cDNA molecules comprising the disclosed nucleic acids are obtained as follows. A subject nucleic acid or a portion thereof comprising at least approximately 8, preferably about 12, preferably about 15, preferably about 25, more preferably about 40 nucleotides up to the full length of a sequence represented in SEQ ID Nos. 1-8 or 9-16, or a sequence complementary thereto, may be used as a hybridization probe to detect hybridizing members of a cDNA library using probe design methods, cloning methods, and clone selection techniques as described in U.S. Patent No. 5,654,173, "Secreted Proteins and Polynucleotides Encoding Them," incorporated herein by reference. Libraries of cDNA may be made from selected tissues, such as normal or tumor tissue, or from tissues of a mammal treated with, for example, a pharmaceutical agent. Preferably, the tissue is the same as that used to generate the nucleic acids, as both the nucleic acid and the cDNA represent expressed genes. Most preferably, the cDNA library is made from the biological material described herein in the Examples. Alternatively, many cDNA libraries are available commercially. (Sambrook et al., Molecular Cloning: A Laboratory Manual, 2nd Ed. (Cold Spring Harbor Press, Cold Spring Harbor, NY 1989). The choice of cell type for library construction may be made after the identity of the protein encoded by the nucleic acid-related gene is known. This will indicate which tissue and cell types are likely to express the related gene, thereby containing the mRNA for generating the cDNA.

Members of the library that are larger than the nucleic acid, and preferably that contain the whole sequence of the native message, may be obtained. To confirm that the entire cDNA has been obtained, RNA protection experiments may be performed as follows. Hybridization of a full-length cDNA to an mRNA may protect the RNA from RNase degradation. If the cDNA is not full length, then the portions of the mRNA that arc not hybridized may be subject to RNase degradation. This may be assayed, as is known in the art, by changes in electrophoretic mobility on polyacrylamide gels, or by detection of released monoribonucleotides. Sambrook et al., Molecular Cloning: A Laboratory Manual, 2nd Ed. (Cold Spring Harbor Press, Cold Spring Harbor, NY 1989). In order to obtain additional sequences 5' to the end of a partial cDNA, 5' RACE (PCR Protocols: A Guide to Methods and Applications (Academic Press, Inc. 1990)) may be performed.

Genomic DNA may be isolated using nucleic acids in a manner similar to the isolation of full-length cDNAs. Briefly, the nucleic acids, or portions thereof, may be used as probes to libraries of genomic DNA. Preferably, the library is obtained from the cell type that was used to generate the nucleic acids. Most preferably, the genomic DNA is obtained from the biological material described herein in the Example. Such libraries may be in vectors suitable for carrying large segments of a genome, such as P1 or YAC, as described in detail in Sambrook et al., 9.4-9.30. In addition, genomic sequences can be isolated from human BAC libraries, which are commercially available from Research Genetics, Inc., Huntville, Alabama, USA, for example. In order to obtain additional 5' or 3' sequences, chromosome walking may be performed, as described in Sambrook et al., such that adjacent and overlapping fragments of genomic DNA are isolated. These may be mapped and pieced together, as is known in the art, using restriction digestion enzymes and DNA ligase.

Using the nucleic acids of the invention, corresponding full length genes can be isolated using both classical and PCR methods to construct and probe cDNA libraries. Using either method, Northern blots, preferably, may be performed on a number of cell types to determine which cell lines express the gene of interest at the highest rate.

Classical methods of constructing cDNA libraries in Sambrook et al., supra. With these methods, cDNA can be produced from mRNA and inserted into viral or expression vectors. Typically, libraries of mRNA comprising poly(A) tails can be produced with poly(T) primers. Similarly, cDNA libraries can be produced using the instant sequences as primers.

PCR methods may be used to amplify the members of a cDNA library that comprise the desired insert. In this case, the desired insert may contain sequence from the full length cDNA that corresponds to the instant nucleic acids. Such PCR methods include gene trapping and RACE methods.

Gene trapping may entail inserting a member of a cDNA library into a vector. The vector then may be denatured to produce single stranded molecules. Next, a substrate-bound probe, such a biotinylated oligo, may be used to trap cDNA inserts of interest. Biotinylated probes can be linked to an avidin-bound solid substrate. PCR methods can be used to amplify the trapped cDNA. To trap sequences corresponding to the full length genes, the labeled probe sequence may be based on the nucleic acids of the invention, e.g., SEQ ID Nos. 1, 3, 5, or 7, or a sequence complementary thereto. Random primers or primers specific to the library vector can be used to amplify the trapped cDNA. Such gene trapping techniques are described in Gruber et al., PCT WO 95/04745 and Gruber et al., U.S. Pat. No. 5,500,356. Kits are commercially available to perform gene trapping experiments from, for example, Life Technologies, Gaithersburg, Maryland, USA.

"Rapid amplification of cDNA ends," or RACE, is a PCR method of amplifying cDNAs from a number of different RNAs. The cDNAs may be ligated to an oligonucleotide linker and amplified by PCR using two primers. One primer may be based on sequence from the instant nucleic acids, for which full length sequence is desired, and a second primer may comprise a sequence that hybridizes to the oligonucleotide linker to amplify the cDNA. A description of this method is reported in PCT Pub. No. WO 97/19110.

In preferred embodiments of RACE, a common primer may be designed to anneal to an arbitrary adaptor sequence ligated to cDNA ends (Apte and Siebert, Biotechniques 15:890-893, 1993; Edwards et al., Nuc. Acids Res. 19:5227-5232, 1991). When a single gene-specific RACE primer is paired with the common primer, preferential amplification of sequences between the single gene specific primer and the common primer occurs. Commercial cDNA pools modified for use in RACE are available.

Another PCR-based method generates full-length cDNA library with anchored ends without specific knowledge of the cDNA sequence. The method uses lock-docking primers (1-VI), where one primer, poly TV (I-Ill) locks over the polyA tail of eukaryotic mRNA producing first strand synthesis and a second primer, polyGH (IV-VI) locks onto the polyC tail added by terminal deoxynucleotidyl transferase (TdT). This method is described in PCT Pub. No. WO 96/40998.

The promoter region of a gene generally is located 5' to the initiation site for RNA polymerase IL Hundreds of promoter regions contain the "TATA" box, a sequence such as TATTA or TATAA, which is sensitive to mutations. The promoter region can be obtained by performing 5' RACE using a primer from the coding region of the gene. Alternatively, the cDNA can be used as a probe for the genomic sequence, and the region 5' to the coding region is identified by "walking up."

If the gene is highly expressed or differentially expressed, the promoter from the gene may be of use in a regulatory construct for a heterologous gene.

Once the full-length cDNA or gene is obtained, DNA encoding variants can be prepared by site-directed mutagenesis, described in detail in Sambrook 15.3 - 15.63. The choice of codon or nucleotide to be replaced can be based on the disclosure herein on optional changes in amino acids to achieve altered protein structure and/or function.

As an alternative method to obtaining DNA or RNA from a biological material, nucleic acid comprising nucleotides having the sequence of one or more nucleic acids of the invention can be synthesized. Thus, the invention encompasses nucleic acid molecules ranging in length from about 8 nucleotides (corresponding to at least 12 contiguous nucleotides which hybridize under stringent conditions to or are at least 80% identical to a nucleic acid represented by one of SEQ ID Nos. 1, 3, 5, or 7, or a sequence complementary thereto) up to a maximum length suitable for one or more biological manipulations, including replication and expression, of the nucleic acid molecule. The invention includes but is not limited to (a) nucleic acid having the size of a full gene, and comprising at least one of SEQ ID Nos. 1 -8 (such as the full length cDNA sequence of one or more of SEQ ID Nos 9-16), or a sequence complementary thereto; (b) the nucleic acid of(a) also comprising at least one additional gene, operably linked to permit expression of a fusion protein; (c) an expression vector comprising (a) or (b); (d) a plasmid comprising (a) or (b); and (e) a recombinant viral particle comprising (a) or (b). Construction of(a) can be accomplished as described below in part IV.

The sequence of a nucleic acid of the present invention is not limited and can be any sequence of A, T, G, and/or C (for DNA) and A, U, 0, and/or C (for RNA) or modified bases thereof, including inosine and pseudouridine. The choice of sequence will depend on the desired function and can be dictated by coding regions desired, the intron-like regions desired, and the regulatory regions desired.

The invention further provides plasmids and vectors, which can be used to express a gene in a host cell. The host cell may be any prokaryotic or eukaryotic cell. Thus, a nucleotide sequence derived from any one of SEQ ID Nos. 1-8 or 9-16, or a sequence complementary thereto, encoding all or a selected portion of a protein, can be used to produce a recombinant form of an polypeptide via microbial or eukaryotic cellular processes. Ligating the polynucleotide sequence into a gene construct, such as an expression vector, and transforming or transfecting into hosts, either eukaryotic (yeast, avian, insect or mammalian) or prokaryotic (bacterial cells), are standard procedures well known in the art.

Vectors that allow expression of a nucleic acid in a cell are referred to as expression vectors. Typically, expression vectors contain a nucleic acid operably linked to at least one transcriptional regulatory sequence. Regulatory sequences are art-recognized and are selected to direct expression of the subject nucleic acids. Transcriptional regulatory sequences are described in Goeddel; Gene Expression Technology: Methods in Enzymology 185, Academic Press, San Diego, CA (1990). In one embodiment, the expression vector includes a recombinant gene encoding a peptide having an agonistic activity of a subject polypeptide, or alternatively, encoding a peptide which is an antagonistic form of a subject polypeptide.

The choice of plasmid will depend on the type of cell in which propagation is desired and the purpose of propagation. Certain vectors are useful for amplifying and making large amounts of the desired DNA sequence. Other vectors are suitable for expression in cells in culture. Still other vectors are suitable for transfer and expression in cells in a whole animal or person. The choice of appropriate vector is well within the skill of the art. Many such vectors are available commercially. The nucleic acid or full-length gene is inserted into a vector typically by means of DNA ligase attachment to a cleaved restriction enzyme site in the vector. Alternatively, the desired nucleotide sequence may be inserted by homologous recombination in vivo. Typically this is accomplished by attaching regions of homology to the vector on the flanks of the desired nucleotide sequence. Regions of homology are added by ligation of oligonucleotides, or by polymerase chain reaction using primers comprising both the region of homology and a portion of the desired nucleotide sequence.

Nucleic acids or full-length genes are linked to regulatory sequences as appropriate to obtain the desired expression properties. These may include promoters (attached either at the 5' end of the sense strand or at the 3' end of the antisense strand), enhancers, terminators, operators, repressors, and inducers. The promoters may be regulated or constitutive. In some situations it may be desirable to use conditionally active promoters, such as tissue-specific or developmental stage-specific promoters. These are linked to the desired nucleotide sequence using the techniques described above for linkage to vectors. Any techniques known in the art may be used.

When any of the above host cells, or other appropriate host cells or organisms, are used to replicate and/or express the polynucleotides or nucleic acids of the invention, the resulting replicated nucleic acid, RNA, expressed protein or polypeptide, is within the scope of the invention as a product of the host cell or organism. The product is recovered by any appropriate means known in the art.

Once the gene corresponding to the nucleic acid is identified, its expression can be regulated in the cell to which the gene is native. For example, an endogenous gene of a cell can be regulated by an exogenous regulatory sequence as disclosed in U.S. Patent No. 5,641,670, "Protein Production and Protein Delivery."

A number of vectors exist for the expression of recombinant proteins in yeast (see, for example, *Broach et al* (1983) in Experimental Manipulation of Gene Expression, ed. M. Inouye, Academic Press, p. 83, incorporated by reference herein). In addition, drug resistance markers such as ampicillin can be used. In an illustrative embodiment, a polypeptide is produced recombinantly utilizing an expression vector generated by sub-cloning one of the nucleic acids represented in one of SEQ ID Nos. 1-8 or 9-16, preferably one of SEQ ID Nos 9-16, or a sequence complementary thereto.

The preferred mammalian expression vectors contain both prokaryotic sequences, to facilitate the propagation of the vector in bacteria, and one or more eukaryotic transcription units that are expressed in eukaryotic cells. The various methods employed in the preparation of plasmids and transformation of host organisms are well known in the art. For other suitable expression systems for both prokaryotic and eukaryotic cells, as well as general recombinant procedures, see Molecular Cloning: A Laboratory Manual, 2 ' Ed., ed. by Sambrook, Fritsch and Maniatis (Cold Spring Harbor Laboratory Press: 1989) Chapters 16 and 17.

When it is desirable to express only a portion of a gene, e.g., a truncation mutant, it may be necessary to add a start codon (ATG) to the oligonucleotide fragment containing the desired sequence to be expressed. It is well known in the art that a methionine at the N-terminal position can be enzymatically cleaved by the use of the enzyme methionine aminopeptidase (MAP). MAP has been cloned from E. coli (Ben-Bassat *et al*., (1987) *J*. *Bacteriol.* 169:751-757) and Salmonella typhimurium and its *in vitro* activity has been demonstrated on recombinant proteins (Miller *et al*. (1987) PNAS 84:2718-1722). Therefore, removal of an N-terminal methionine, if desired, can be achieved either in vivo by expressing polypeptides in a host which produces MAP (e.g., E. coli or CM89 or S. cerevisiae), *or in vitro* by use of purified MAP (e.g., procedure of Miller *et al*., supra).

Moreover, the nucleic acid constructs of the present invention can also be used as part of a gene therapy protocol to deliver nucleic acids such as antisense nucleic acids. Thus, another aspect of the invention features expression vectors for in vivo or in vitro transfection with an antisense oligonucleotide.

Nucleic acid molecules and constructs providing transgenes such as antisense oligonucleotides under the control of highly cell-type specific promoters and amplification promoter elements, can be incorporated into a vector and administered to any mammal, including a human. Many such vectors are commercially available, and other suitable vectors can be readily prepared and obvious to the skilled artisan. The exact design of the vector depends on such factors as the choice of the host cell to be transformed and/or the type of protein desired to be expressed. Suitable vectors can be produced by ligating the desired construct into a plasmid or viral vector suitable for expression in eukaryotic cells (see, for example, Broach, et al., Experimental Manipulation of Gene Expression, ed. M. Inouye (Academic Press, 1983) p. 83; Molecular Cloning: A Laboratory Manual, 2nd Ed., ed. Sambrook, et al. (Cold Spring Harbor Laboratory Press, 1989) Chapters 16 and 17, the entireties of which are incorporated by reference herein).

Examples of vectors that can be used include, but are not limited to, plasmids such as pBR322, pUC, or Co1E1; adenovirus; Sindbis virus; simian virus 40; cytomegalovirus; and retroviral vectors such as murine sarcoma virus, mouse mammary tumor virus, Moloney murine leukemia virus, and Rous sarcoma virus. Bacterial vectors can be used, such as *Salmonella ssp*., *Yersinia enterocolitica, Shigella spp*., *Vibrio cholerae, Mycobacterium* strain *BCG,* and *Listeria monocytogenes*. Minichromosomes such as MC and MC1, bacteriophages, cosmids (plasmids into which phage lambda cos sites have been inserted) and replicons (genetic elements that are capable of independent extrachromosomal replication).

The vectors described above can additionally comprise sequences encoding one or more selectable markers, including, but not limited to, the gene that encodes dihydrofolate reductase and the genes that confer resistance to neomycin, tetracycline, ampicillin, chloramphenicol, kanamycin and streptomycin resistance. To improve incorporation into the genome of the target cell (if desired), a retroviral vector can be used, and long terminal repeat (LTR) sequences can be added on either side of the expression construct (see, e.g., Vile, et al., Virology 214: 307-313 (1995), the entirety of which is incorporated by reference herein).

Delivery of a nucleic acid construct comprising a nucleotide sequence of the present invention under the control of a highly cell-type specific promoter can be by any means known in the art, including oral or intranasal administration; intramuscular, intradermal, intraperitoneal, or subcutaneous injection, including injection using a biological ballistic gun ("gene gun"). Administration of the nucleic acid for therapeutic purposes can be repeated at any desired interval as needed to achieve therapeutic efficacy. Additional components can be added to a vector to improve its selective delivery to target cells and to repress its delivery to non-target cells. Examples of approaches that can be used include host range extension, entry enhancement, and host range restriction, as described in Peng and Russell, Cur. Opin. Biotech. 10: 454-457 (1999), the entirety of which is incorporated herein by reference.

In addition to viral transfer methods, non-viral methods can also be employed to introduce a subject nucleic acid, e.g., a sequence represented by one of SEQ ID Nos. 1-8 or 9-16, or a sequence complementary thereto, into the tissue of an animal. Most nonviral methods of gene transfer rely on normal mechanisms used by mammalian cells for the uptake and intracellular transport of macromolecules. In preferred embodiments, non-viral targeting means of the present invention rely on endocytic pathways for the uptake of the subject nucleic acid by the targeted cell. Exemplary targeting means of this type include liposomal derived systems, polylysine conjugates, and artificial viral envelopes.

A nucleic acid of any of SEQ ID Nos. 1-8 or 9-16, or a sequence complementary thereto, the corresponding cDNA, or the full-length gene may be used to express the partial or complete gene product. Appropriate nucleic acid constructs are purified using standard recombinant DNA techniques as described in, for example, *Sambrook et al*., (1989) *Molecular Cloning: A Laboratory Manual*, 2nd ed. (Cold Spring Harbor Press, Cold Spring Harbor, New York), and under current regulations described in United States Dept. of HHS, National Institute of Health (NIH) Guidelines for Recombinant

DNA Research. The polypeptides encoded by the nucleic acid may be expressed in any expression system, including, for example, bacterial, yeast, insect, amphibian and mammalian systems. Suitable vectors and host cells are described in U.S. Patent No. 5,654,173.

Bacteria. Expression systems in bacteria include those described in Chang *et al*., *Nature* (1978) *275*:615, Goeddel *et al*., *Nature* (1979) 281 :5, Goeddel *et al*., *Nucleic Acids Rec.* (1980) 8:4057; EP 0 036,776, U.S. Patent No. 4,551,433, *DeBoer et al*., *Proc. Natl. Acad. Sci. (USA)* (1983) *80*:2125, and Siebenlist *et al*., *Cell* (1980) 20:269.

Yeast. Expression systems in yeast include those described in Hinnen *et al*., *Proc. Natl. Acad. Sci. ( USA)* (1978) *75*:1929; Ito *et al*., *J*. *Bacteriol.* (1983) *153*:163; Kurtz *et al*., *Mol. Cell. Biol.* (1986) *6*:142; Kunze *et al*., *J. Basic Microbiol.* (1985) *25*:141; *Gleeson et al*., *J. Gen. Microbiol.* (1986) *132*:3459, Roggenkamp *et al*., *Mol. Gen. Genet.* (1986) *202*:302) Das *et al*., *J. Bacteriol.* (1984) *158*:1165; De Louvencourt *et al*., *J*. *Bacteriol.* (1983) *154*:737, Van den Berg *et al*., Bio/Technology (1990) *8*:135; *Kunze et al*., J. Basic Microbiol. (1985) *25*:141; Cregg *et al*., *Mol. Cell. Biol.* (1985) 5:3376, U.S. Patent Nos. 4,837,148 and 4,929,555; Beach and Nurse, Nature (1981) *300*:706; Davidow *et al*., Curr. Genet. (1985) *10*:380, Gaillardin *et al*., Curr. Genet. (1985) *10*:49, Ballance *et al*., *Biochem.* Biophys. Res. Commun. (1983) *112*:284289; Tilburn *et al*., Gene (1983) 26:205221, *Yelton et al*., Proc. Natl. Acad. Sci. (USA) (1984) *81*:14701474, Kelly and Hynes, EMBO J. (1985) *4*:475479; EP 0 244,234, and WO 91/00357.

Insect Cells. Expression of heterologous genes in insects is accomplished as described in U.S. Patent No. 4,745,051, Friesen *et al*., (1986) "The Regulation of Baculovirus Gene Expression" in: The Molecular Biology Of Baculoviruses (W. Doerfler, ed.), EP 0 127,839, EP 0 155,476, and Vlak *et al*., J. *Gen. Virol*. (1988) 69:765776, Miller *et al*., *Ann. Rev. Microbiol.* (1988) *42*:177, Carbonell *et al*., *Gene* (1988) 73:409, Maeda *et al*., Nature (1985) *315*:592594, Lebacq Verheyden et at., Mol. Cell. Biol. (1988) *8*:3129; *Smith et al*., Proc. Nail. Acad. Sci. (USA) (1985) *82*:8404, *Miyajima et al*., Gene (1987) 58:273; and Martin*et al*., DNA (1988) 7:99. Numerous baculoviral strains and variants and corresponding permissive insect host cells from hosts are described in *Luckow et al*., Bio/Technology (1988) *6*:4755, Miller *et al*., Generic Engineering (Setlow, J.K. *et al*. eds.), Vol. 8 (Plenum Publishing, 1986), pp. 277279, and *Maeda et al*., *Nature*, (1985) *315*:592-594.

Mammalian Cells. Mammalian expression is accomplished as described in Dijkema *et al*., *EMBO J*. (1985) *4*:761, Gorman *et al*., *Proc. Natl. Acad*. *Sci.* (*USA*) (1982) 79:6777, Boshart *et al*., Cell (1985) *41*:52 1 and U.S. Patent No. 4,399,216. Other features of mammalian expression are facilitated as described in Ham and Wallace, *Meth*. *Enz*. (1979) *58*:44, Barnes and Sato, *Anal. Biochem.* (1980) *102*:255, U.S. Patent Nos. 4,767,704, 4,657,866, 4,927,762, 4,560,655, WO 90/103430, WO 87/00195, and U.S. RE 30,985.

One aspect of the invention relates to the use of the isolated nucleic acid, e.g., SEQ ID Nos. 1-8 or 9-16, or a sequence complementary thereto, in antisense therapy. As used herein, antisense therapy refers to administration or *in situ* generation of oligonucleotide molecules or their derivatives which specifically hybridize (e.g., bind) under cellular conditions with the cellular mRNA and/or genomic DNA, thereby inhibiting transcription and/or translation of that gene. The binding may be by conventional base pair complementarity, or, for example, in the case of binding to DNA duplexes, through specific interactions in the major groove of the double helix. In general, antisense therapy refers to the range of techniques generally employed in the art, and includes any therapy which relies on specific binding to oligonucleotide sequences.

An antisense construct of the present invention can be delivered, for example, as an expression plasmid which, when transcribed in the cell, produces RNA which is complementary to at least a unique portion of the cellular mRNA. Alternatively, the antisense construct is an oligonucleotide probe which is generated ex vivo and which, when introduced into the cell, causes inhibition of expression by hybridizing with the mRNA and/or genomic sequences of a subject nucleic acid. Such oligonucleotide probes are preferably modified oligonucleotides which are resistant to endogenous nucleases, e.g., exonucleases and/or endonucleases, and are therefore stable in vivo. Exemplary nucleic acid molecules for use as antisense oligonucleotides are phosphoramidate, phosphorothioate and methylphosphonate analogs of DNA (see also U.S. Patents 5,176,996; 5,264,564; and 5,256,775). Additionally, general approaches to constructing oligomers useful in antisense therapy have been reviewed, for example, by Van der Krol *et al*. (1988) BioTechniques 6:958-976; and Stein *et al*. (1988) Cancer Res 48:2659-2668. With respect to antisense DNA, oligodeoxyribonucleotides derived from the translation initiation site, e.g., between the -10 and +10 regions of the nucleotide sequence of interest, are preferred.

Antisense approaches involve the design of oligonucleotides (either DNA or RNA) that are complementary to mRNA. The antisense oligonucleotides will bind to the mRNA transcripts and prevent translation. Absolute complementarity, although preferred, is not required. In the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

Oligonucleotides that are complementary to the 5' end of the mRNA, e.g., the 5' untranslated sequence up to and including the AUG initiation codon, should work most efficiently at inhibiting translation. However, sequences complementary to the 3' untranslated sequences of mRNAs have recently been shown to be effective at inhibiting translation of mRNAs as well. (Wagner, R. 1994. Nature 372:333). Therefore, oligonucleotides complementary to either the 5' or 3' untranslated, non - coding regions of a gene could be used in an antisense approach to inhibit translation of endogenous mRNA. Oligonucleotides complementary to the 5' untranslated region of the mRNA should include the complement of the AUG start codon. Antisense oligonucleotides complementary to mRNA coding regions are typically less efficient inhibitors of translation but could also be used in accordance with the invention. Whether designed to hybridize to the 5', 3', or coding region of subject mRNA, antisense nucleic acids should be at least six nucleotides in length, and are preferably less that about 100 and more preferably less than about 50,25, 17 or 10 nucleotides in length.

Regardless of the choice of target sequence, it is preferred that *in vitro* studies are first performed to quantitate the ability of the antisense oligonucleotide to quantitate the ability of the antisense oligonucleotide to inhibit gene expression. It is preferred that these studies utilize controls that distinguish between antisense gene inhibition and nonspecific biological effects of oligonucleotides. It is also preferred that these studies compare levels of the target RNA or protein with that of an internal control RNA or protein. Additionally, it is envisioned that results obtained using the antisense oligonucleotide are compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridization to the target sequence.

The oligonucleotides can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides (e.g., for targeting host cell receptors), or agents facilitating transport across the cell membrane (see, e.g., Letsinger *et al*., 1989, Proc. Natl. Acad. Sci. U.S.A. 86:6553-6556; Lemaitre *et al*., 1987, Proc. Natl. Acad. Sci. 84:648-652; PCT Publication No. WO 88/098 10, published December 15, 1988) or the blood-brain barrier (see, e.g., PCT Publication No. WO 89/10 134, published April 25, 1988), hybridization-triggered cleavage agents (See, e.g., Krol *et al*., 1988, BioTechniques 6:958-976), or intercalating agents (See, e.g., Zon, 1988, Pharm. Res. 5:539-549). To this end, the oligonucleotide may be conjugated to another molecule, e.g., a peptide, hybridization triggered cross-linking agent, transport agent, hybridization-triggered cleavage agent, etc.

The antisense oligonucleotide may comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxytriethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including but not limited to arabinose, 2-fluoroarabinose, xylulose, and hexose.

The antisense oligonucleotide can also contain a neutral peptide-like backbone. Such molecules are termed peptide nucleic acid (PNA)-oligomers and are described, e.g., in Peny-O'Keefe *et al*. (1996) Proc. Natl. Acad. Sci. U.S.A. 93:14670 and in Eglom *et al*. (1993) Nature 365:566. One advantage of PNA oligomers is their capability to bind to complementary DNA essentially independently from the ionic strength of the medium due to the neutral backbone of the DNA. In yet another embodiment, the antisense oligonucleotide comprises at least one modified phosphate backbone selected from the group consisting of a phosphorothioate, a phosphorodithioate, a phosphoramidothioate, a phosphoramidate, a phosphordiamidate, a methyiphosphonate, an alkyl phosphotriester, and a formacetal or analog thereof.

In yet a further embodiment, the antisense oligonucleotide is an a-anomeric oligonucleotide. An a-anomeric oligonucleotide forms specific double-stranded hybrids with complementary RNA in which, contrary to the usual n-units, the strands run parallel to each other (Gautier *et al*, 1987, Nucl. Acids Res. 15:6625-6641). The oligonucleotide is a 2'-O-methylribonucleotide (Inoue *et al*., 1987, Nucl. Acids Res. 15:6131-12148), or a chimeric RNA-DNA analogue (Jnoue *et al*., 1987, FEBS Lett. 215:327-330).

Oligonucleotides of the invention may be synthesized by standard methods known in the art, e.g., by use of an automated DNA synthesizer (such as are commercially available from Biosearch, Applied Biosystems, etc.). As examples, phosphorothioate oligonucleotides may be synthesized by the method of Stein *et al*. (1988, Nucl. Acids Res. 16:3209), methylphosphonate olgonucleotides can be prepared by use of controlled pore glass polymer supports *(Sarin et al*., 1988, Proc. Natl. Acad. Sci. U.S.A. 85:7448-7451), etc.

While antisense nucleotides complementary to a coding region sequence can be used, those complementary to the transcribed untranslated region and to the region comprising the initiating methionine are most preferred.

The antisense molecules can be delivered to cells which express the target nucleic acid *in vivo.* A number of methods have been developed for delivering antisense DNA or RNA to cells; e.g., antisense molecules can be injected directly into the tissue site, or modified antisense molecules, designed to target the desired cells (e.g., antisense linked to peptides or antibodies that specifically bind receptors or antigens expressed on the target cell surface) can be administered systemically.

However, it is often difficult to achieve intracellular concentrations of the antisense sufficient to suppress translation on endogenous mRNAs. Therefore, a preferred approach utilizes a recombinant DNA construct in which the antisense oligonucleotide is placed under the control of a strong pol III or pol II promoter. The use of such a construct to transfect target cells in the patient will result in the transcription of sufficient amounts of single stranded RNAs that will form complementary base pairs with the endogenous transcripts and thereby prevent translation of the target mRNA. For example, a vector can be introduced in vivo such that it is taken up by a cell and directs the transcription of an antisense RNA. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art for replication and expression in mammalian cells. Expression of the sequence encoding the antisense RNA can be by any promoter known in the art to act in mammalian, preferably human cells. Such promoters can be inducible or constitutive. Such promoters include but are not limited to: the SV40 early promoter region (Bernoist and Chambon, 1981, Nature 290:304-3 10), the promoter contained in the 3' long terminal repeat of Rous sarcoma virus (Yamamoto *et al*., 1980, Cell 22:787-797), the herpes thymidine kinase promoter *(Wagner et al*., 1981, Proc. Natl. Acad. Sci. U.S.A. 78: 1441-1445), the regulatory sequences of the metallothionein gene (Brinster et at, 1982, Nature 296:39-42), etc. Any type of plasmid, cosmid, YAC or viral vector can be used to prepare the recombinant DNA construct which can be introduced directly into the tissue site; e.g., the choroid plexus or hypothalamus. Alternatively, viral vectors can be used which selectively infect the desired tissue (e.g., for brain, herpesvirus vectors may be used), in which case administration may be accomplished by another route (e.g., systemically).

In another aspect of the invention, ribozyme molecules designed to catalytically cleave target mRNA transcripts can be used to prevent translation of target mRNA and expression of a target protein (See, e.g., PCT International Publication WO90/11364, published October 4, 1990; Sarver *et al*., 1990, Science 247:1222-1225 and U.S. Patent No. 5,093,246). While ribozymes that cleave mRNA at site specific recognition sequences can be used to destroy target mRNAs, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach, 1988, Nature, 334:585-591. Preferably the ribozyme is engineered so that the cleavage recognition site is located near the 5' end of the target mRNA; i.e., to increase efficiency and minimize the intracellular accumulation of non- functional mRNA transcripts.

The ribozymes of the present invention also include RNA endoribonucleases (hereinafter "Cech-type ribozymes") such as the one which occurs naturally in *Tetrahymena thermophila* (known as the IVS, or L-19 IVS RNA) and which has been extensively described by Thomas Cech and collaborators (Zaug, et al., 1984, Science, 224:574-578; Zaug and Cech, 1986, Science, 231:470-475; Zaug, et al., 1986, Nature, 324:429-433; published International patent application No. W088/04300 by University Patents Inc.; Been and Cech, 1986, Cell, 47:207-216). The Cech-type ribozymes have an eight base pair active site which hybridizes to a target RNA sequence whereafter cleavage of the target RNA takes place. The invention encompasses those Cech-type ribozymes which target eight base-pair active site sequences that are present in a target gene.

As in the antisense approach, the ribozymes can be composed of modified oligonucleotides (e.g., for improved stability, targeting, etc.) and should be delivered to cells which express the target gene *in vivo.* A preferred method of delivery involves using a DNA construct "encoding" the ribozyme under the control of a strong constitutive pol III or pol II promoter, so that transfected cells will produce sufficient quantities of the ribozyme to destroy endogenous messages and inhibit translation. Because ribozymes, unlike antisense molecules, are catalytic, a lower intracellular concentration is required for efficiency.

Antisense RNA, DNA, and ribozyme molecules of the invention may be prepared by any method known in the art for the synthesis of DNA and RNA molecules. These include techniques for chemically synthesizing oligodeoxyribonucleotides and oligoribonucleotides well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

Moreover, various well-known modifications to nucleic acid molecules may be introduced as a means of increasing intracellular stability and half-life. Possible modifications include but are not limited to the addition of flanking sequences of ribonucleotides or deoxyribonucleotides to the 5' and/or 3' ends of the molecule or the use of phosphorothioate or 2' 0-methyl rather than phosphodiesterase linkages within the oligodeoxyribonucleotide backbone.

The present invention makes available isolated polypeptides which are isolated from, or otherwise substantially free of other cellular proteins, especially other signal transduction factors and/or transcription factors which may normally be associated with the polypeptide. Subject polypeptides of the present invention include polypeptides encoded by full length cDNA sequences of the nucleic acids of SEQ ID Nos. 9-16, or a sequence complementary thereto. Preferred polypeptides are those having the amino acid sequence of SEQ ID Nos. 17-24. Polypeptides of the present invention include those proteins which are differentially regulated in tumor cells, especially colon cancer-derived cell lines (relative to normal cells, e.g., normal colon tissue and non-colon tissue). In preferred embodiments, the polypeptides are upregulated in tumor cells, especially colon cancer cancer-derived cell lines. In other embodiments, the polypeptides are downregulated in tumor cells, especially colon cancer-derived cell lines. Proteins which are upregulated, such as oncogenes, or downregulated, such as tumor suppressors, in aberrantly proliferating cells may be targets for diagnostic or therapeutic techniques. For example, upregulation of the *cdc2* gene induces mitosis. Overexpression of the *mytl* gene, a mitotic deactivator, negatively regulates the activity of *cdc2*. Aberrant proliferation may thus be induced either by upregulating *cdc2* or by downregulating *mytl*.

The term "substantially free of other cellular proteins" (also referred to herein as "contaminating proteins") or "substantially pure or purified preparations" are defined as encompassing preparations of polypeptides having less than about 20% (by dry weight) contaminating protein, and preferably having less than about 5% contaminating protein. Functional forms of the subject polypeptides can be prepared, for the first time, as purified preparations by using a cloned nucleic acid as described herein. Full length proteins or fragments corresponding to one or more particular motifs and/or domains or to arbitrary sizes, for example, at least about 5, 10, 25, 50, 75, or 100 amino acids in length are within the scope of the present invention.

Preferred polypeptides are those that are encoded by nucleic acid sequences at least about 70%, 75%, 80%, 90%, 95%, 97%, or 98% identical to a mRNA sequence complementary to the nucleic acid sequence of SEQ ID Nos. 9-16, particularly preferred polypeptides are those of SEQ ID Nos. 17-24.

Isolated peptidyl portions of proteins can be obtained by screening peptides recombinantly produced from the corresponding fragment of the nucleic acid encoding such peptides. In addition, fragments can be chemically synthesized using techniques known in the art such as conventional Merrifield solid phase f-Moc or t-Boc chemistry. For example, a polypeptide of the present invention may be arbitrarily divided into fragments of desired length with no overlap of the fragments, or preferably divided into overlapping fragments of a desired length. The fragments can be produced (recombinantly or by chemical synthesis) and tested to identify those peptidyl fragments which can function as either agonists or antagonists of a wild-type (e.g., "authentic") protein.

Another aspect of the present invention concerns recombinant forms of the subject proteins. Recombinant polypeptides preferred by the present invention, in addition to native proteins, as described above are encoded by a nucleic acid, which is at least about 60%, more preferably at least about 80%, and more preferably about 85%, and more preferably about 90%, and more preferably about 95% identical to an amino acid sequence encoded by SEQ ID Nos. 9-16, or a sequence complementary thereto. Polypeptides which are encoded by a nucleic acid that is at least about 98-99% identical with the sequence of SEQ ID Nos. 9-16, or a sequence complementary thereto are also within the scope of the invention. Also included in the present invention are peptide fragments comprising at least a portion of such a protein.

In a preferred embodiment, a polypeptide of the present invention is a mammalian polypeptide and even more preferably a human polypeptide. In particularly preferred embodiment, the polypeptide retains wild-type bioactivity. It will be understood that certain post-translational modifications, e.g., phosphorylation and the like, can increase the apparent molecular weight of the polypeptide relative to the unmodified polypeptide chain. In a preferred embodiment, a polypeptide of the present invention has the sequence of SEQ ID Nos. 17-24.

The present invention further pertains to recombinant forms of one of the subject polypeptides. Such recombinant polypeptides preferably are capable of functioning in one of either role of antagonist or antagonist of at least one biological activity of a wild-type ("authentic") polypeptide of the appended sequence listing. The term "evolutionarily related to", with respect to amino acid sequences of proteins, refers to both polypeptides having amino acid sequences which have arisen naturally, and also to mutational variants of human polypeptides which are derived, for example, by combinatorial mutagenesis.

In general, polypeptides referred to herein as having an activity (e.g., are "bioactive") of a protein are defined as polypeptides which include an amino acid sequence of SEQ ID Nos. 17-24, and which mimic or antagonize all or a portion of the biological/biochemical activities of a naturally occurring protein. According to the present invention, a polypeptide has biological activity if it is a specific agonist or antagonist of a naturally occurring form of a protein.

Assays for determining whether a compound, e. g, a protein or variant thereof, has one or more of the above biological activities are well known in the art. In certain embodiments, the polypeptides of the present invention have activities such as those outlined above.

In another embodiment, the coding sequences for the polypeptide can be incorporated as a part of a fusion gene including a nucleotide sequence encoding a different polypeptide. This type of expression system can be useful under conditions where it is desirable to produce an immunogenic fragment of a polypeptide (see, for example, EP Publication No: 0259149; and Evans *et al*. (1989) Nature 339:3 85; Huang et at. (1988) J. Virol. 62:3 855; and Schlienger *et al*., (1992) J. Virol. 66:2). In addition to utilizing fusion proteins to enhance immunogenicity, it is widely appreciated that fusion proteins can also facilitate the expression of proteins, and, accordingly, can be used in the expression of the polypeptides of the present invention (see, for example, Current Protocols in Molecular Biology, eds. Ausubel et at. (N.Y. John Wiley & Sons, 1991)). In another embodiment, a fusion gene coding for a purification leader sequence, such as a poly-(His)/enterokinase cleavage site sequence at the N-terminus of the desired portion of the recombinant protein, can allow purification of the expressed fusion protein by affinity chromatography using a Ni²⁺metal resin. The purification leader sequence can then be subsequently removed by treatment with enterokinase to provide the purified protein (e.g., see Hochuli *et al*. (1987)J. Chromatography 411:177; and Janknecht *et al*. PNAS 88:8972).

Techniques for making fusion genes are known to those skilled in the art. Essentially, the joining of various DNA fragments coding for different polypeptide sequences is performed in accordance with conventional techniques, employing blunt-ended or stagger-ended termini for ligation, restriction enzyme digestion to provide for appropriate termini, filling -in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of nucleic acid fragments can be carried out using anchor primers which give rise to complementary overhangs between two consecutive nucleic acid fragments which can subsequently be annealed to generate a chimeric nucleic acid sequence (see, for example, Current Protocols in Molecular Biology, eds. *Ausubel et al*. John Wiley & Sons: 1992).

The present invention further pertains to methods of producing the subject polypeptides. For example, a host cell transfected with a nucleic acid vector directing expression of a nucleotide sequence encoding the subject polypeptides can be cultured under appropriate conditions to allow expression of the peptide to occur. Suitable media for cell culture are well known in the art. The recombinant polypeptide can be isolated from cell culture medium, host cells, or both using techniques known in the art for purifying proteins including ion-exchange chromatography, gel filtration chromatography, ultrafiltration, electrophoresis, and immunoaffinity purification with antibodies specific for such peptide. In a preferred embodiment, the recombinant polypeptide is a fusion protein containing a domain which facilitates its purification, such as GST fusion protein.

Moreover, it will be generally appreciated that, under certain circumstances, it may be advantageous to provide homologs of one of the subject polypeptides which function in a limited capacity as one of either an agonist (mimetic) or an antagonist, in order to promote or inhibit only a subset of the biological activities of the naturally occurring form of the protein. Thus, specific biological effects can be elicited by treatment with a homolog of limited function, and with fewer side effects relative to treatment with agonists or antagonists which are directed to all of the biological activities of naturally occurring forms of subject proteins.

Homologs of each of the subject polypeptide can be generated by mutagenesis, such as by discrete point mutation(s), or by truncation. For instance, mutation can give rise to homologs which retain substantially the same, or merely a subset, of the biological activity of the polypeptide from which it was derived. Alternatively, antagonistic forms of the polypeptide can be generated which are able to inhibit the function of the naturally occurring form of the protein, such as by competitively binding to a receptor.

The recombinant polypeptides of the present invention also include homologs of the wild-type proteins, such as versions of those proteins which are resistant to proteolytic cleavage, for example, due to mutations which alter ubiquitination or other enzymatic targeting associated with the protein.

Polypeptides may also be chemically modified to create derivatives by forming covalent or aggregate conjugates with other chemical moieties, such as glycosyl groups, lipids, phosphate, acetyl groups and the like. Covalent derivatives of proteins can be prepared by linking the chemical moieties to functional groups on amino acid sidechains of the protein or at the N-terminus or at the C-terminus of the polypeptide.

Modification of the structure of the subject polypeptides can be for such purposes as enhancing therapeutic or prophylactic efficacy, stability (e.g., ex vivo shelf life and resistance to proteolytic degradation), or post-translational modifications (e.g., to alter phosphorylation pattern of protein). Such modified peptides, when designed to retain at least one activity of the naturally occurring form of the protein, or to produce specific antagonists thereof, are considered functional equivalents of the polypeptides described in more detail herein. Such modified peptides can be produced, for instance, by amino acid substitution, deletion, or addition. The substitutional variant may be a substituted conserved amino acid or a substituted non-conserved amino acid.

For example, it is reasonable to expect that an isolated replacement of a leucine with an isoleucine or valine, an aspartate with a glutamate, a threonine with a serine, or a similar replacement of an amino acid with a structurally related amino acid (i.e., isosteric and/or isoelectric mutations) will not have a major effect on the biological activity of the resulting molecule. Conservative replacements are those that take place within a family of amino acids that are related in their side chains. Genetically encoded amino acids can be divided into four families: (1) acidic = aspartate, glutamate; (2) basic = lysine, arginine, histidine; (3) nonpolar = alanine, valine, leucine, isoleucine, proline, phenylalanine, methionine, tryptophan; and (4) uncharged polar = glycine, asparagine, glutamine, cysteine, serine, threonine, tyrosine. In similar fashion, the amino acid repertoire can be grouped as (1) acidic = aspartate, glutamate; (2) basic = lysine, arginine histidine, (3) aliphatic = glycine, alanine, valine, leucine, isoleucine, serine, threonine, with serine and threonine optionally be grouped separately as aliphatic-hydroxyl; (4) aromatic = phenylalanine, tyrosine, tiyptophan; (5) amide = asparagine, glutamine; and (6) sulfur -containing = cysteine and methionine. (see, for example, *Biochemistry, 2* ed., Ed. by L. Stryer, WH Freeman and Co.: 1981). Whether a change in the amino acid sequence of a peptide results in a functional homolog (e.g., functional in the sense that the resulting polypeptide mimics or antagonizes the wild-type form) can be readily determined by assessing the ability of the variant peptide to produce a response in cells in a fashion similar to the wild-type protein, or competitively inhibit such a response.

Polypeptides in which more than one replacement has taken place can readily be tested in the same manner. The variant may be designed so as to retain biological activity of a particular region of the protein. In a non-limiting example, *Osawa et al*., 1994, *Biochem*istry and Molecular International 34:1003-1009, discusses the actin binding region of a protein from several different species. The actin binding regions of the these species are considered homologous based on the fact that they have amino acids that fall within "homologous residue groups." Homologous residues are judged according to the following groups (using single letter amino acid designations): STAG; IL VMF; HRK; DEQN; and FYW. For example, an S, a T, an A or a G can be in a position and the function (in this case actin binding) is retained.

Additional guidance on amino acid substitution is available from studies of protein evolution. Go et al., 1980, Int. J. Peptide Protein Res. 15: 211-224, classified amino acid residue sites as interior or exterior depending on their accessibility. More frequent substitution on exterior sites was confirmed to be general in eight sets of homologous protein families regardless of their biological functions and the presence or absence of a prosthetic group. Virtually all types of amino acid residues had higher mutabilities on the exterior than in the interior. No correlation between mutability and polarity was observed of amino acid residues in the interior and exterior, respectively. Amino acid residues were classified into one of three groups depending on their polarity: polar (Arg, Lys, His, Gln, Asn, Asp, and Glu); weak polar (Ala, Pro, Gly, Thr, and Ser), and nonpolar (Cys, Val, Met, Ile, Leu, Phe, Tyr, and Trp). Amino acid replacements during protein evolution were very conservative: 88% or 76% of them in the interior or exterior, respectively, were within the same group of the three. Intergroup replacements are such that weak polar residues are replaced more often by nonpolar residues in the interior and more often by polar residues on the exterior.

Querol et al., 1996, Prot. Eng. 9:265-271, provides general rules for amino acid substitutions to enhance protein thermostability. New glycosylation sites can be introduced as discussed in Olsen and Thomsen, 1991, J. Gen. Microbiol. 137 :579-585. An additional disulfide bridge can be introduced, as discussed by Perry and Wetzel, 1984, Science 226:555-557; Pantoliano et al., 1987, Biochemistry 26:2077-2082; Matsumura et al., 1989, Nature 342:291-293; Nishikawa et al., 1990, Protein Eng. 3:443-448; Takagi et al., 1990, J. Biol. Chem, 265:6874-6878; Clarke et al., 1993, Biochemistry 32:4322-43299; and Wakarchuk et al., 1994, Protein Eng. 7:1379-1386.

An additional metal binding site can be introduced, according to Toma et al., 1991, Biochemistry 30:97-106, and Haezerbrouck et al., 1993, Protein Eng. 6:643-649. Substitutions with prolines in loops can be made according to Masul et al., 1994, Appl Env. Microbiol. 60:3579-3584; and Hardy et al., FEBS Lett. 317:89-92.

Cysteine-depleted muteins are considered variants within the scope of the invention. These variants can be constructed according to methods disclosed in U.S. Patent No. 4,959,314, which discloses how to substitute other amino acids for cysteines, and how to determine biological activity and effect of the substitution. Such methods are suitable for proteins according to this invention that have cysteine residues suitable for such substitutions, for example to eliminate disulfide bond formation.

To learn the identity and function of the gene that correlates with an nucleic acid, the nucleic acids or corresponding amino acid sequences can be screened against profiles of protein families. Such profiles focus on common structural motifs among proteins of each family. Publicly available profiles are described above. Additional or alternative profiles are described below.

In comparing a new nucleic acid with known sequences, several alignment tools are available. Examples include PileUp, which creates a multiple sequence alignment, and is described in Feng et al., J. Mol. Evol. (1987) 25:35 1-360. Another method, GAP, uses the alignment method of Needleman et al., J. Mol. Biol. (1970) 48:443-453. GAP is best suited for global alignment of sequences. A third method, BestFit, functions by inserting gaps to maximize the number of matches using the local homology algorithm of Smith and Waterman, Adv. Appl. Math. (1981) 2:482 - 489.

Examples of such profiles are described below.

Chemokines. Chemokines are a family of proteins that have been implicated in lymphocyte trafficking, inflammatory diseases, angiogenesis, hematopoiesis, and viral infection. See, for example, Rollins, *Blood* (1997) 90(*3*):909-928, and Wells *et al*., *J*. *Leuk. Biol*. (1997) *61*:545-550. U.S. Patent No. 5,605,817 discloses DNA encoding a chemokine expressed in fetal spleen. U.S. Patent No. 5,656,724 discloses chemokine-like proteins and methods of use. U.S. Patent No. 5,602,008 discloses DNA encoding a chemokine expressed by liver.

Mutants of the encoded chemokines are polypeptides having an amino acid sequence that possesses at least one amino acid substitution, addition, or deletion as compared to native chemokines. Fragments possess the same amino acid sequence of the native chemokines; mutants may lack the amino and/or carboxyl terminal sequences. Fusions are mutants, fragments, or the native chemokines that also include amino and/or carboxyl terminal amino acid extensions.

The number or type of the amino acid changes is not critical, nor is the length or number of the amino acid deletions, or amino acid extensions that are incorporated in the chemokines as compared to the native chemokine amino acid sequences. A polynucleotide encoding one of these variant polypeptides will retain at least about 80% amino acid identity with at least one known chemokine. Preferably, these polypeptides will retain at least about 85% amino acid sequence identity, more preferably, at least about 90%; even more preferably, at least about 95%. In addition, the variants will exhibit at least 80%; preferably about 90%; more preferably about 95% of at least one activity exhibited by a native chemokine. Chemokine activity includes immunological, biological, receptor binding, and signal transduction functions of the native chemokine.

Chemotaxis. Assays for chemotaxis relating to neutrophils are described in Walz *et al*., *Biochem. Biophys. Res. Commun.* (1987) *149*:755, Yoshimura *et al*., *Proc*. *Natl*. *Acad. Sci. (USA)* (1987) *84*:9233, and Schroder *et al*., *J*. *Immunol.* (1987) *139*:3474; to lymphocytes, Larsen *et al*., *Science* (1989) *243*:1464, Carr *et al*., *Proc. Natl. Acad. Sci.* (*USA*) (1994) *91*:3652; to tumor-infiltrating lymphocytes, Liao *et al*., *J. Exp. Med.* (*1995*). *182*:1301; to hemopoietic progenitors, Aiuti *et al*., *J*. *Exp. Med*. (1997) *185*:111; to monocytes, Valente *et al*., *Biochem.* (1988) *27*:4162; and to natural killer cells, Loetscher *et al*., *J. Immunol*. (1996) 156:322, and Allavena *et al*., *Eur*. *J*. *Immunol.* (1994) *24*:3233.

Assays for determining the biological activity of attracting eosinophils are described in *Dahinden et al*, *J*. *Exp. Med.* (1994) 179:751, *Weber et al*., *J Immunol*. (1995) *154*:4166, and Noso *et al*., *Biochem*. *Biophys. Res. Commun.* (1994) *200*:*1470*; for attracting dendritic cells, Sozzani *et al*., *J. Immunol.* (1995) 155:3292; for attracting basophils, in *Dahinden et al*., *J*. *Exp. Med.* (1994) *179*:751, Alam *et al*., *J*. *Immunol.* (1994) 152:1298, Alam *et al*., *J*. *Exp. Med.* (1992) 176:781; and for activating neutrophils, Maghazaci *et al*., *Eur. J*. *Immunol.* (1996) 26:315, and Taub *et al*, *J*. *Immunol.* (1995) 155:3877. Native chemokines can act as mitogens for fibroblasts, assayed as described in Mullenbach *et al*., *J*. *Biol*. *Chem*. (1986) 261:719.

Receptor Binding. Native chemokines exhibit binding activity with a number of receptors. Description of such receptors and assays to detect binding are described in, for example, Murphy *et al*., *Science* (1991) 253:1280; Combadiere *et al*., *J*. *Biol*. *Chem.* (1995) 270:29671; Daugherty *et al*., *J. Exp. Med.* (1996) 183:2349; Samson *et al*., *Biochem.* (1996) 35:3362; Raport *et al*., *J*. *Biol*. *Chem.* (1996) 271:17161; Combadiere *et al*., *J*. *Leukoc. Biol*. (1996) *60* :147; Baba *et al*., *J*. *Biol*. *Chem*. (1997) 23: 14893; Yosida *et al*., *J*. *Biol. Chem.* (1997) 272:13803; Arvannitakis *et al*., Nature (1997) 385:347, and many other assays are known in the art.

Kinase Activation. Assays for kinase activation are described by *Yen et al*., *J*. *Leukoc. Biol*. (1997) 61:529; *Dubois et al*., *J*. *Immunol*. (1996) 156:1356; Turner *et al*., *J*. *Immunol*. (1995) 155:2437. Assays for inhibition of angiogenesis or cell proliferation are described in *Malone et al*., *Science* (1990) 247:77. Glycosaminoglycan production can be induced by native chemokines, assayed as described in *Castor et al*., *Proc. Natl*. *Acad*. *Sci. (USA)* (1983) *80:765.* Chemokine-mediated histamine release from basophils is assayed as described in *Dahinden et al*., *J*. *Exp. Med.* (1989) 170:1787; and White *et al*., *Immunol*. *Lett.* (1989) 22:151. Heparin binding is described in *Luster et al*., *J*. *Exp. Med* .(1995) 182:219.

Dimerization Activity. Chemokines can possess dimerization activity, which can be assayed according to Burrows *et al*., *Biochem* (1994) 33 12741, and Zhang *et al*., *Mol*. *Cell. Biol*. (1995) 15:4851. Native chemokines can play a role in the inflammatory response of viruses. This activity can be assayed as described in Bleul *et al*., *Nature* (1996) 382:829; and Oberlin *et al*., *Nature* (1996) 382:833. Exocytosis of monocytes can be promoted by native chemokines. The assay for such activity is described in Uguccioni *et al*., Eur. *J*. *Immunol*. (1995) 25:64. Native chemokines also can inhibit hemapoietic stem cell proliferation. The method for testing for such activity is reported in Graham *et al*., *Nature* (1990) 344:442.

Death Domain Proteins. Several protein families contain death domain motifs (Feinstein and Kimchi, *TIBS Letters* (1995) 20:242-244). Some death domain-containing proteins are implicated in cytotoxic intracellular signaling (Cleveland and Ihle, Cell (1995) 81:479-482, *Pan et al*., *Science* (1997) 276:111-113, Duan and Dixit, Nature (1997) 385:86-89, and Chinnaiyan *et al*., Science (1996) 274:990-992). U.S. Patent No. 5,563,039 describes a protein homologous to TRADD (Tumor Necrosis Factor Receptor-1 Associated Death Domain containing protein), and modifications of the active domain of TRADD that retain the functional characteristics of the protein, as well as apoptosis assays for testing the function of such death domain containing proteins. U.S. Patent No. 5,658,883 discloses biologically active TGF-B 1 peptides. U.S. Patent No. 5,674,734 discloses protein RIP which contains a C-terminal death domain and an N-terminal kinase domain.

Leukemia Inhibitory Factor (LIF). An LIF profile is constructed from sequences of leukemia inhibitor factor, CT-I (cardiotrophin-1), CNTF (ciliary neurotrophic factor), OSM (oncostatin M), and IL-6 (interleukin-6). This profile encompasses a family of secreted cytokines that have pleiotropic effects on many cell types including hepatocytes, osteoclasts, neuronal cells and cardiac myocytes, and can be used to detect additional genes encoding such proteins. These molecules are all structurally related and share a common co-receptor gp130 which mediates intracellular signal transduction by cytoplasmic tyrosine kinases such as src.

Novel proteins related to this family are also likely to be secreted, to activate gp130 and to function in the development of a variety of cell types. Thus new members of this family would be candidates to be developed as growth or survival factors for the cell types that they stimulate. For more details on this family of cytokines, see Pennica *et al*., *Cytokine and Growth Factor Reviews* (1996) 7:81-91. U.S. Patent No. 5,420,247 discloses LIF receptor and fusion proteins. U.S. Patent No. 5,443,825 discloses human LIP.

Angiopoietin. Angiopoietin-1 is a secreted ligand of the TIE-2 tyrosine kinase; it functions as an angiogenic factor critical for normal vascular development. Angiopoietin-2 is a natural antagonist of angiopoietin-1 and thus functions as an antiangiogenic factor. These two proteins are structurally similar and activate the same receptor. (Folkman and D'Amore, *Cell* (1996) 87:1153-1155, and *Davis et al*., *Cell* (1996) 87:1161-1 169.)

The angiopoietin molecules are composed of two domains, a coiled-coil region and a region related to fibrinogen. The fibrinogen domain is found in many molecules including ficolin and tesascin, and is well defined structurally with many members.

Receptor Protein-Tyrosine Kinases. Receptor Protein-Tyrosine Kinases or RPTKs are described in Lindberg. *Annu. Rev*. *Cell Biol*. (1994) 10:25 1-337.

Growth Factors: Epidermal Growth Factor (EGF) and Fibroblast Growth Factor (FGF). For a discussion of growth factor superfamilies, see Growth Factors: A Practical Approach. Appendix Al (Ed. McKay and Leigh, Oxford University Press, NY, 1993) pp. 237-243.

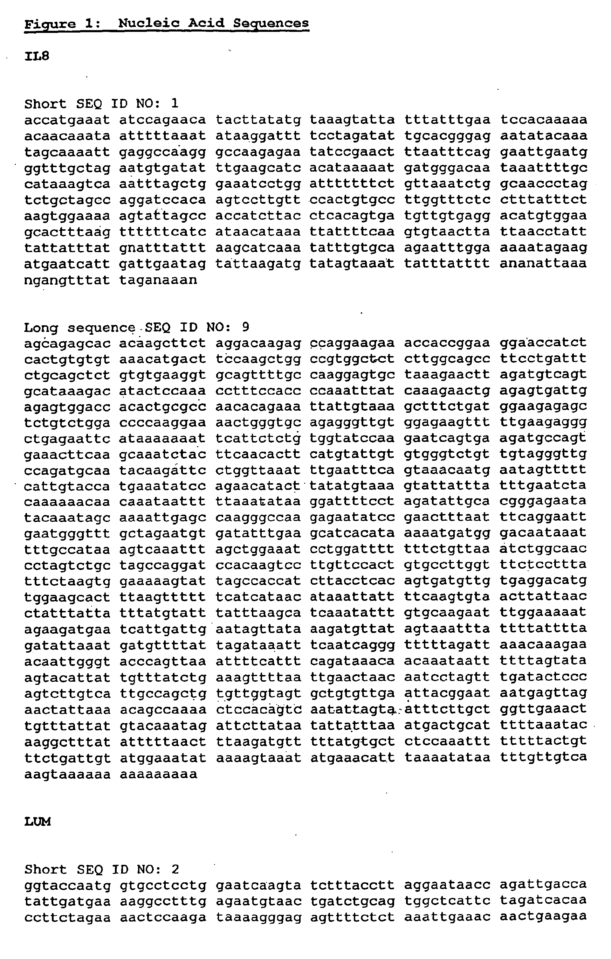

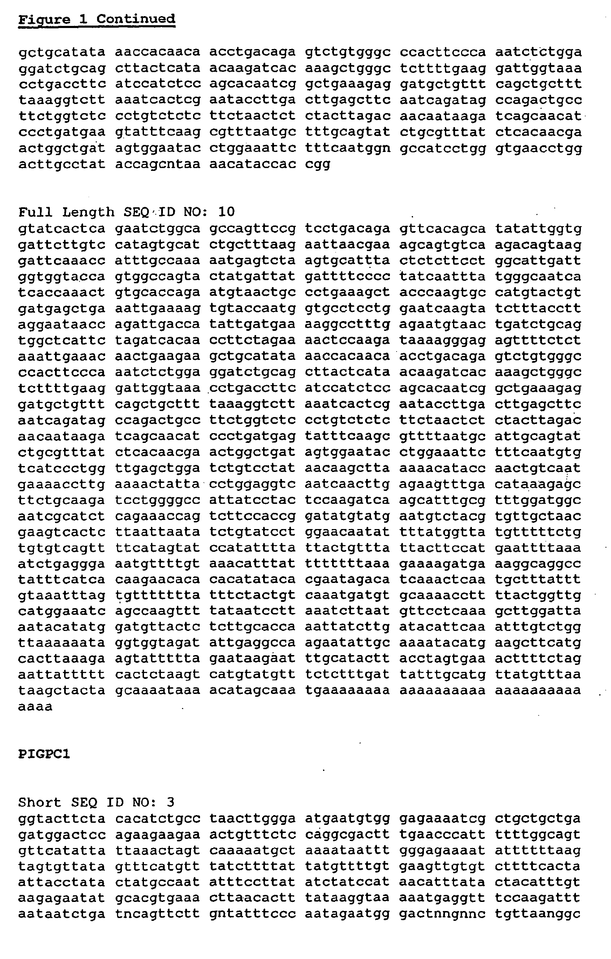

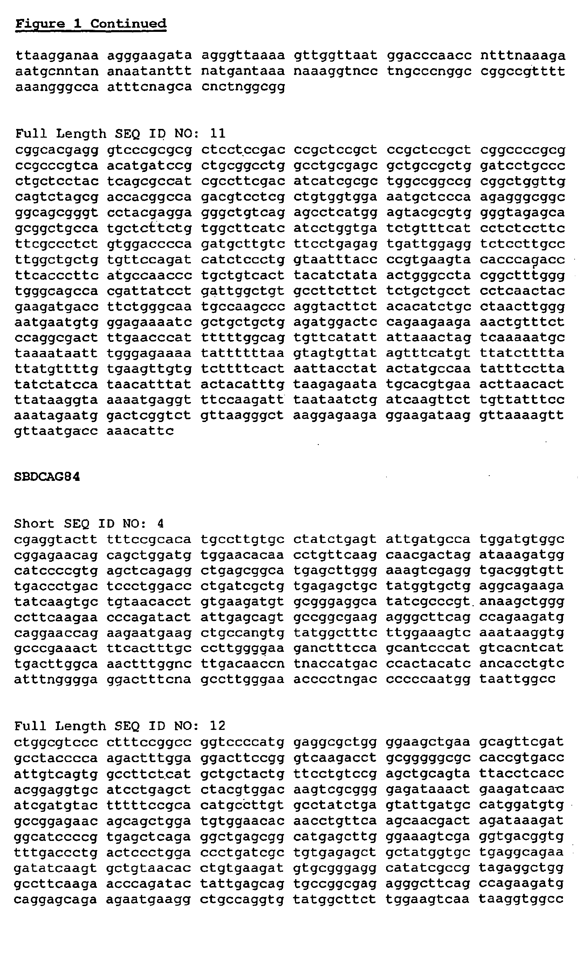

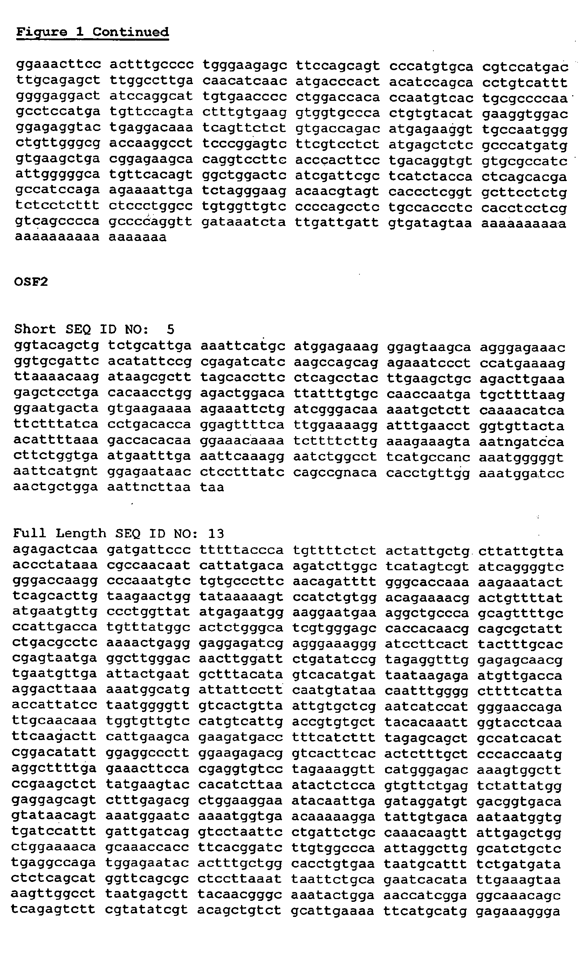

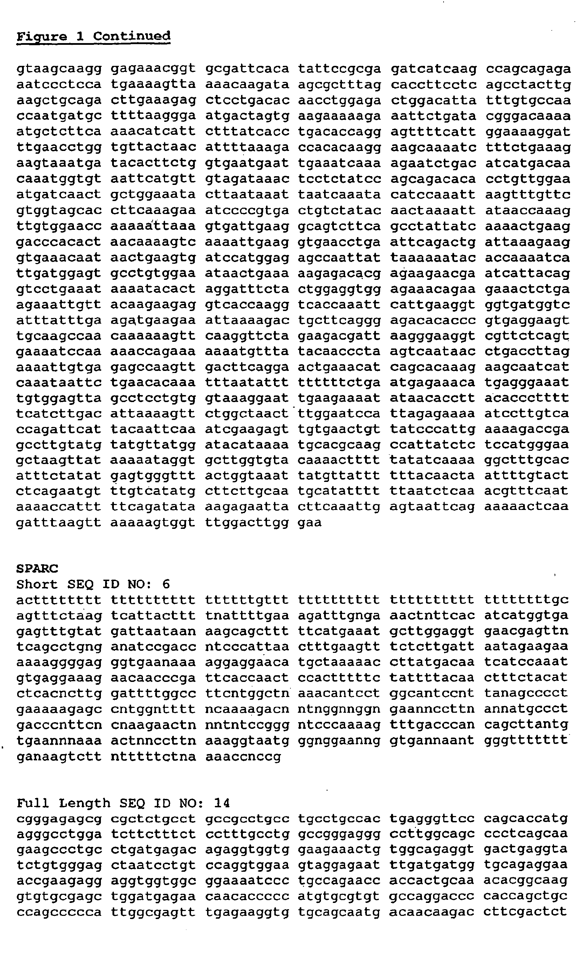

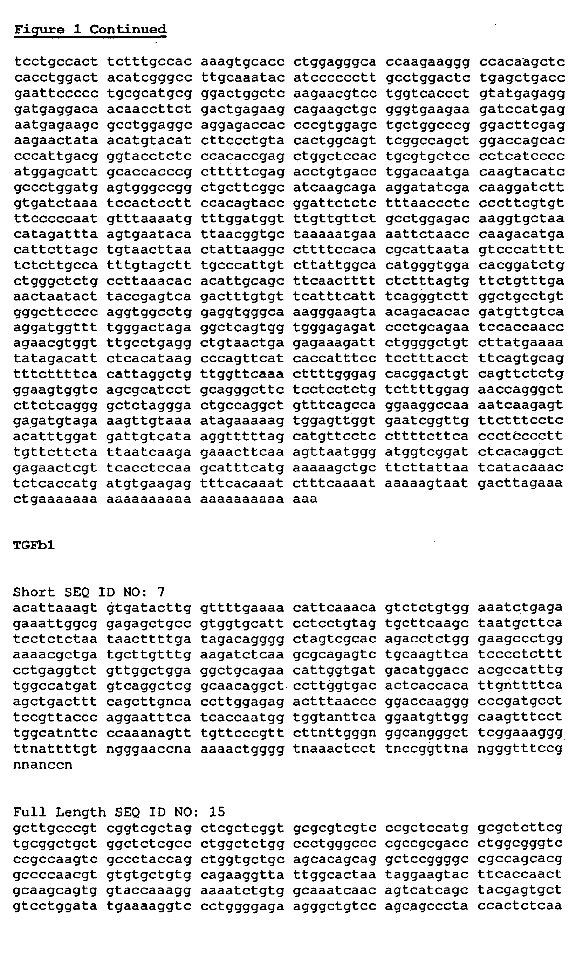

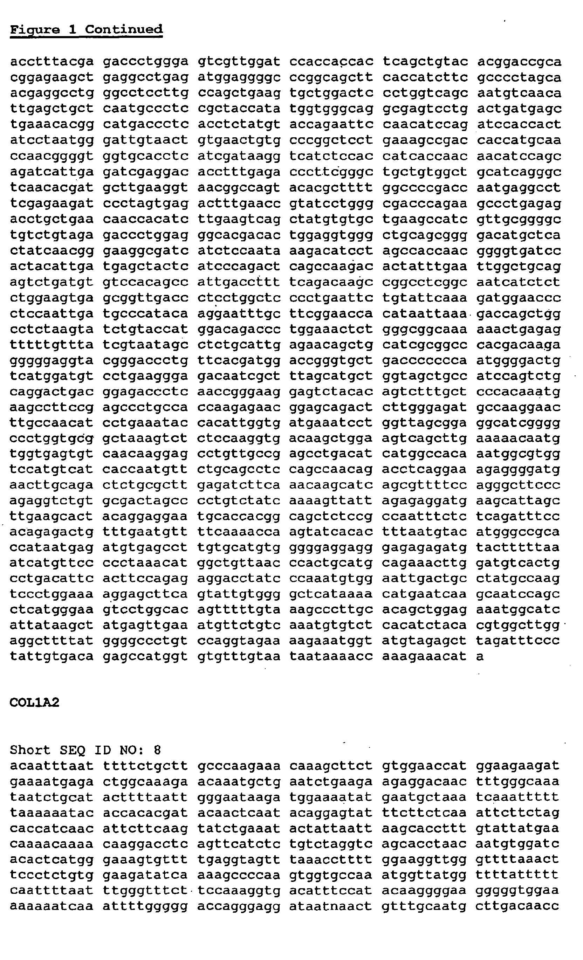

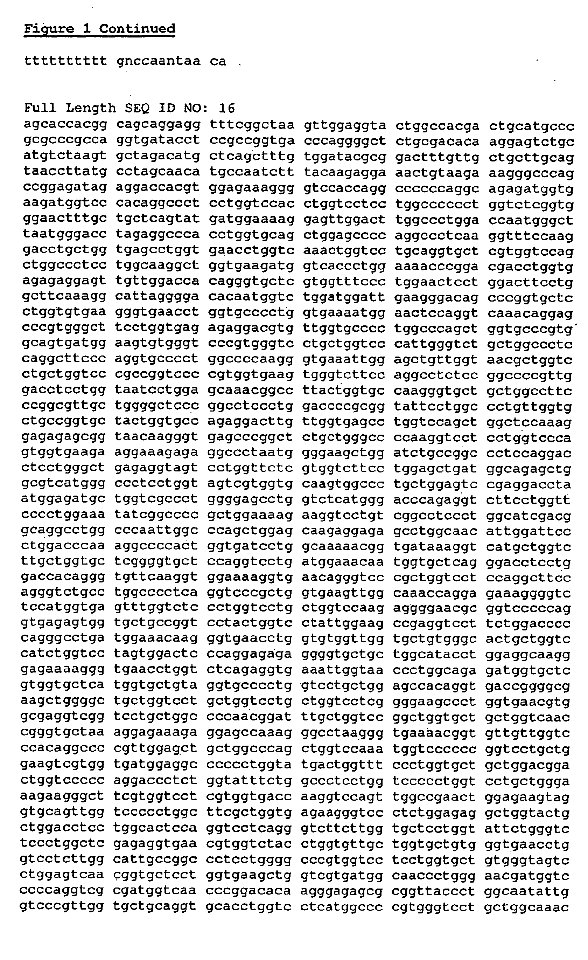

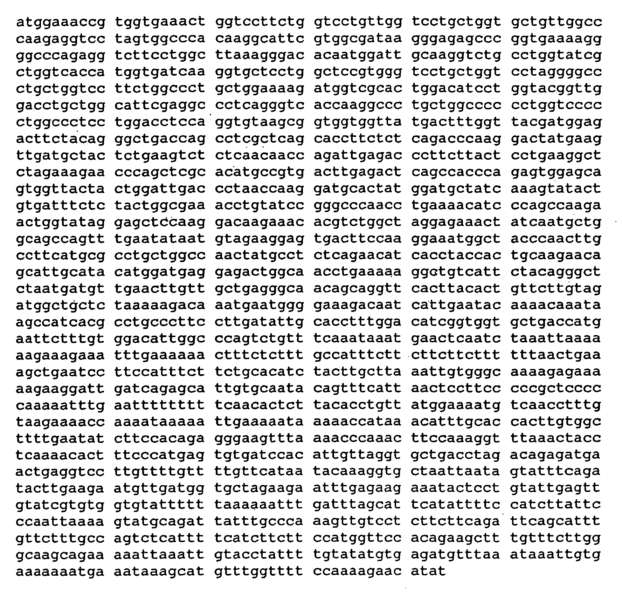

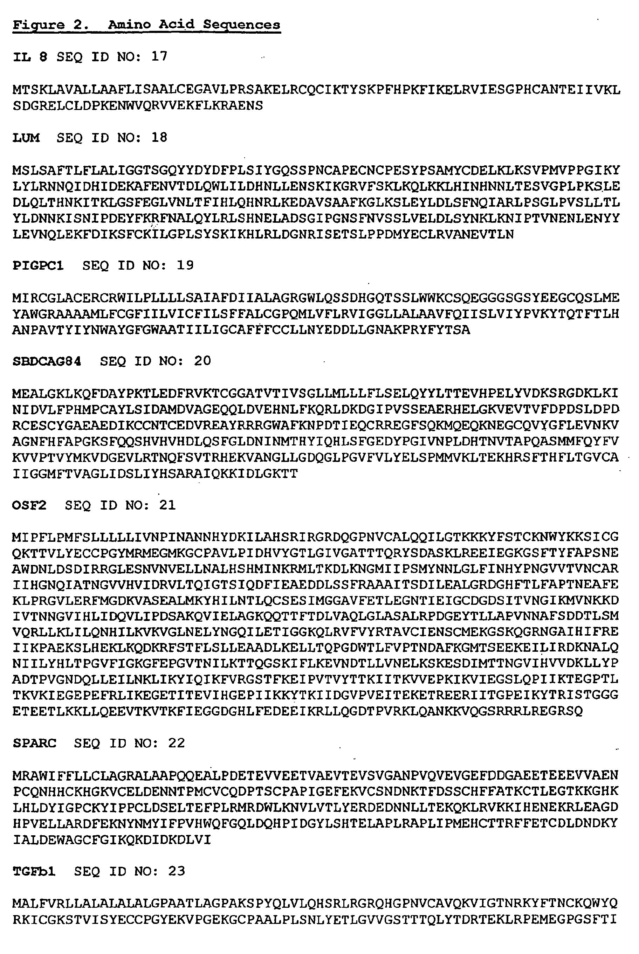

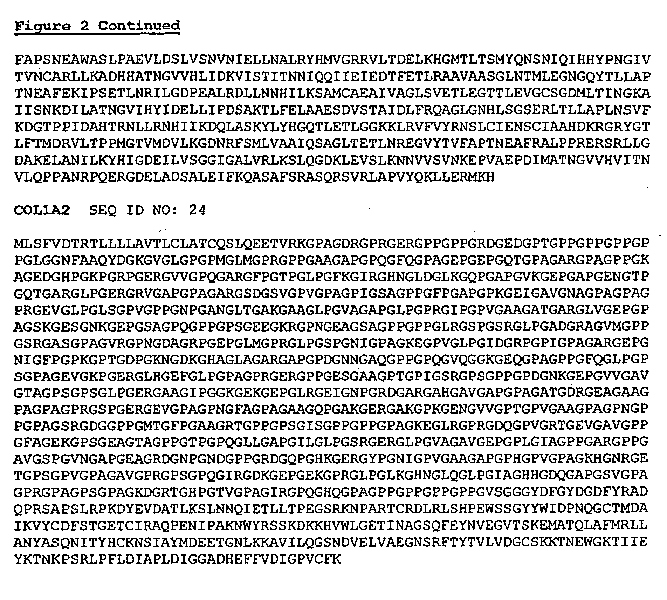

The alignments (pretty box) for EGF and FGF are shown in Figures 1 and 2, respectively. U.S. Patent No. 4,444,760 discloses acidic brain fibroblast growth factor, which is active in the promotion of cell division and wound healing. U.S. Patent No. 5,439,818 discloses DNA encoding human recombinant basic fibroblast growth factor, which is active in wound healing. U.S. Patent No. 5,604,293 discloses recombinant human basic fibroblast growth factor, which is useful for wound healing. U.S. Patent No. 5,410,832 discloses brain-derived and recombinant acidic fibroblast growth factor, which act as mitogens for mesoderm and neuroectoderm-derived cells in culture, and promote wound healing in soft tissue, cartilaginous tissue and musculo-skeletal tissue. U.S. Patent No. 5,387,673 discloses biologically active fragments of FGF that retain activity.

Proteins of the TNF Family. A profile derived from the TNF family is created by aligning sequences of the following TNF family members: nerve growth factor (NGF), lymphotoxin, Fas ligand, tumor necrosis factor (TNF), CD4O ligand, TRAIL, ox40 ligand, 4-IBB ligand, CD27 ligand, and CD30 ligand. The profile is designed to identify sequences of proteins that constitute new members or homologues of this family of proteins.

U.S. Patent No. 5,606,023 discloses mutant TNF proteins; U.S. Patent No. 5,597,899 and U.S. Patent No. 5,486,463 disclose TNF muteins; and U.S. Patent No. 5,652,353 discloses DNA encoding TNF-a muteins.

Members of the TNF family of proteins have been show in vitro to multimerize, as described in *Burrows et al*., *Biochem.* (1994) 33:12741 and Zhang *et al*., *Mol*. *Cell. Biol*. (1995) 154851 and bind receptors as described in Browning *et al*., *J*. *Immunol.* (1994) 147:1230, Androlewicz *et al*., *J. Biol. Chem*.(1992) 267:2542, and *Crowe et al*., *Science* (1994) 264:707.

In vivo, TNFs proteolytically cleave a target protein as described in Kriegel *et al*., *Cell* (1988) 53:45 and Mohler *et al*., *Nature* (1994) 370:218 and demonstrate cell proliferation and differentiation activity. T-cell or thymocyte proliferation is assayed as described in *Armitage et al*., Eur. *J*. *Immunol.* (1992) 22:447; Current Protocols in Immunology, ed. J.E. Coligan *et al*., 3.1-3.19; Takai *et al*., *J*. *Immunol.* (1986) 137:3494-3500, Bertagnoli *et al*., *J*. *Immunol.* (1990) 145:1706-1712, Bertagnoli *et al*., *J*. *Immunol.* (1991) 133:327-340, Bertagnoli *et al*., *J*. *Immunol*. (1992) 149:3778-3783, and Bowman *et al*., *J*. *Immunol.* (1994) 152:1756-1761. B cell proliferation and Ig secretion are assayed as described in Maliszewski, *J*. *Immunol.* (1990) 144:3028-3033, and Assays for B Cell Function: *In vitro* antibody production, Mond and Brunswick, Current Protocols in Immunol., Coligan Ed vol 1 pp 3.8.1-3.8.16, John Wiley and Sons, Toronto 1994, *Kebrl et al*., Science (1987) 238:1144 and Boussiotis *et al*., PNAS USA (1994) 91 :7007.

Other in vivo activities include upregulation of cell surface antigens, upregulation of costimulatory molecules, and cellular aggregation/adhesion as described in Barrett *et al*., *J*. *Immunol*. (1991) 146:1722; Bjorck *et al*., Eur. *J*. *Immunol.* (1993) 23:1771; Clark *et al*., *Annu Rev. Immunol.* (1991) 9:97; Ranheim *et al*., J. Exp. Med. (1994) 177:925; Yellin, *J*. *Immunol.* (1994) 153:666; and Gruss *et al*., Blood (1994) 84:2305.

Proliferation and differentiation of hematopoietic and lymphopoietic cells has also been shown in vivo for TNFs, using assays for embryonic differentiation and hematopoiesis as described in Johansson *et al*., *Cellular Biology* (1995)15:141-151, Keller *et al*., *Mol. Cell. Biol.* (1993) 13:473-486, McClanahan *et al*., *Blood* (1993) 81 :2903-2915 and using assays to detect stem cell survival and differentiation as described in Culture of Hematopoietic Cells, *Freshney et al*. eds, pp 1-21, 23-29, 139-162, 163-179, and 265-268, Wiley-Liss, Inc., New York, NY, 1994, and Hirajama *et al*., *PNAS USA* (1992) 89:5907-5911.

In vivo activities of TNFs also include lymphocyte survival and apoptosis, assayed as described in Darzynkewicz *et al*., *Cytometry* (1992) 13:795-808; Gorczca *et al*., *Leukemia* (1993) 7:659-670; Itoh *et al*., *Cell* (1991) 66:233-243; Zacharduk, *J. Immunol*. (1990) 145:4037-4045; Zamai *et al*., *Cytometry* (1993) 14:891-897; and *Gorczyca et al*., *Int'l Oncol.* (1992) 1:639-648.

Some members of the TNF family are cleaved from the cell surface; others remain membrane bound. The three-dimensional structure of TNF is discussed in Sprang and Eck, Tumor Necrosis Factors; *supra*.

TNF proteins include a transmembrane domain. The protein is cleaved into a shorter soluble version, as described in Kriegler *et al*., Cell (1988) 53:45-53, *Perez et al*., *Cell* (1990) 63:251-258, and *Shaw et al*., Cell (1986) 46:659-667. The transmembrane domain is between amino acid 46 or 77 and the cytoplasmic domain is between position 1 and 45 on the human form of TNFα. The 3-dimensional motifs of TNF include a sandwich of two pleated 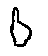 -sheets. Each sheet is composed of anti-parallel α-strands, α-Strands facing each other on opposite sites of the sandwich are connected by short polypeptide loops, as described in Van Ostade *et al*., Protein Engineering (1994) 7(1):5-22, and Sprang *et al*., Tumor Necrosis Factors; *supra*.

Residues of the TNF family proteins that are involved in the 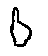 -sheet secondary structure have been identified as described in Van Ostade *et al*., *Protein Engineering* (1994) 7(1):5-22, and Sprang *et al*., Tumor Necrosis Factors; *supra*.

TNF receptors are disclosed in U.S. Patent No. 5,395,760. A profile derived from the TNF receptor family is created by aligning sequences of the TNF receptor family, including Apol/Fas, TNFR I and II, death receptor³ (DR3), CD4O, ox40, CD27, and CD30. Thus, the profile is designed to identify, from the nucleic acids of the invention, sequences of proteins that constitute new members or homologs of this family of proteins.

Tumor necrosis factor receptors exist in two forms in humans: p55 TNFR and p75 TNFR, both of which provide intracellular signals upon binding with a ligand. The extracellular domains of these receptor proteins are cysteine rich. The receptors can remain membrane bound, although some forms of the receptors are cleaved forming soluble receptors. The regulation, diagnostic, prognostic, and therapeutic value of soluble TNF receptors is discussed in Aderka, *Cytokine and Growth Factor Reviews,* (1996) 7(3):231-240.

PDGF Family U.S. Patent No. 5,326,695 discloses platelet derived growth factor agonists; bioactive portions of PDGF-B are used as agonists. U.S. Patent No. 4,845,075 discloses biologically active B-chain homodimers, and also includes variants and derivatives of the PDGF-B chain. U.S. Patent No. 5,128,321 discloses PDGF analogs and methods of use. Proteins having the same bioactivity as PDGF are disclosed, including A and B chain proteins.

Kinase (Including MKK) Family U.S. Patent No. 5,650,501 discloses serine/threonine kinase, associated with mitotic and meiotic cell division; the protein has a kinase domain in its N-terminal and 3 PEST regions in the C-terminus. U.S. Patent No. 5,605,825 discloses human PAK65, a serine protein kinase.

The foregoing discussion provides a few examples of the protein profiles that can be compared with the nucleic acids of the invention. One skilled in the art can use these and other protein profiles to identify the genes that correlate with the nucleic acids.

Ribozymes, antisense constructs, dominant negative mutants, and triplex formation can be used to determine function of the expression product of an nucleic acid-related gene.

### A. Ribozymes

Trans-cleaving catalytic RNAs (ribozymes) are RNA molecules possessing endoribonuclease activity. Ribozymes are specifically designed for a particular target, and the target message must contain a specific nucleotide sequence. They are engineered to cleave any RNA species site-specifically in the background of cellular RNA. The cleavage event renders the mRNA unstable and prevents protein expression. Importantly, ribozymes can be used to inhibit expression of a gene of unknown function for the purpose of determining its function in an *in vitro* or in vivo context, by detecting the phenotypic effect.

One commonly used ribozyme motif is the hammerhead, for which the substrate sequence requirements are minimal. Design of the hammerhead ribozyme is disclosed in Usman *et al*., *Current Opin. Struct. Biol.* (1996) 6:527-533. Usman also discusses the therapeutic uses of ribozymes. Ribozymes can also be prepared and used as described in Long *et al*., *FASEB J*. (1993) 7:25; Symons, *Ann. Rev. Biochem.* (1992) 61 :641; Perrotta *et al*., *Biochem.* (1992) 31:16-17; Ojwang *et al*., Proc. Natl. Acad. Sci. (USA) (1992) 89:10802-10806; and U.S. Patent No. 5,254,678. Ribozyme cleavage of HIV-I RNA is described in U.S. Patent No. 5,144,019; methods of cleaving RNA using ribozymes is described in U.S. Patent No. 5,116,742; and methods for increasing the specificity of ribozymes are described in U.S. Patent No. 5,225,337 and *Koizumi et al*., Nucleic Acid Res. (1989) 17:7059-7071. Preparation and use of ribozyme fragments in a hammerhead structure are also described by Koizumi *et al*., *Nucleic Acids Res.* (1989) 17:7059-7071. Preparation and use of ribozyme fragments in a hairpin structure are described by Chowrira and Burke, *Nucleic Acids Res.* (1992) 20:2835. Ribozymes can also be made by rolling transcription as described in Daubendiek and Kool, *Nat. Biotechnol.* (1997) 15(3):273-277.

The hybridizing region of the ribozyme may be modified or may be prepared as a branched structure as described in Horn and Urdea, *Nucleic Acids Res.* (1989) 17:6959-67. The basic structure of the ribozymes may also be chemically altered in ways familiar to those skilled in the art, and chemically synthesized ribozymes can be administered as synthetic oligonucleotide derivatives modified by monomeric units. In a therapeutic context, liposome mediated delivery of ribozymes improves cellular uptake, as described in Birikh *et al*., *Eur. J. Biochem*. (1997) 245:1-16.

Using the nucleic acid sequences of the invention and methods known in the art, ribozymes are designed to specifically bind and cut the corresponding mRNA species. Ribozymes thus provide a means to inhibit the expression of any of the proteins encoded by the disclosed nucleic acids or their full-length genes. The full-length gene need not be known in order to design and use specific inhibitory ribozymes. In the case of a nucleic acid or cDNA of unknown function, ribozymes corresponding to that nucleotide sequence can be *tested in vitro* for efficacy in cleaving the target transcript. Those ribozymes that effect cleavage *in vitro* are further tested in vivo. The ribozyme can also be used to generate an animal model for a disease, as described in Birikh *et al*., *Eur. J*. *Biochem.* (1997) 245:1-16. An effective ribozyme is used to determine the function of the gene of interest by blocking its transcription and detecting a change in the cell. Where the gene is found to be a mediator in a disease, an effective ribozyme is designed and delivered in a gene therapy for blocking transcription and expression of the gene.

Therapeutic and functional genomic applications of ribozymes proceed beginning with knowledge of a portion of the coding sequence of the gene to be inhibited. Thus, for many genes, a partial nucleic acid sequence provides adequate sequence for constructing an effective ribozyme. A target cleavage site is selected in the target sequence, and a ribozyme is constructed based on the 5' and 3' nucleotide sequences that flank the cleavage site. Retro viral vectors are engineered to express monomeric and multimeric hammerhead ribozymes targeting the mRNA of the target coding sequence. These monomeric and multimeric ribozymes are *tested in vitro* for an ability to cleave the target mRNA. A cell line is stably transduced with the retroviral vectors expressing the ribozymes, and the transduction is confirmed by Northern blot analysis and reverse-transcription polymerase chain reaction (RT-PCR). The cells are screened for inactivation of the target mRNA by such indicators as reduction of expression of disease markers or reduction of the gene product of the target mRNA.

### B. Antisense

Antisense nucleic acids are designed to specifically bind to RNA, resulting in the formation of RNA-DNA or RNA-RNA hybrids, with an arrest of DNA replication, reverse transcription or messenger RNA translation. Antisense polynucleotides based on a selected nucleic acid sequence can interfere with expression of the corresponding gene. Antisense polynucleotides are typically generated within the cell by expression from antisense constructs that contain the antisense nucleic acid strand as the transcribed strand. Antisense nucleic acids will bind and/or interfere with the translation of nucleic acid-related mRNA. The expression products of control cells and cells treated with the antisense construct are compared to detect the protein product of the gene corresponding to the nucleic acid. The protein is isolated and identified using routine *Biochem*ical methods.

One rationale for using antisense methods to determine the function of the gene corresponding to a nucleic acid is the biological activity of antisense therapeutics. Antisense therapy for a variety of cancers is in clinical phase and has been discussed extensively in the literature. Reed reviewed antisense therapy directed at the Bcl-2 gene in tumors; gene transfer-mediated overexpression of Bcl-2 in tumor cell lines conferred resistance to many types of cancer drugs. (Reed, J.C., *N*.*C*.*I*. (1997) 89:988-990). The potential for clinical development of antisense inhibitors of *ras* is discussed by Cowsert, L.M., *Anti-Cancer Drug Design* (1997) 12:359-371. Additional important antisense targets include leukemia (Geurtz, A.M., *Anti-Cancer Drug Design* (1997) 12:341-358); human C-ref kinase (Monia, B.P., *Anti-Cancer Drug Design* (1997) 12:327-339); and protein kinase C (McGraw *et al*., *Anti-Cancer Drug Design* (1997) 12:315-326.

Given the extensive background literature and clinical experience in anti sense therapy, one skilled in the art can use selected nucleic acids of the invention as additional potential therapeutics. The choice of nucleic acid can be narrowed by first testing them for binding to "hot spot" regions of the genome of cancerous cells. If a nucleic acid is identified as binding to a "hot spot", testing the nucleic acid as an antisense compound in the corresponding cancer cells clearly is warranted.

Ogunbiyi *et al*., *Gastroenterology* (1997) 113(3):761-766 describe prognostic use of audio loss in colon cancer; Barks *et al*., *Genes, Chromosomes, and Cancer* (1997) 19(4):278-285 describe increased chromosome copy number detected by FISH in malignant melanoma; Nishjzake *et al*., *Genes. Chromosomes, and Cancer* (1997) 19(4):267-272 describe genetic alterations in primary breast cancer and their metastases and direct comparison using modified comparative genome hybridization; and Elo *et al*., *Cancer Research* (1997) 57(16):3356-3359 disclose that loss of heterozygosity at 16z24.1-q24.2 is significantly associated with metastatic and aggressive behavior of prostate cancer.

### C. Dominant Negative Mutations

As an alternative method for identifying function of the nucleic acid-related gene, dominant negative mutations are readily generated for corresponding proteins that are active as homomultimers. A mutant polypeptide will interact with wild-type polypeptides (made from the other allele) and form a non- functional multimer. Thus, a mutation is in a substrate-binding domain, a catalytic domain, or a cellular localization domain. Preferably, the mutant polypeptide will be overproduced. Point mutations are made that have such an effect. In addition, fusion of different polypeptides of various lengths to the terminus of a protein can yield dominant negative mutants. General strategies are available for making dominant negative mutants. See Herskowitz, *Nature* (1987) 329:219-222. Such a technique can be used for creating a loss-of-function mutation, which is useful for determining the function of a protein.

### D. Triplex Formation

Endogenous gene expression can also be reduced by inactivating or "knocking out" the gene or its promoter using targeted homologous recombination. (E.g., see Smithies *et al*., 1985, Nature 317:230-234; Thomas & Capecchi, 1987, Cell 51:503-512; *Thompson et al*., 1989 Cell 5:313-321; each of which is incorporated by reference herein in its entirety). For example, a mutant, non-functional gene (or a completely unrelated DNA sequence) flanked by DNA homologous to the endogenous gene (either the coding regions or regulatory regions of the gene) can be used, with or without a selectable marker and/or a negative selectable marker, to transfect cells that express that gene in vivo. Insertion of the DNA construct, via targeted homologous recombination, results in inactivation of the gene.

Alternatively, endogenous gene expression can be reduced by targeting deoxyribonucleotide sequences complementary to the regulatory region of the target gene (i.e., the gene promoter and/or enhancers) to form triple helical structures that prevent transcription of the gene in target cells in the body. (See generally, Helene, C. 1991, Anticancer Drug Des., 6(6):569-84; Helene, C., *et al*., 1992, Ann, N.Y. Accad. Sci., 660:27-36; and Maher, L.J., 1992, Bioassays 14(12):807-15).

Nucleic acid molecules to be used in triple helix formation for the inhibition of transcription are preferably single stranded and composed of deoxyribonucleotides. The base composition of these oligonucleotides should promote triple helix formation via Hoogsteen base-pairing rules, which generally require sizable stretches of either purines or pyrimidines to be present on one strand of a duplex. Nucleotide sequences may be pyrimidine-based, which will result in TAT and CGC triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, containing a stretch of G residues. These molecules will form a triple helix with a DNA duplex that is rich in GC pairs, in which the majority of the purine residues are located on a single strand of the targeted duplex, resulting in CGC triplets across the three strands in the triplex.

Alternatively, the potential sequences that can be targeted for triple helix formation may be increased by creating a so called "switchback" nucleic acid molecule. Switchback molecules are synthesized in an alternating 5'-3', 3'-5' manner, such that they base pair with first one strand of a duplex and then the other, eliminating the necessity for a sizable stretch of either purines or pyrimidines to be present on one strand of a duplex.

Antisense RNA and DNA, ribozyme, and triple helix molecules of the invention may be prepared by any method known in the art for the synthesis of DNA and RNA molecules. These include techniques for chemically synthesizing oligodeoxyribonucleotides and oligoribonucleotides well known in the art such as for example solid phase phosphoramidite chemical synthesis. Alternatively, RNA molecules may be generated by *in vitro* and in vivo transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

Moreover, various well known modifications to nucleic acid molecules may be introduced as a means of increasing intracellular stability and half-life. Possible modifications include but are not limited to the addition of flanking sequences of ribonucleotides or deoxyribonucleotides to the 5' and/or 3' ends of the molecule or the use of phosphorothioate or 2' 0-methyl rather than phosphodiesterase linkages within the oligodeoxyribonucleotide backbone.

The present invention provides assays for the analysis of biological samples from cancer patients, and/or healthy patients, and/or patients suspected of having cancer to detect and determine the differential expression of a sequence comprising one or more of SEQ ID Nos 1-8 or 9-16, to determine if the level of expression of one or more of these gene sequences is elevated with respect to the level of expression in a normal patient. Alternatively, the invention provides assays for the analysis of biological samples from cancer patients, and/or healthy patients, and/or patients suspected of having cancer to detect and measure levels of protein encoded by one or more of SEQ ID Nos 9-16, or having the amino acid sequence of one or more of SEQ ID Nos 17-24 to determine if the levels of one or more of these proteins is increased in cancer patients compared to their respective levels in normal patients. The determination of a measureable or detectable increase in the level differential expression of one or more of SEQ ID Nos 1-8 or 9-16 , or an increase in protein expression of one or more of SEQ ID Nos 17-24 in a cancer patient compared to a normal patient provides a means of monitoring the patient's disease status, and/or patient response or benefit to cancer therapy, including both conventional anti-cancer and anti-neoplastic disease treatements and therapies (e.g., drugs, hormones, and the like), or treatments and therapies that more particularly target one or more of the gene sequence or encoded proteins of the invention.

As used herein, the term "biomarker" or "biological marker" refers to one or more of the polynucleotide sequences of SEQ ID Nos 1-8 or 9-16, and/or one or more of the polypeptide sequences of SEQ ID Nos 17-24. One of skill in the art will readily appreciate that the use of the term "biomarker", when referring, for example, to the detection of a biomarker by Northern analysis, will refer to the detection of one or more of the polynucleotide sequences of SEQ ID Nos 1-8 or 9-17, and will, thus, also appreciate that the use of the term "biomarker" to refer to the detection of, for example, biomarker expression by ELISA, refers to the detection of one or more of the polypeptide sequences of SEQ ID Nos 17-24.

The present invention provides method for determining whether a subject is at risk for developing a disease or condition characterized by unwanted cell proliferation by detecting the disclosed biomarkers, i.e., the disclosed nucleic acid markers (SEQ ID Nos: 1-8 or 9-16, or a sequence complementary thereto, or a sequence which hybridizes to one or more of SEQ ID Nos 1-8 or 9-16) and/or polypeptide markers for the disease or condition encoded thereby.

In clinical applications, human tissue samples can be screened for the presence and/or absence of the biomarkers identified herein. Such samples may comprise tissue samples, whole cells, cell lysates, or isolated nucleic acids, including, for example, needle biopsy cores, surgical resection samples, lymph node tissue, or serum. For example, these methods include obtaining a biopsy, which is optionally fractionated by cryostat sectioning to enrich tumor cells to about 80% of the total cell population. In certain embodiments, nucleic acids extracted from these samples may be amplified using techniques well known in the art. The levels of selected markers detected would be compared with statistically valid groups of metastatic, non-metastatic malignant, benign, or normal colon tissue samples.

In one embodiment, the diagnostic method comprises determining whether a subject has an abnormal mRNA and/or protein level of the disclosed markers, such as by Northern blot analysis, reverse transcription-polymerase chain reaction (RT-PCR), in situ hybridization, immunoprecipitation, Western blot hybridization, or immunohistochemistry. According to the method, cells are obtained from a subject and the levels of the disclosed biomarkers, protein or mRNA level, is determined and compared to the level of these markers in a healthy subject. An abnormal level of the biomarker polypeptide or mRNA levels is likely to be indicative of cancer such as colon cancer.

Accordingly, in one aspect, the invention provides probes and primers that are specific to the unique nucleic acid markers disclosed herein. Accordingly, the nucleic acid probes comprise a region of a nucleic acid sequence of SEQ ID Nos 1 -8 or 9-16 sufficient to hybridize with a nucleic acid substantially complementary to the sequence of SEQ ID Nos 1-8 or 9-16. Preferred nucleic acid molecules for use as probes/primers can further comprise a region of nucleic acid sequence substantially complementary to the sequence of SEQ ID Nos. 1-8 or 9-16 sufficient to hybridize with the sequence of SEQ ID Nos. 1-8 or 9-16. In addition, nucleic acid sequences useful as probes/primers comprise a nucleotide sequence at least about 8 nucleotides in length, at least about 12 nucleotides in length, preferably at least about 15 nucleotides, more preferably about 25 nucleotides, and most preferably at least 40 nucleotides, and up to all or nearly all of the coding sequence which is complementary to a portion of the coding sequence of a marker nucleic acid sequence, which nucleic acid sequence is represented by SEQ ID Nos: 1-8 or 9-16, or a sequence complementary thereto.

In one embodiment, the method comprises using a nucleic acid probe to determine the presence of cancerous cells in a tissue from a patient comprising detecting the presence of a sequence comprising the sequence of one or more of SEQ ID Nos 1-8. Specifically, the method comprises:
1. providing a nucleic acid probe comprising a nucleotide sequence at least about 8 nucleotides in length, at least about 12 nucleotides in length, preferably at least about 15 nucleotides, more preferably about 25 nucleotides, and most preferably at least about 40 nucleotides, and up to all or nearly all of the coding sequence which is complementary to a portion of the coding sequence of a nucleic acid sequence represented by SEQ ID Nos: 1-8 or 9-16, or a sequence complementary thereto, and is differentially expressed in tumors cells, such as colon cancer cells;
2. obtaining a tissue sample from a patient potentially comprising cancerous cells;
3. providing a second tissue sample containing cells substantially all of which are non-cancerous;
4. contacting the nucleic acid probe under stringent conditions with RNA of each of said first and second tissue samples (e.g., in a Northern blot or in situ hybridization assay); and
5. comparing (a) the amount of hybridization of the probe with RNA of the first tissue sample, with (b) the amount of hybridization of the probe with RNA of the second tissue sample; wherein a statistically significant difference in the amount of hybridization with the RNA of the first tissue sample as compared to the amount of hybridization with the RNA of the second tissue sample is indicative of the presence of cancerous cells in the first tissue sample.

In one aspect, the method comprises *in situ* hybridization with a probe derived from a given marker nucleic acid sequence, which nucleic acid sequence is represented by SEQ ID Nos: 1-8 or 9-16, or a sequence complementary thereto. The method comprises contacting the labeled hybridization probe with a sample of a given type of tissue potentially containing cancerous or pre-cancerous cells as well as normal cells, and determining whether the probe labels some cells of the given tissue type to a degree significantly different (e.g., by at least 0.5 fold, at least 2 fold, at least 5 fold, at least 20 fold, or at least 50 fold) than the degree to which it labels other cells of the same tissue type.

Also within the invention is a method of determining the phenotype of a test cell from a given human tissue, e.g., whether the cell is (a) normal, or (b) cancerous or precancerous, by contacting the mRNA of a test cell with a nucleic acid probe at least approximately 8 nucleotides in length, preferably about 12, preferably about 15, preferably about 25, more preferably about 40 nucleotides in length, and up to all or nearly all of a sequence which is complementary to a portion of the coding sequence of a nucleic acid sequence represented by SEQ ID Nos: 9-16, or a sequence complementary thereto, and which is differentially expressed in tumor cells as compared to normal cells of the given tissue type; and determining the approximate amount of hybridization of the probe to the mRNA, an amount of hybridization either more or less than that seen with the mRNA of a normal cell of that tissue type being indicative that the test cell is cancerous or pre-cancerous.

Alternatively, the above diagnostic assays may be carried out using antibodies to detect the protein product encoded by the marker nucleic acid sequence, which nucleic acid sequence is represented by SEQ ID Nos: 1-8 or 9-16, or a sequence complementary thereto. Preferably, the protein product has the sequence of one or more of SEQ ID Nos. 17-24. Accordingly, in one embodiment, the assay would include contacting the proteins of the test cell with an antibody specific for the gene product of a nucleic acid represented by SEQ ID Nos: 9-16, or a sequence complementary thereto, the marker nucleic acid being one which is expressed at a given control level in normal cells of the same tissue type as the test cell, and determining the approximate amount of immunocomplex formation by the antibody and the proteins of the test cell, wherein a statistically significant difference in the amount of the immunocomplex formed with the proteins of a test cell as compared to a normal cell of the same tissue type is an indication that the test cell is cancerous or pre-cancerous.

Another such method includes the steps of: providing an antibody specific for the gene product of a marker nucleic acid sequence represented by SEQ ID Nos 9-16, or a sequence complementary thereto, the gene product being present in cancerous tissue of a given tissue type (e.g., colon tissue) at a level more or less than the level of the gene product in non-cancerous tissue of the same tissue type; obtaining from a patient a first sample of tissue of the given tissue type, which sample potentially includes cancerous cells; providing a second sample of tissue of the same tissue type (which may be from the same patient or from a normal control, e.g. another individual or cultured cells), this second sample containing normal cells and essentially no cancerous cells; contacting the antibody with protein (which may be partially purified, in lysed but unfractionated cells, or in situ) of the first and second samples under conditions permitting immunocomplex formation between the antibody and the marker nucleic acid sequence product present in the samples; and comparing (a) the amount of immunocomplex formation in the first sample, with (b) the amount of immunocomplex formation in the second sample, wherein a statistically significant difference in the amount of immunocomplex formation in the first sample less as compared to the amount of immunocomplex formation in the second sample is indicative of the presence of cancerous cells in the first sample of tissue.

The subject invention further provides a method of determining whether a cell sample obtained from a subject possesses an abnormal amount of marker polypeptide which comprises (a) obtaining a cell sample from the subject, (b) quantitatively determining the amount of the marker polypeptide in the sample so obtained, and (c) comparing the amount of the marker polypeptide so determined with a known standard, so as to thereby determine whether the cell sample obtained from the subject possesses an abnormal amount of the marker polypeptide. Such marker polypeptides may be detected by immunohistochemical assays, dot-blot assays, ELISA and the like.

Immunoassays are commonly used to quantitate the levels of proteins in cell samples, and many other immunoassay techniques are known in the art. The invention is not limited to a particular assay procedure, and therefore is intended to include both homogeneous and heterogeneous procedures. Exemplary immunoassays which can be conducted according to the invention include fluorescence polarization immunoassay (FPIA), fluorescence immunoassay (FIA), enzyme immunoassay (EIA), nephelometric inhibition immunoassay (NIA), enzyme linked immunosorbent assay (ELISA), and radioimmunoassay (RIA). An indicator moiety, or label group, can be attached to the subject antibodies and is selected so as to meet the needs of various uses of the method which are often dictated by the availability of assay equipment and compatible immunoassay procedures. General techniques to be used in performing the various immunoassays noted above are known to those of ordinary skill in the art.

In another embodiment, the level of the encoded product, i.e., the product encoded by SEQ ID Nos 9-16, or a sequence complementary thereto, in a biological fluid (e.g., blood or urine) of a patient may be determined as a way of monitoring the level of expression of the marker nucleic acid sequence in cells of that patient. Such a method would include the steps of obtaining a sample of a biological fluid from the patient, contacting the sample (or proteins from the sample) with an antibody specific for a encoded marker polypeptide, and determining the amount of immune complex formation by the antibody, with the amount of immune complex formation being indicative of the level of the marker encoded product in the sample. This determination is particularly instructive when compared to the amount of immune complex formation by the same antibody in a control sample taken from a normal individual or in one or more samples previously or subsequently obtained from the same person.

In another embodiment, the method can be used to determine the amount of marker polypeptide present in a cell, which in turn can be correlated with progression of a hyperproliferative disorder, e.g., colon cancer. The level of the marker polypeptide can be used predictively to evaluate whether a sample of cells contains cells which are, or are predisposed towards becoming, transformed cells. Moreover, the subject method can be used to assess the phenotype of cells which are known to be transformed, the phenotyping results being useful in planning a particular therapeutic regimen. For instance, very high levels of the marker polypeptide in sample cells is a powerful diagnostic and prognostic marker for a cancer, such as colon cancer. The observation of marker polypeptide level can be utilized in decisions regarding, e.g., the use of more aggressive therapies.

Where the methods of the invention comprise the detection and/or measurement of protein levels in a biological sample (i.e., levels of one or more of SEQ ID Nos 17-24), antibodies may be generated which will specifically bind to one or more of SEQ ID Nos 17-24, and which may be used according to numerous methods known to those of skill in the art for detecting protein expression, including but not limited to ELISA, immnoassay, ligand binding assay, lectin binding assays, affinity chromatography, and the like. Antibodies directed against the peptides of the invention, or antigenic or immunogenic epitopes thereof, can be, for example, polyclonal or monoclonal antibodies. Antibodies suitable for use in the assays of the present invention also include chimeric, single chain, and humanized antibodies, as well as Fab, F(ab')2, or Fv fragments, or the product of a phage display library, e.g., an Fab expression library. Various procedures known in the art may be used for the production of such antibodies and antibody fragments. Examples of phage display methods that can be used to make antibodies for use in the present invention include those disclosed in Brinkman et al., 1995, J. Immunol. Methods, 182:41-50; Ames et al., 1995, J. Immunol. Methods, 184:177-186; Kettleborough et al., 1994, Eur. J. Immunol., 24:952-958; Persic et al., 1997, Gene, 187:9-18; Burton et al., 1994, Advances in Immunology, 57:191-280; and in U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

Antibodies generated against one or more of the peptides of SEQ ID Nos 17-24 can be obtained by direct injection of an immunogenic polypeptide preparation comprising one or more of SEQ ID Nos 17-24 into an animal, or by administering all, or a portion, of the polypeptide to an animal, preferably a nonhuman animal. For the preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunol. Today, 4:72), and the EBV-hybridoma technique to produce human monoclonal antibodies (Cole et al., 1985. In: Monoclonal Antibodies and Cancer Therapy, Alan R. Liss, Inc., pp. 77-96). Techniques described for the production of single chain antibodies (U.S. Patent No. 4,946,778) can be adapted to produce single chain antibodies to the uPA, PAI-1, and/or uPA:PAI-1 PA system analytes. Also, transgenic mice may be used to express humanized antibodies to the polypeptides of SEQ ID Nos 17-24.

As set out herein, one aspect of the present invention relates to diagnostic assays for determining, in the context of a biolgical sample isolated from a patient, if the level of a marker polypeptide (e.g., SEQ ID Nos 17-24) is reduced in the sample cells. The term "reduced" refers to a state in which the level of expression of a polypeptide of the invention is decreased by at least 10%, preferably decreased by at least 20%, 40%, 60%, and up to 100% as measured in a biological sample obtained from a patient at a second time point compared to a similar (e.g., same tissue or cell type) biological sample obtained from the same patient at first time point. The first and second time points may reflect pre- and post-therapeutic treatment, or alternatively, may be successive time points during a therapeutic treatment regime. In particular, the assay evaluates the level of marker polypeptide in the test cells, and, preferably, compares the measured level with marker polypeptide detected in at least one control cell, e.g., a normal cell and/or a transformed cell of known phenotype.

Of particular importance to the subject invention is the ability to quantitate the level of marker polypeptide as determined by the number of cells associated with a normal or abnormal marker polypeptide level. The number of cells with a particular marker polypeptide phenotype may then be correlated with patient prognosis. In one embodiment of the invention, the marker polypeptide phenotype of the lesion is determined as a percentage of cells in a biopsy which are found to have abnormally high/low levels of the marker polypeptide. Such expression may be detected by immunohistochemical assays, dot-blot assays, ELISA and the like.

Where tissue samples are employed, immunohistochemical staining may be used to determine the number of cells having the marker polypeptide phenotype. For such staining, a multiblock of tissue is taken from the biopsy or other tissue sample and subjected to proteolytic hydrolysis, employing such agents as protease K or pepsin. In certain embodiments, it may be desirable to isolate a nuclear fraction from the sample cells and detect the level of the marker polypeptide in the nuclear fraction.

The tissue samples are fixed by treatment with a reagent such as formalin, glutaraldehyde, methanol, or the like. The samples are then incubated with an antibody, preferably a monoclonal antibody, with binding specificity for the marker polypeptides. This antibody may be conjugated to a label for subsequent detection of binding. Samples are incubated for a time sufficient for formation of the immunocomplexes. Binding of the antibody is then detected by virtue of a label conjugated to this antibody. Where the antibody is unlabeled, a second labeled antibody may be employed, e.g., which is specific for the isotype of the anti-marker polypeptide antibody. Examples of labels which may be employed include radionuclides, fluorescers, chemiluniinescers, enzymes and the like.

Where enzymes are employed, the substrate for the enzyme may be added to the samples to provide a colored or fluorescent product. Examples of suitable enzymes for use in conjugates include horseradish peroxidase, alkaline phosphatase, malate dehydrogenase and the like. Where not commercially available, such antibody-enzyme conjugates are readily produced by techniques known to those skilled in the art.

In one embodiment, the assay is performed as a dot blot assay. The dot blot assay finds particular application where tissue samples are employed as it allows determination of the average amount of the marker polypeptide associated with a single cell by correlating the amount of marker polypeptide in a cell- free extract produced from a predetermined number of cells.

It is well established in the cancer literature that tumor cells of the same type (e.g., breast and/or colon tumor cells) may not show uniformly increased expression of individual oncogenes or uniformly decreased expression of individual tumor suppressor genes. There may also be varying levels of expression of a given marker gene even between cells of a given type of cancer, further emphasizing the need for reliance on a battery of tests rather than a single test. Accordingly, in one aspect, the invention provides for a battery of tests utilizing a number of probes of the invention, in order to improve the reliability and/or accuracy of the diagnostic test.

In one embodiment, the present invention also provides a method wherein nucleic acid probes are immobilized on a DNA chip in an organized array. Oligonucleotides can be bound to a solid support by a variety of processes, including lithography. For example a chip can hold up to 250,000 oligonucleotides (GeneChip, Affymetrix). These nucleic acid probes comprise a nucleotide sequence at least about 8 nucleotides in length, preferably at least about 12 preferably at least about 15 nucleotides, more preferably at least about 25 nucleotides, and most preferably at least about 40 nucleotides, and up to all or nearly all of a sequence which is complementary to a portion of the coding sequence of a marker nucleic acid sequence represented by SEQ ID Nos: 9-16 and is differentially expressed in tumor cells, such as colon cancer cells. The present invention provides significant advantages over the available tests for various cancers, such as colon cancer, because it increases the reliability of the test by providing an array of nucleic acid markers on a single chip.

The method includes obtaining a biopsy, which is optionally fractionated by cryostat sectioning to enrich tumor cells to about 80% of the total cell population. The DNA or RNA is then extracted, amplified, and analyzed with a DNA chip to determine the presence of absence of the marker nucleic acid sequences.

In one embodiment, the nucleic acid probes are spotted onto a substrate in a two-dimensional matrix or array. Samples of nucleic acids can be labeled and then hybridized to the probes. Double-stranded nucleic acids, comprising the labeled sample nucleic acids bound to probe nucleic acids, can be detected once the unbound portion of the sample is washed away.

The probe nucleic acids can be spotted on substrates including glass, nitrocellulose, etc. The probes can be bound to the substrate by either covalent bonds or by non-specific interactions, such as hydrophobic interactions. The sample nucleic acids can be labeled using radioactive labels, fluorophores, chromophores, etc.

Techniques for constructing arrays and methods of using these arrays are described in EP No. 0 799 897; PCT No. WO 97/292 12; PCT No. WO 97127317; EP No. 0 785 280; PCT No. WO 97/02357; U.S. Pat. No. 5,593,839; U.S. Pat. No. 5,578,832; EP No. 0 728 520; U.S. Pat. No. 5,599,695; EP No. 0 721 016; U.S. Pat. No. 5,556,752; PCT No. WO 95/22058; and U.S. Pat. No. 5,631,734.

Further, arrays can be used to examine differential expression of genes and can be used to determine gene function. For example, arrays of the instant nucleic acid sequences can be used to determine if any of the nucleic acid sequences are differentially expressed between normal cells and cancer cells, for example. High expression of a particular message in a cancer cell, which is not observed in a corresponding normal cell, can indicate a cancer specific protein.

In yet another embodiment, the invention contemplates using a panel of antibodies which are generated against the marker polypeptides of this invention, which polypeptides are SEQ ID Nos: 17-24. Such a panel of antibodies may be used as a reliable diagnostic probe for colon cancer. The assay of the present invention comprises contacting a biopsy sample containing cells, e.g., colon cells, with a panel of antibodies to one or more of the encoded products to determine the presence or absence of the marker polypeptides.

The diagnostic methods of the subject invention may also be employed as follow-up to treatment, e.g., quantitation of the level of marker polypeptides may be indicative of the effectiveness of current or previously employed cancer therapies as well as the effect of these therapies upon patient prognosis.

Accordingly, the present invention makes available diagnostic assays and reagents for detecting gain and/or loss of marker polypeptides from a cell in order to aid in the diagnosis and phenotyping of proliferative disorders arising from, for example, tumorigenic transformation of cells.

The diagnostic assays described above can be adapted to be used as prognostic assays, as well. Such an application takes advantage of the sensitivity of the assays of the invention to events which take place at characteristic stages in the progression of a tumor. For example, a given marker gene may be up- or downregulated at a very early stage, perhaps before the cell is irreversibly committed to developing into a malignancy, while another marker gene may be characteristically up or down regulated only at a much later stage. Such a method could involve the steps of contacting the mRNA of a test cell with a nucleic acid probe derived from a given marker nucleic acid which is expressed at different characteristic levels in cancerous or precancerous cells at different stages of tumor progression, and determining the approximate amount of hybridization of the probe to the mRNA of the cell, such amount being an indication of the level of expression of the gene in the cell, and thus an indication of the stage of tumor progression of the cell; alternatively, the assay can be carried out with an antibody specific for the gene product of the given marker nucleic acid, contacted with the proteins of the test cell. A battery of such tests will disclose not only the existence and location of a tumor, but also will allow the clinician to select the mode of treatment most appropriate for the tumor, and to predict the likelihood of success of that treatment.

The methods of the invention can also be used to follow the clinical course of a tumor. For example, the assay of the invention can be applied to a tissue sample from a patient; following treatment of the patient for the cancer, another tissue sample is taken and the test repeated. Successful treatment will result in either removal of all cells which demonstrate differential expression characteristic of the cancerous or precancerous cells, or a substantial increase in expression of the gene in those cells, perhaps approaching or even surpassing normal levels.

In yet another embodiment, the invention provides methods for determining whether a subject is at risk for developing a disease, such as a predisposition to develop cancer, for example colon cancer, associated with an aberrant activity of any one of the polypeptides of SEQ ID Nos: 17-24, wherein the aberrant activity of the polypeptide is characterized by detecting the presence or absence of a genetic lesion characterized by at least one of (i) an alteration affecting the integrity of a gene encoding a marker polypeptides, or (ii) the mis-expression of the encoding nucleic acid. To illustrate, such genetic lesions can be detected by ascertaining the existence of at least one of(i) a deletion of one or more nucleotides from the nucleic acid sequence, (ii) an addition of one or more nucleotides to the nucleic acid sequence, (iii) a substitution of one or more nucleotides of the nucleic acid sequence, (iv) a gross chromosomal rearrangement of the nucleic acid sequence, (v) a gross alteration in the level of a messenger RNA transcript of the nucleic acid sequence, (vii) aberrant modification of the nucleic acid sequence, such as of the methylation pattern of the genomic DNA, (vii) the presence of a non-wild type splicing pattern of a messenger RNA transcript of the gene, (viii) a non-wild type level of the marker polypeptide, (ix) allelic loss of the gene, and/or (x) inappropriate post-translational modification of the marker polypeptide.

The present invention provides assay techniques for detecting lesions in the encoding nucleic acid sequence. These methods include, but are not limited to, methods involving sequence analysis, Southern blot hybridization, restriction enzyme site mapping, and methods involving detection of absence of nucleotide pairing between the nucleic acid to be analyzed and a probe.

Specific diseases or disorders, e.g., genetic diseases or disorders, are associated with specific allelic variants of polymorphic regions of certain genes, which do not necessarily encode a mutated protein. Thus, the presence of a specific allelic variant of a polymorphic region of a gene in a subject can render the subject susceptible to developing a specific disease or disorder. Polymorphic regions in genes, can be identified, by determining the nucleotide sequence of genes in populations of individuals. If a polymorphic region is identified, then the link with a specific disease can be determined by studying specific populations of individuals, e.g, individuals which developed a specific disease, such as colon cancer. A polymorphic region can be located in any region of a gene, e.g., exons, in coding or non coding regions of exons, introns, and promoter region.

In an exemplary embodiment, there is provided a nucleic acid composition comprising a nucleic acid probe including a region of nucleotide sequence which is capable of hybridizing to a sense or antisense sequence of a gene or naturally occurring mutants thereof, or 5' or 3' flanking sequences or intronic sequences naturally associated with the subject genes or naturally occurring mutants thereof. The nucleic acid of a cell is rendered accessible for hybridization, the probe is contacted with the nucleic acid of the sample, and the hybridization of the probe to the sample nucleic acid is detected. Such techniques can be used to detect lesions or allelic variants at either the genomic or mRNA level, including deletions, substitutions, etc., as well as to determine mRNA transcript levels.

A preferred detection method is allele specific hybridization using probes overlapping the mutation or polymorphic site and having about 5, 10, 20, 25, or 30 nucleotides around the mutation or polymorphic region. In a preferred embodiment of the invention, several probes capable of hybridizing specifically to allelic variants are attached to a solid phase support, e.g., a "chip". Mutation detection analysis using these chips comprising oligonucleotides, also termed "DNA probe arrays" is described e.g., in Cronin et al. (1996) Human Mutation 7:244. In one embodiment, a chip comprises all the allelic variants of at least one polymorphic region of a gene. The solid phase support is then contacted with a test nucleic acid and hybridization to the specific probes is detected. Accordingly, the identity of numerous allelic variants of one or more genes can be identified in a simple hybridization experiment.

In certain embodiments, detection of the lesion comprises utilizing the probe/primer in a polymerase chain reaction (PCR) (see, e.g. U.S. Patent Nos. 4,683,195 and 4,683,202), such as anchor PCR or RACE PCR, or, alternatively, in a ligase chain reaction (LCR) (see, e.g., Landegran *et al*. (1988) *Science* 241:1077-1080; and *Nakazawa et al*. (1994) *PNAS* 91:360-364), the latter of which can be particularly useful for detecting point mutations in the gene (sec Abravaya *et al*. (1995) *Nuc Acid Res* 23:675-682). In a merely illustrative embodiment, the method includes the steps of (i) collecting a sample of cells from a patient, (ii) isolating nucleic acid (e.g., genomic, mRNA or both) from the cells of the sample, (iii) contacting the nucleic acid sample with one or more primers which specifically hybridize to a nucleic acid sequence under conditions such that hybridization and amplification of the nucleic acid (if present) occurs, and (iv) detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli, J.C. *et al*., 1990, Proc. Natl. Acad. Sci. USA 87:1874 -1878), transcriptional amplification system (Kwoh, D.Y. et al., 1989, Proc. NatI. Acad. Sci. USA 86:1173-1177), Q-Beta Replicase (Lizardi, *P.M. et al*., 1988, Bio/Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In a preferred embodiment of the subject assay, mutations in, or allelic variants, of a gene from a sample cell are identified by alterations in restriction enzyme cleavage patterns. For example, sample and control DNA is isolated, amplified (optionally), digested with one or more restriction endonucleases, and fragment length sizes are determined by gel electrophoresis. Moreover, the use of sequence specific ribozymes (see, for example, U.S. Patent No. 5,498,531) can be used to score for the presence of specific mutations by development or loss of a ribozyme cleavage site.

As set out herein, one aspect of the present invention relates to diagnostic assays for determining, in the context of a biolgical sample isolated from a patient, if the expression level of a nucleic acid sequence of the invention (e.g., a sequence comprising one or more of SEQ ID Nos 1-8 and/or one or more of SEQ ID Nos 9-16) is reduced in the sample cells. The term "reduced" refers to a state in which the expression level of a differentially expressed polynucleotoide sequence of the invention is decreased by at least 10%, preferably decreased by at least 20%, 40%, 60%, and up to 100% as measured in a biological sample obtained from a patient at a second time point compared to a similar (e.g., same tissue or cell type) biological sample obtained from the same patient at first time point. The first and second time points may reflect pre- and post-therapeutic treatment, or alternatively, may be successive time points during a therapeutic treatment regime. In particular, the assay evaluates the level of marker polypeptide in the test cells, and, preferably, compares the measured level with marker polypeptide detected in at least one control cell, e.g., a normal cell and/or a transformed cell of known phenotype.

According to the present invention, a method is provided that permits the assessment and/or monitoring of patients who will be likely to benefit from both traditional and non-traditional treatments and therapies for colon cancers. The present invention thus embraces testing, screening and monitoring of patients undergoing anti-cancer treatments and therapies, used alone, in combination with each other, and/or in combination with anti-cancer drugs, anti-neoplastic agents, chemotherapeutics and/or radiation and/or surgery, to treat cancer patients.

An advantage of the present invention is the ability to monitor, or screen over time, those patients who can benefit from one, or several, of the available cancer therapies, and preferably, to monitor patients receiving a particular type of therapy, or a combination therapy, over time to determine how the patient is faring from the treatment(s), if a change, alteration, or cessation of treatment is warranted; if the patient's disease has been reduced, ameliorated, or lessened; or if the patient's disease state or stage has progressed, or become metastatic or invasive. The cancer treatments embraced herein also include surgeries to remove or reduce in size a tumor, or tumor burden, in a patient. Accordingly, the methods of the invention are useful to monitor patient progress and disease status post-surgery.

The identification of the correct patients for a cancer therapy according to this invention can provide an increase in the efficacy of the treatment and can avoid subjecting a patient to unwanted and life-threatening side effects of the therapy. By the same token, the ability to monitor a patient undergoing a course of therapy using the methods of the present invention can determine whether a patient is adequately responding to therapy over time, to determine if dosage or amount or mode of delivery should be altered or adjusted, and to ascertain if a patient is improving during therapy, or is regressing or is entering a more severe or advanced stage of disease, including invasion or metastasis, as discussed further herein.

A method of monitoring according to this invention reflects the serial, or sequential, testing or analysis of a cancer patient by testing or analyzing the patient's body fluid sample over a period of time, such as during the course of treatment or therapy, or during the course of the patient's disease. For instance, in serial testing, the same patient provides a body fluid sample, e.g., serum or plasma, or has sample taken, for the purpose of observing, checking, or examining the levels of one or more of the biomarkers of the invention in the patient by measuring the levels of one or more of these biomarkers during the course of treatment, and/or during the course of the disease, according to the methods of the invention.

Similarly, a patient can be screened over time to assess the levels of one or more of the biomarkers in a body fluid sample for the purposes of determining the status of his or her disease and/or the efficacy, reaction, and response to cancer or neoplastic disease treatments or therapies that he or she is undergoing. It will be appreciated that one or more pretreatment sample(s) is/are optimally taken from a patient prior to a course of treatment or therapy, or at the start of the treatment or therapy, to assist in the analysis and evaluation of patient progress and/or response at one or more later points in time during the period that the patient is receiving treatment and undergoing clinical and medical evaluation.

In monitoring a patient's levels of one or more of the biomarkers of the invention over a period of time, which may be days, weeks, months, and in some cases, years, or various intervals thereof, the patient's body fluid sample, e.g., a serum or plasma sample, is collected at intervals, as determined by the practitioner, such as a physician or clinician, to determine the levels of one or more of the biomarkers in the cancer patient compared to the respective levels of one or more of these analytes in normal individuals over the course or treatment or disease. For example, patient samples can be taken and monitored every month, every two months, or combinations of one, two, or three month intervals according to the invention. Quarterly, or more frequent monitoring of patient samples, is advisable.

The levels of the one or more biomarkers found in the patient are compared with the respective levels of the one or more of these biomarkers in normal individuals, and with the patient's own biomarker levels, for example, obtained from prior testing periods, to determine treatment or disease progress or outcome. Accordingly, use of the patient's own biomarker levels monitored over time can provide, for comparison purposes, the patient's own values as an internal personal control for long-term monitoring of biomarker levels, and thus cancer presence and/or progression. As described herein, following a course of treatment or disease, the determination of an increase in one or more of the biomarker levels in the cancer patient over time compared to the respective levels of one or more of these biomarkers in normal individuals reflects the ability to determine the severity or stage of a patient's cancer, or the progress, or lack thereof, in the course or outcome of a patient's cancer therapy or treatment.

Elevations or increases in the levels of the biomarkers in cancer patients is determined by comparing the values obtained from analyzing cancer patient samples compared to the normal control range expression levels, wherein a biomarker is said to be overexpressed if expression of the biomarker is at least 1.5 fold greater in the cancer patient relative to a normal control.

In monitoring a patient over time, a reduction in the levels of one or more of a patient's biomarker levels from increased levels (i.e., at least 1.5 fold overexpressed) compared to normal range values to levels at or near to the levels of the analytes found in normal individuals is indicative of treatment progress or efficacy, and/or disease improvement, remission, tumor reduction or elimination, and the like. Likewise, in all of the methods described in the embodiments of this invention, a determination of a reduction of one or more of a patient's biomarker levels from an elevated level (i.e., at least 1.5 fold overexpressed) to, or approximately to, the respective levels of one or more of these analytes found in normal individuals provides a further aspect of the methods of the invention, in which a patient's improvement, recovery or remission, and/or treatment progress or efficacy, is able to be ascertained over time following performance of the method.

Another embodiment of the present invention encompasses a method of monitoring a cancer patient's course of disease, or the efficacy of a cancer patient's treatment or therapy. The patient's treatment or therapy can involve traditional therapies, such as hormone therapy, chemotherapeutic drug therapy, radiation, or novel therapies, or a combination of any of the foregoing. The method involves measuring levels of one or more biomarkers in a body fluid sample of the cancer patient and determining if the levels of one or more of the biomarkers in the patient's sample are increased by at least 1.5 fold compared to the respective levels of one or more of these analytes in normal controls during the course of disease or cancer treatment. In accordance with the method, an increase in the levels of the biomarker in the cancer patient compared to the respective levels of the biomarker in normal controls is indicative of an increase in stage, grade, severity or progression of the patient's cancer and/or a lack of efficacy or benefit of the cancer treatment or therapy provided to the patient during a course of treatment, e.g., poor treatment or clinical outcome.

As will be understood by the skilled practitioner in the art, the monitoring method according to this invention is preferably, performed in a serial or sequential fashion, using samples taken from a patient during the course of disease, or a disease treatment regimen, (e.g., after a number of days, weeks, months, or occasionally, years, or various multiples of these intervals) to allow a determination of disease progression or outcome, and/or treatment efficacy or outcome. If the sample is amenable to freezing or cold storage, the samples may be taken from a patient (or normal individual) and stored for a period of time prior to analysis.

In another of its embodiments, the present invention encompasses the determination of the amounts or levels of one or more additional cancer markers in conjunction with the determination of the levels of one or more of the biomarkers of the invention in a sample to be analyzed.

In another of its aspects, the present invention encompasses a method of monitoring disease severity or progression of a cancer patient. The method comprises measuring levels of one or more biomarkers in a biological sample, e.g.,serum or plasma sample, of the cancer patient and determining if the cancer patient has increased (i.e., at least a 1.5 fold increase) serum or plasma levels of one or more of the biomarkers compared to the respective biomarker level values in normal individuals. In the method, cancer severity or progression is monitored in the patient based upon elevated or increased levels in the patient's sample compared to the respective normal biomarker levels in normal individuals. According to this method, the most severe cancer stage correlates with the most elevated levels (e.g., greater than 1.5 fold) of plasma or serum biomarkers compared to the respective normal control levels of these biomarkers.

In yet another of its embodiments, the present invention provides a method of monitoring cancer treatment, or efficacy thereof, in a cancer patient undergoing such treatment. The method involves measuring the serum or plasma levels of one or more biomarkers in a cancer patient and determining if the levels of one or more of these biomarkers in the patient increases during the cancer treatment compared to the respective levels of one or more of the biomarkers determined in the serum or plasma samples of normal controls, where an elevation or increase in one or more of the biomarker levels in the cancer patient compared to the respective levels of one or more of these biomarkers in normal control plasma or serum during the monitoring period indicates one or more of the following: (i) cancer progression; (ii) a more severe stage of the cancer; (iii) lack of response by the patient to the cancer treatment; or (iv) poor outcome or shorter survival time.

In addition, if, during the course of monitoring the levels of one or more of the biomarkers in the patient undergoing treatment, a change is found in the levels of one or more of these biomarkers being measured, such that a reduction in the levels of one or more of the biomarkers is determined, an assessment can be made as to one or more of the following events: (i) the patient is progressing well on the treatment, (ii) the treatment is effective; (iii) the patient is responding to the treatment; and/or (iv) the patient's cancer is not progressing or has been ameliorated or eliminated by the treatment.

In accordance with the present invention, such a method of monitoring and assessment of one or more of the biomarker levels during a patient's course of treatment or therapy, compared with the respective normal level values of the biomarker, can provide the physician or clinician with a determination of a patient's progress, or regression, as the case may be, as a consequence of a particular cancer or anti-neoplastic disease treatment or therapy. Such a determination advantageously allows tailoring of the treatment or therapy to better or more aggressively attack (or treat) a cancer; it also allows altering dosage, mode of administration, modifying the regimen, or combining therapies to achieve a more effective overall treatment and outcome for the individual patient.

It is to be understood that in all of the embodiments describing the methods according to the present invention, the monitoring of a cancer patient for disease progression or outcome, or for cancer treatment or therapy efficacy or outcome, can include the analysis of a pretreatment sample taken from the patient at a first time point, and can also include the analysis of a patient's samples taken at a second, third, fourth, or subsequent time, during the course of disease or during a cancer or anti-neoplastic treatment or therapy regimen, or a combination of treatment or therapy regimens.

Another aspect of the invention is directed to the identification of agents capable of modulating the differentiation and proliferation of cells characterized by aberrant proliferation. In this regard, the invention provides assays for determining compounds that modulate the expression of the marker nucleic acids (SEQ ID Nos: 1-8 or 9-16, or a sequence complementary thereto) and/or alter for example, inhibit the bioactivity of the polypeptide encoded by, for example, SEQ ID Nos 9-16.

Several in vivo methods can be used to identify compounds that modulate expression of the marker nucleic acids (SEQ ID Nos: 1-8 or 9-16, or a sequence complementary thereto) and/or alter for example, inhibit the bioactivity of the polypeptide encoded by SEQ ID Nos 9-16.

Drug screening is performed by adding a test compound to a sample of cells, and monitoring the effect. A parallel sample which does not receive the test compound is also monitored as a control. The treated and untreated cells are then compared by any suitable phenotypic criteria, including but not limited to microscopic analysis, viability testing, ability to replicate, histological examination, the level of a particular RNA or polypeptide associated with the cells, the level of enzymatic activity expressed by the cells or cell lysates, and the ability of the cells to interact with other cells or compounds. Differences between treated and untreated cells indicates effects attributable to the test compound.

Desirable effects of a test compound include an effect on any phenotype that was conferred by the cancer-associated marker nucleic acid sequence. Examples include a test compound that limits the overabundance of mRNA, limits production of the encoded protein, or limits the functional effect of the protein. The effect of the test compound would be apparent when comparing results between treated and untreated cells.

The invention thus also encompasses methods of screening for agents which inhibit expression of the nucleic acid markers (SEQ ID Nos: 1-8 or 9-16, preferably SEQ ID Nos 9-16 or a sequence complementary thereto) in vitro, comprising exposing a cell or tissue in which the marker nucleic acid mRNA is detectable in cultured cells to an agent in order to determine whether the agent is capable of inhibiting production of the mRNA; and determining the level of mRNA in the exposed cells or tissue, wherein a decrease in the level of the mRNA after exposure of the cell line to the agent is indicative of inhibition of the marker nucleic acid mRNA production.

Alternatively, the screening method may include in vitro screening of a cell or tissue in which marker protein is detectable in cultured cells to an agent suspected of inhibiting production of the marker protein; and determining the level of the marker protein in the cells or tissue, wherein a decrease in the level of marker protein after exposure of the cells or tissue to the agent is indicative of inhibition of marker protein production.

The invention also encompasses in vivo methods of screening for agents which inhibit expression of the marker nucleic acids, comprising exposing a mammal having tumor cells in which marker mRNA or protein is detectable to an agent suspected of inhibiting production of marker mRNA or protein; and determining the level of marker mRNA or protein in tumor cells of the exposed mammal. A decrease in the level of marker mRNA or protein after exposure of the mammal to the agent is indicative of inhibition of marker nucleic acid expression.

Accordingly, the invention provides a method comprising incubating a cell expressing the marker nucleic acids (SEQ ID Nos: 1-8 or 9-16, preferably SEQ ID Nos 9-16, or a sequence complementary thereto) with a test compound and measuring the mRNA or protein level. The invention further provides a method for quantitatively determining the level of expression of the marker nucleic acids in a cell population, and a method for determining whether an agent is capable of increasing or decreasing the level of expression of the marker nucleic acids in a cell population. The method for determining whether an agent is capable of increasing or decreasing the level of expression of the marker nucleic acids in a cell population comprises the steps of(a) preparing cell extracts from control and agent-treated cell populations, (b) isolating the marker polypeptides from the cell extracts, (c) quantifying (e.g., in parallel) the amount of an immunocomplex formed between the marker polypeptide and an antibody specific to said polypeptide. The marker polypeptides of this invention may also be quantified by assaying for its bioactivity. Agents that induce increased the marker nucleic acid expression may be identified by their ability to increase the amount of immunocomplex formed in the treated cell as compared with the amount of the immunocomplex formed in the control cell. In a similar manner, agents that decrease expression of the marker nucleic acid may be identified by their ability to decrease the amount of the immunocomplex formed in the treated cell extract as compared to the control cell.

mRNA levels can be determined by Northern blot hybridization. mRNA levels can also be determined by methods involving PCR. Other sensitive methods for measuring mRNA, which can be used in high throughput assays, e.g., a method using a DELFIA endpoint detection and quantification method, are described, e.g., in Webb and Hurskainen (1996) *Journal of Biomolecular Screening* 1:119. Marker protein levels can be determined by immunoprecipitations or immunohistochemistiy using an antibody that specifically recognizes the protein product of SEQ ID Nos: 17-24.

Agents that are identified as active in the drug screening assay are candidates to be tested for their capacity to block cell proliferation activity. These agents would be useful for treating a disorder involving aberrant growth of cells, especially colon cells.

A variety of assay formats will suffice and, in light of the present disclosure, those not expressly described herein will nevertheless be comprehended by one of ordinary skill in the art. For instance, the assay can be generated in many different formats, and include assays based on cell-free systems, e.g., purified proteins or cell lysates, as well as cell-based assays which utilize intact cells.

In many drug screening programs which test libraries of compounds and natural extracts, high throughput assays are desirable in order to maximize the number of compounds surveyed in a given period of time. Assays of the present invention which are performed in cell-free systems, such as may be derived with purified or semi-purified proteins or with lysates, are often preferred as "primary" screens in that they can be generated to permit rapid development and relatively easy detection of an alteration in a molecular target which is mediated by a test compound. Moreover, the effects of cellular toxicity and/or bioavailability of the test compound can be generally ignored in the *in vitro* system, the assay instead being focused primarily on the effect of the drug on the molecular target as may be manifest in an alteration of binding affinity with other proteins or changes in enzymatic properties of the molecular target.

### A. Use of Nucleic Acids as Probes in Mapping and in Tissue Profiling Probes

Polynucleotide probes as described above, e g , comprising at least 8 contiguous nucleotides selected from the nucleotide SEQ ID Nos. 1-8 or 9-16, or a sequence complementary thereto, are used for a variety of purposes, including identification of human chromosomes and determining transcription levels. Additional disclosure about preferred regions of the nucleic acid sequences is found in the accompanying tables.

The nucleotide probes are labeled, for example, with a radioactive, fluorescent, biotinylated, or chemiluminescent label, and detected by well known methods appropriate for the particular label selected. Protocols for hybridizing nucleotide probes to preparations of metaphase chromosomes are also well known in the art. A nucleotide probe will hybridize specifically to nucleotide sequences in the chromosome preparations which are complementary to the nucleotide sequence of the probe. A probe that hybridizes specifically to a nucleic acid should provide a detection signal at least 5-, 10-, or 20-fold higher than the background hybridization provided with other unrelated sequences.

In a non-limiting example, commercial programs are available for identifying regions of chromosomes commonly associated with disease, such as cancer. Nucleic acids of the invention can be used to probe these regions. For example, if, through profile searching, a nucleic acid is identified as corresponding to a gene encoding a kinase, its ability to bind to a cancer-related chromosomal region will suggest its role as a kinase in one or more stages of tumor cell development/growth. Although some experimentation would be required to elucidate the role, the nucleic acid constitutes a new material for isolating a specific protein that has potential for developing a cancer diagnostic or therapeutic.

Nucleotide probes are used to detect expression of a gene corresponding to the nucleic acid. For example, in Northern blots, mRNA is separated electrophoretically and contacted with a probe. A probe is detected as hybridizing to an mRNA species of a particular size. The amount of hybridization is quantitated to determine relative amounts of expression, for example under a particular condition. Probes are also used to detect products of amplification by polymerase chain reaction. The products of the reaction are hybridized to the probe and hybrids are detected. Probes are used for in situ hybridization to cells to detect expression. Probes can also be used in vivo for diagnostic detection of hybridizing sequences. Probes are typically labeled with a radioactive isotope. Other types of detectable labels may be used such as chromophores, fluorophores, and enzymes.

Expression of specific mRNA can vary in different cell types and can be tissue specific. This variation of mRNA levels in different cell types can be exploited with nucleic acid probe assays to determine tissue types. For example, PCR, branched DNA probe assays, or blotting techniques utilizing nucleic acid probes substantially identical or complementary to nucleic acids of SEQ ID Nos. 1-8 or 9-16, or a sequence complementary thereto, can determine the presence or absence of target cDNA or mRNA.

Examples of a nucleotide hybridization assay are described in Urdea *et al*., PCT W092/02526 and *Urdea et al*., U.S. Patent No. 5,124,246, both incorporated herein by reference. The references describe an example of a sandwich nucleotide hybridization assay.

Alternatively, the Polymerase Chain Reaction (PCR) is another means for detecting small amounts of target nucleic acids, as described in Mullis *et al*., *Met*/*i*. *Enzymol*. (1987) *155:*335-350; U.S. Patent No. 4,683,195; and U.S. Patent No. 4,683,202, all incorporated herein by reference. Two primer polynucleotides nucleotides hybridize with the target nucleic acids and are used to prime the reaction. The primers may be composed of sequence within or 3' and *5*' to the polynucleotides of the Sequence Listing. Alternatively, if the primers are 3' and 5' to these polynucleotides, they need not hybridize to them or the complements. A thermostable polymerase creates copies of target nucleic acids from the primers using the original target nucleic acids as a template. After a large amount of target nucleic acids is generated by the polymerase, it is detected by methods such as Southern blots. When using the Southern blot method, the labeled probe will hybridize to a polynucleotide of the Sequence Listing or complement.

Furthermore, mRNA or cDNA can be detected by traditional blotting techniques described in Sambrook *et al*., "Molecular Cloning: A Laboratory Manual" (New York, Cold Spring Harbor Laboratory, 1989). mRNA or cDNA generated from mRNA using a polymerase enzyme can be purified and separated using gel electrophoresis. The nucleic acids on the gel are then blotted onto a solid support, such as nitrocellulose. The solid support is exposed to a labeled probe and then washed to remove any unhybridized probe. Next, the duplexes containing the labeled probe are detected. Typically, the probe is labeled with radioactivity.

### Mapping

Nucleic acids of the present invention are used to identify a chromosome on which the corresponding gene resides. For example, using *in situ* hybridization (ISH), or fluorescence in situ hybridization (FISH) on normal metaphase spreads, comparative genomic hybridization allows total genome assessment of changes in relative copy number of DNA sequences. See Schwartz and Samad, *Current* *Opinions in Biotechnology* (1994) 8:70-74; Kallioniemi *et al*., *Seminars in Cancer Biology* (1993) 4:41-46; Valdes and Tagle, *Methods in Molecular Biology* (1997) 68:1, Boultwood, ed., Human Press, Totowa, NJ.

Preparations of human metaphase chromosomes are prepared using standard cytogenetic techniques from human primary tissues or cell lines. Nucleotide probes comprising at least 12 contiguous nucleotides selected from the nucleotide sequence of SEQ ID Nos. 1-8 or 9-16, or a sequence complementary thereto, are used to identify the corresponding chromosome. The nucleotide probes are labeled, for example, with a radioactive, fluorescent, biotinylated, or chemiluminescent label, and detected by well known methods appropriate for the particular label selected. Protocols for hybridizing nucleotide probes to preparations of metaphase chromosomes are also well known in the art. A nucleotide probe will hybridize specifically to nucleotide sequences in the chromosome preparations that are complementary to the nucleotide sequence of the probe. A probe that hybridizes specifically to a target gene provides a detection signal at least 5-, 10-, or 20-fold higher than the background hybridization provided with unrelated coding sequences.

Nucleic acids are mapped to particular chromosomes using, for example, radiation hybrids or chromosome-specific hybrid panels. See Leach *et al*., *Advances in Genetics, (1995) 33:63-99;* Walter *et al*., *Nature Genetics* (1994) 7:22-28; Walter and Goodfellow, *Trends in Genetics* (1992) *9:352.* Panels for radiation hybrid mapping are available from Research Genetics, Inc., Huntsville, Alabama, USA. Databases for markers using various panels are available via the world wide web at http:/F/shgc-www.stanford.edu, and other locations. The statistical program RHMAP can be used to construct a map based on the data from radiation hybridization with a measure of the relative likelihood of one order versus another, RHMAP is available via the world wide web at http://www.sph.umich.edu/group/statgen/software.

Such mapping can be useful in identifying the function of the target gene by its proximity to other genes with known function. Function can also be assigned to the target gene when particular syndromes or diseases map to the same chromosome.

### Tissue Profiling

The nucleic acids of the present invention can be used to determine the tissue type from which a given sample is derived. For example, a metastatic lesion is identified by its developmental organ or tissue source by identifying the expression of a particular marker of that organ or tissue. If a nucleic acid is expressed only in a specific tissue type, and a metastatic lesion is found to express that nucleic acid, then the developmental source of the lesion has been identified. Expression of a particular nucleic acid is assayed by detection of either the corresponding mRNA or the protein product. Immunological methods, such as antibody staining, are used to detect a particular protein product. Hybridization methods may be used to detect particular mRNA species, including but not limited to in situ hybridization and Northern blotting.

### Use of Polymorphisms

A nucleic acid will be useful in forensics, genetic analysis, mapping, and diagnostic applications if the corresponding region of a gene is polymorphic in the human population. A particular polymorphic form of the nucleic acid may be used to either identify a sample as deriving from a suspect or rule out the possibility that the sample derives from the suspect. Any means for detecting a polymorphism in a gene are used, including but not limited to electrophoresis of protein polymorphic variants, differential sensitivity to restriction enzyme cleavage, and hybridization to an allele-specific probe.

### B. Use of Nucleic Acids and Encoded Polypeptides to Raise Antibodies

Expression products of a nucleic acid, the corresponding mRNA or cDNA, or the corresponding complete gene are prepared and used for raising antibodies for experimental, diagnostic, and therapeutic purposes. For nucleic acids to which a corresponding gene has not been assigned, this provides an additional method of identifying the corresponding gene. The nucleic acid or related cDNA is expressed as described above, and antibodies are prepared. These antibodies are specific to an epitope on the encoded polypeptide, and can precipitate or bind to the corresponding native protein in a cell or tissue preparation or in a cell-free extract of an in vitro expression system.

Immunogens for raising antibodies are prepared by mixing the polypeptides encoded by the nucleic acids of the present invention with adjuvants. Alternatively, polypeptides are made as fusion proteins to larger immunogenic proteins. Polypeptides are also covalently linked to other larger immunogenic proteins, such as keyhole limpet hemocyanin. Immunogens are typically administered intradermally, subcutaneously, or intramuscularly. Immunogens are administered to experimental animals such as rabbits, sheep, and mice, to generate antibodies. Optionally, the animal spleen cells are isolated and fused with myeloma cells to form hybridomas which secrete monoclonal antibodies. Such methods are well known in the art. According to another method known in the art, the nucleic acid is administered directly, such as by intramuscular injection, and expressed in vivo. The expressed protein generates a variety of protein-specific immune responses, including production of antibodies, comparable to administration of the protein.

Preparations of polyclonal and monoclonal antibodies specific for nucleic acid-encoded proteins and polypeptides are made using standard methods known in the art. The antibodies specifically bind to epitopes present in the polypeptides of SEQ ID Nos. 17-24. Typically, at least about 6, 8, 10, or 12 contiguous amino acids are required to form an epitope. However, epitopes which involve noncontiguous amino acids may require more, for example, at least about 15, 25, or 50 amino acids. A short sequence of a nucleic acid may then be unsuitable for use as an epitope to raise antibodies for identifying the corresponding novel protein, because of the potential for cross-reactivity with a known protein. However, the antibodies may be useful for other purposes, particularly if they identify common structural features of a known protein and a novel polypeptide encoded by a nucleic acid of the invention.

Antibodies that specifically bind to human nucleic acid-encoded polypeptides should provide a detection signal at least about 5-, 10-, or 20- fold higher than a detection signal provided with other proteins when used in Western blots or other immunochemical assays. Preferably, antibodies that specifically bind nucleic acid T-encoded polypeptides do not detect other proteins in immunochemical assays and can immunoprecipitate nucleic acid-encoded proteins from solution.

To test for the presence of serum antibodies to the nucleic acid-encoded polypeptide in a human population, human antibodies are purified by methods well known in the art. Preferably, the antibodies are affinity purified by passing antiserum over a column to which a nucleic acid-encoded protein, polypeptide, or fusion protein is bound. The bound antibodies can then be eluted from the column, for example using a buffer with a high salt concentration.

In addition to the antibodies discussed above, genetically engineered antibody derivatives are made, such as single chain antibodies.

Antibodies may be made by using standard protocols known in the art (See, for example, Antibodies: A Laboratory Manual ed. by Harlow and Lane (Cold Spring Harbor Press: 1988)). A mammal, such as a mouse, hamster, or rabbit can be immunized with an immunogenic form of the peptide (e.g., a mammalian polypeptide or an antigenic fragment which is capable of eliciting an antibody response, or a fusion protein as described above).

In one aspect, this invention includes monoclonal antibodies that show a subject polypeptide is highly expressed in colorectal tissue or tumor tissue, especially colon cancer tissue or colon cancer-derived cell lines. Therefore, in one embodiment, this invention provides a diagnostic tool for the analysis of expression of a subject polypeptide in general, and in particular, as a diagnostic for colon cancer.

Techniques for conferring immunogenicity on a protein or peptide include conjugation to carriers or other techniques well known in the art. An immunogenic portion of a protein can be administered in the presence of adjuvant. The progress of immunization can be monitored by detection of antibody titers in plasma or serum. Standard ELISA or other immunoassays can be used with the immunogen as antigen to assess the levels of antibodies. In a preferred embodiment, the subject antibodies are immunospecific for antigenic determinants of a protein of a mammal, e.g., antigenic determinants of a protein of SEQ ID Nos. 17-24 or closely related homologs (e.g., at least 90% identical, and more preferably at least 95% identical).

Following immunization of an animal with an antigenic preparation of a polypeptide, antisera can be obtained and, if desired, polyclonal antibodies isolated from the serum. To produce monoclonal antibodies, antibody-producing cells (lymphocytes) can be harvested from an immunized animal and fused by standard somatic cell fusion procedures with immortalizing cells such as myeloma cells to yield hybridoma cells. Such techniques are well known in the art, and include, for example, the hybridoma technique (originally developed by Kohler and Milstein, (1975) Nature, 256: 495-497), the human B cell hybridoma technique *(Kozbar et al*., (1983) Immunology Today, 4: 72), and the EBV-hybridoma technique to produce human monoclonal antibodies (Cole et al., (1985) Monoclonal Antibodies and Cancer Therapy, Alan R. Liss, Inc. pp. 77-96). Hybridoma cells can be screened immunochemically for production of antibodies specifically reactive with a polypeptide of the present invention and monoclonal antibodies isolated from a culture comprising such hybridoma cells.

The term antibody as used herein is intended to include fragments thereof which are also specifically reactive with one of the subject polypeptides. Antibodies can be fragmented using conventional techniques and the fragments screened for utility in the same manner as described above for whole antibodies. For example, F(ab)₂ fragments can be generated by treating antibody with pepsin. The resulting F(ab)₂ fragment can be treated to reduce disulfide bridges to produce Fab fragments. The antibody of the present invention is further intended to include bispecific, single-chain, and chimeric and humanized molecules having affinity for a polypeptide conferred by at least one CDR region of the antibody. In preferred embodiments, the antibodies, the antibody further comprises a label attached thereto and able to be detected, (e.g., the label can be a radioisotope, fluorescent compound, chemiluminescent compound, enzyme, or enzyme co-factor).

Antibodies can be used, e.g., to monitor protein levels in an individual for determining, e.g., whether a subject has a disease or condition, such as colon cancer, associated with an aberrant protein level, or allowing determination of the efficacy of a given treatment regimen for an individual afflicted with such a disorder. The level of polypeptides may be measured from cells in bodily fluid, such as in blood samples.

Another application of antibodies of the present invention is in the immunological screening of cDNA libraries constructed in expression vectors such as gtl1, gtl8-23, ZAP, and ORF8. Messenger libraries of this type, having coding sequences inserted in the correct reading frame and orientation, can produce fusion proteins. For instance, gtl 1 will produce fusion proteins whose amino termini consist of 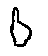 -galactosidase amino acid sequences and whose carboxyl termini consist of a foreign polypeptide. Antigenic epitopes of a protein, e.g., other orthologs of a particular protein or other paralogs from the same species, can then be detected with antibodies, as, for example, reacting nitrocellulose filters lifted from infected plates with antibodies. Positive phage detected by this assay can then be isolated from the infected plate. Thus, the presence of homologs can be detected and cloned from other animals, as can alternate isoforms (including splicing variants) from humans.

In another embodiment, a panel of monoclonal antibodies may be used, wherein each of the epitope's involved functions are represented by a monoclonal antibody. Loss or perturbation of binding of a monoclonal antibody in the panel would be indicative of a mutational attention of the protein and thus of the corresponding gene.

### C. Differential Expression

The present invention also provides a method to identify abnormal or diseased tissue in a human. For nucleic acids corresponding to profiles of protein families as described above, the choice of tissue may be dictated by the putative biological function. The expression of a gene corresponding to a specific nucleic acid is compared between a first tissue that is suspected of being diseased and a second, normal tissue of the human. The normal tissue is any tissue of the human, especially those that express the target gene including, but not limited to, brain, thymus, testis, heart, prostate, placenta, spleen, small intestine, skeletal muscle, pancreas, and the mucosal lining of the colon.

The tissue suspected of being abnormal or diseased can be derived from a different tissue type of the human, but preferably it is derived from the same tissue type; for example an intestinal polyp or other abnormal growth should be compared with normal intestinal tissue. A difference between the target gene, mRNA, or protein in the two tissues which are compared, for example in molecular weight, amino acid or nucleotide sequence, or relative abundance, indicates a change in the gene, or a gene which regulates it, in the tissue of the human that was suspected of being diseased.

The target genes in the two tissues are compared by any means known in the art. For example, the two genes are sequenced, and the sequence of the gene in the tissue suspected of being diseased is compared with the gene sequence in the normal tissue. The target genes, or portions thereof, in the two tissues are amplified, for example using nucleotide primers based on the nucleotide sequence shown in the Sequence Listing, using the polymerase chain reaction. The amplified genes or portions of genes are hybridized to nucleotide probes selected from a corresponding nucleotide sequence shown SEQ ID No. 1-8 or 9-16. A difference in the nucleotide sequence of the target gene in the tissue suspected of being diseased compared with the normal nucleotide sequence suggests a role of the nucleic acid-encoded proteins in the disease, and provides a lead for preparing a therapeutic agent.

The nucleotide probes are labeled by a variety of methods, such as radiolabeling, biotinylation, or labeling with fluorescent or chemiluminescent tags, and detected by standard methods known in the art.

Alternatively, target mRNA in the two tissues is compared. PolyA⁺RNA is isolated from the two tissues as is known in the art. For example, one of skill in the art can readily determine differences in the size or amount of target mRNA transcripts between the two tissues using Northern blots and nucleotide probes selected from the nucleotide sequence shown in the Sequence Listing. Increased or decreased expression of a target mRNA in a tissue sample suspected of being diseased, compared with the expression of the same target mRNA in a normal tissue, suggests that the expressed protein has a role in the disease, and also provides a lead for preparing a therapeutic agent.

Any method for analyzing proteins is used to compare two nucleic acid-encoded proteins from matched samples. The sizes of the proteins in the two tissues are compared, for example, using antibodies of the present invention to detect nucleic acid-encoded proteins in Western blots of protein extracts from the two tissues. Other changes, such as expression levels and subcellular localization, can also be detected immunologically, using antibodies to the corresponding protein. A higher or lower level of nucleic acid-encoded protein expression in a tissue suspected of being diseased, compared with the same nucleic acid-encoded protein expression level in a normal tissue, is indicative that the expressed protein has a role in the disease, and provides another lead for preparing a therapeutic agent.

Similarly, comparison of gene sequences or of gene expression products, e.g., mRNA and protein, between a human tissue that is suspected of being diseased and a normal tissue of a human, are used to follow disease progression or remission in the human. Such comparisons of genes, mRNA, or protein are made as described above.

For example, increased or decreased expression of the target gene in the tissue suspected of being neoplastic can indicate the presence of neoplastic cells in the tissue. The degree of increased expression of the target gene in the neoplastic tissue relative to expression of the gene in normal tissue, or differences in the amount of increased expression of the target gene in the neoplastic tissue over time, is used to assess the progression of the neoplasia in that tissue or to monitor the response of the neoplastic tissue to a therapeutic protocol over time.

The expression pattern of any two cell types can be compared, such as low and high metastatic tumor cell lines, or cells from tissue which have and have not been exposed to a therapeutic agent. A genetic predisposition to disease in a human is detected by comparing an target gene, mRNA, or protein in a fetal tissue with a normal target gene, mRNA, or protein. Fetal tissues that are used for this purpose include, but are not limited to, amniotic fluid, chorionic villi, blood, and the blastomere of an in vitro-fertilized embryo. The comparable normal target gene is obtained from any tissue. The mRNA or protein is obtained from a normal tissue of a human in which the target gene is expressed. Differences such as alterations in the nucleotide sequence or size of the fetal target gene or mRNA, or alterations in the molecular weight, amino acid sequence, or relative abundance of fetal target protein, can indicate a germline mutation in the target gene of the fetus, which indicates a genetic predisposition to disease.

### D. Use of Nucleic Acids, and Encoded Polypeptides to Screen for Peptide Analogs and Antagonists

Polypeptides of SEQ ID Nos. 17-24 or polypeptides encoded by the instant nucleic acids, e.g., SEQ ID Nos. 9-16, or a sequence complementary thereto, and corresponding full length genes can be used to screen peptide libraries to identify binding partners, such as receptors, from among the encoded polypeptides.

A library of peptides may be synthesized following the methods disclosed in U.S. Pat. No. 5,010,175, and in PCT WO 91/17823. As described below in brief, one prepares a mixture of peptides, which is then screened to identify the peptides exhibiting the desired signal transduction and receptor binding activity. In the '175 method, a suitable peptide synthesis support (e.g., a resin) is coupled to a mixture of appropriately protected, activated amino acids. The concentration of each amino acid in the reaction mixture is balanced or adjusted in inverse proportion to its coupling reaction rate so that the product is an equimolar mixture of amino acids coupled to the starting resin. The bound amino acids are then deprotected, and reacted with another balanced amino acid mixture to form an equimolar mixture of all possible dipeptides. This process is repeated until a mixture of peptides of the desired length (e.g., hexamers) is formed. Note that one need not include all amino acids in each step: one may include only one or two amino acids in some steps (e.g., where it is known that a particular amino acid is essential in a given position), thus reducing the complexity of the mixture. After the synthesis of the peptide library is completed, the mixture of peptides is screened for binding to the selected polypeptide. The peptides are then tested for their ability to inhibit or enhance activity. Peptides exhibiting the desired activity are then isolated and sequenced.

The method described in WO 91/17823 is similar. However, instead of reacting the synthesis resin with a mixture of activated amino acids, the resin is divided into twenty equal portions (or into a number of portions corresponding to the number of different amino acids to be added in that step), and each amino acid is coupled individually to its portion of resin. The resin portions are then combined, mixed, and again divided into a number of equal portions for reaction with the second amino acid. In this manner, each reaction may be easily driven to completion. Additionally, one may maintain separate "subpools" by treating portions in parallel, rather than combining all resins at each step. This simplifies the process of determining which peptides are responsible for any observed receptor binding or signal transduction activity.

In such cases, the subpools containing, *e*.*g*., 1-2,000 candidates each are exposed to one or more polypeptides of the invention. Each subpool that produces a positive result is then resynthesized as a group of smaller subpools (sub-subpools) containing, e.g., 20-100 candidates, and reassayed. Positive sub-subpools may be resynthesized as individual compounds, and assayed finally to determine the peptides that exhibit a high binding constant. These peptides can be tested for their ability to inhibit or enhance the native activity. The methods described in WO 91/7823 and U.S. Patent No. 5,194,392 (herein incorporated by reference) enable the preparation of such pools and subpools by automated techniques in parallel, such that all synthesis and resynthesis may be performed in a matter of days.

Peptide agonists or antagonists are screened using any available method, such as signal transduction, antibody binding, receptor binding, mitogenic assays, chemotaxis assays, etc. The methods described herein are presently preferred. The assay conditions ideally should resemble the conditions under which the native activity is exhibited *in vivo,* that is, under physiologic pH, temperature, and ionic strength. Suitable agonists or antagonists will exhibit strong inhibition or enhancement of the native activity at concentrations that do not cause toxic side effects in the subject. Agonists or antagonists that compete for binding to the native polypeptide may require concentrations equal to or greater than the native concentration, while inhibitors capable of binding irreversibly to the polypeptide may be added in concentrations on the order of the native concentration.

The end results of such screening and experimentation will be at least one novel polypeptide binding partner, such as a receptor, encoded by a nucleic acid of the invention, and at least one peptide agonist or antagonist of the novel binding partner. Such agonists and antagonists can be used to modulate, enhance, or inhibit receptor function in cells to which the receptor is native, or in cells that possess the receptor as a result of genetic engineering. Further, if the novel receptor shares biologically important characteristics with a known receptor, information about agonist/antagonist binding may help in developing improved agonists/antagonists of the known receptor.

### E. Pharmaceutical Compositions and Therapeutic Uses

Pharmaceutical compositions can comprise polypeptides, antibodies, or polynucleotides of the claimed invention. The pharmaceutical compositions will comprise a therapeutically effective amount of either polypeptides, antibodies, or polynucleotides of the claimed invention.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms, such as decreased body temperature. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician.

For purposes of the present invention, an effective dose will be from about 0.01 mg/kg to 50 mg/kg or 0.05 mg/kg to about 10 mg/kg of the DNA constructs in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in *Remington's Pharmaceutical Sciences* (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

### Delivery Methods

Once formulated, the nucleic acid compositions of the invention can be (I) administered directly to the subject; (2) delivered ex vivo, to cells derived from the subject or (3) delivered in vitro for expression of recombinant proteins.

Direct delivery of the compositions will generally be accomplished by injection, either subcutaneously, intraperitoneally, intravenously or intramuscularly, or delivered to the interstitial space of a tissue. The compositions can also be administered into a tumor or lesion. Other modes of administration include oral and pulmonary administration, suppositories, and transdermal applications, needles, and gene guns or hyposprays. Dosage treatment may be a single dose schedule or a multiple dose schedule.

Methods for the ex vivo delivery and reimplantation of transformed cells into a subject are known in the art and described in e.g., International Publication No. WO 93/14778. Examples of cells useful in ex vivo applications include, for example, stem cells, particularly hematopoetic, lymph cells, macrophages, dendritic cells, or tumor cells.

Generally, delivery of nucleic acids for both ex vivo and in vitro applications can be accomplished by, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

Once a subject gene has been found to correlate with a proliferative disorder, such as neoplasia, dysplasia, and hyperplasia, the disorder may be amenable to treatment by administration of a therapeutic agent based on the nucleic acid or corresponding polypeptide.

Preparation of antisense polypeptides is discussed above. Neoplasias that are treated with the antisense composition include, but are not limited to, cervical cancers, melanomas, colorectal adenocarcinomas, Wilms' tumor, retinoblastoma, sarcomas, myosarcomas, lung carcinomas, leukemias, such as chronic myelogenous leukemia, promyelocytic leukemia, monocytic leukemia, and myeloid leukemia, and lymphomas, such as histiocytic lymphoma. Proliferative disorders that are treated with the therapeutic composition include disorders such as anhydric hereditary ectodermal dysplasia, congenital alveolar dysplasia, epithelial dysplasia of the cervix, fibrous dysplasia of bone, and mammary dysplasia. Hyperplasias, for example, endometrial, adrenal, breast, prostate, or thyroid hyperplasias or pseudoepitheliomatous hyperplasia of the skin, are treated with antisense therapeutic compositions. Even in disorders in which mutations in the corresponding gene are not implicated, downregulation or inhibition of nucleic acid-related gene expression can have therapeutic application. For example, decreasing nucleic acid-related gene expression can help to suppress tumors in which enhanced expression of the gene is implicated.

Both the dose of the antisense composition and the means of administration are determined based on the specific qualities of the therapeutic composition, the condition, age, and weight of the patient, the progression of the disease, and other relevant factors. Administration of the therapeutic antisense agents of the invention includes local or systemic administration, including injection, oral administration, particle gun or catheterized administration, and topical administration. Preferably, the therapeutic antisense composition contains an expression construct comprising a promoter and a polynucleotide segment of at least about 12, 22, 25, 30, or 35 contiguous nucleotides of the antisense strand of a nucleic acid. Within the expression construct, the polynucleotide segment is located downstream from the promoter, and transcription of the polynucleotide segment initiates at the promoter.

Various methods are used to administer the therapeutic composition directly to a specific site in the body. For example, a small metastatic lesion is located and the therapeutic composition injected several times in several different locations within the body of tumor. Alternatively, arteries which serve a tumor are identified, and the therapeutic composition injected into such an artery, in order to deliver the composition directly into the tumor. A tumor that has a necrotic center is aspirated and the composition injected directly into the now empty center of the tumor. The antisense composition is directly administered to the surface of the tumor, for example, by topical application of the composition. X-ray imaging is used to assist in certain of the above delivery methods.

Receptor-mediated targeted delivery of therapeutic compositions containing an antisense polynucleotide, subgenomic polynucleotides, or antibodies to specific tissues is also used. Receptor-mediated DNA delivery techniques are described in, for example, Findeis *et al*., *Trends in Biotechnol*. (1993) *11:202-205;* Chiou *et al*., (1994) Gene Therapeutics: Methods And Applications Of Direct Gene Transfer (J.A. Wolff, ed.); Wu & Wu, *J*. *Biol*. *Chem*. (1988) *263:621-24;* Wu *et al*., *J*. *Biol. Chem*. (1994) *269:542-46;* Zenke *et al*., *Proc. Nail*. *Acad. Sci. (USA)* (1990) *87:3655-59;* Wu *et al*., *J. Biol. Chem.* (1991) *266*:338-42. Preferably, receptor-mediated targeted delivery of therapeutic compositions containing antibodies of the invention is used to deliver the antibodies to specific tissue.

Therapeutic compositions containing antisense subgenomic polynucleotides are administered in a range of about 100 ng to about 200 mg of DNA for local administration in a gene therapy protocol. Concentration ranges of about 500 ng to about 50 mg, about I mg to about 2 mg, about 5 mg to about 500 mg, and about 20 mg to about 100 mg of DNA can also be used during a gene therapy protocol. Factors such as method of action and efficacy of transformation and expression are considerations which will affect the dosage required for ultimate efficacy of the antisense subgenomic nucleic acids. Where greater expression is desired over a larger area of tissue, larger amounts of antisense subgenomic nucleic acids or the same amounts readministered in a successive protocol of administrations, or several administrations to different adjacent or close tissue portions of, for example, a tumor site, may be required to effect a positive therapeutic outcome. In all cases, routine experimentation in clinical trials will determine specific ranges for optimal therapeutic effect. A more complete description of gene therapy vectors, especially retroviral vectors, is contained in U.S. Serial No. 08/869,309, which is expressly incorporated herein, and in section F below.

For genes encoding polypeptides or proteins with anti-inflammatory activity, suitable use, doses, and administration are described in U.S. Patent No. 5,654,173, incorporated herein by reference. Therapeutic agents also include antibodies to proteins and polypeptides encoded by the subject nucleic acids, as described in U.S. Patent No. 5,654,173.

### F. Gene Therapy

The therapeutic nucleic acids of the present invention may be utilized in gene delivery vehicles. The gene delivery vehicle may be of viral or non-viral origin (see generally, Jolly, *Cancer Gene Therapy* (1994) *1:*51-64; Kimura, *Human Gene Therapy* (1994) *5:845-852;* Connelly, *Human Gene Therapy (1995) 1:*185-193; and Kaplitt, *Nature Genetics* (1994) *6*:148-153). Gene therapy vehicles for delivery of constructs including a coding sequence of a therapeutic of the invention can be administered either locally or systemically. These constructs can utilize viral or non-viral vector approaches. Expression of such coding sequences can be induced using endogenous mammalian or heterologous promoters. Expression of the coding sequence can be either constitutive or regulated.

The present invention can employ recombinant retroviruses which are constructed to carry or express a selected nucleic acid molecule of interest. Retrovirus vectors that can be employed include those described in EP 0415 731; WO 90/07936; WO 94/03622; WO 93/25698; WO 93/25234; U.S. Patent No. 5,219,740; WO 93/1 1230; WO 93/10218; Vile and Hart, *Cancer Res.* (1993) *53*:3860-3864; Vile and Hart, *Cancer Res.* (1993) 53:962-967; Ram et al., *Cancer Res.* (1993) 53:83-88; Takamiya et el., J. *Neurosci. Re.s.* (1992) *33:493-503;* Baba et al., *J*. *Neurosurg.* (1993) *79*:729-735; U.S. Patent No. 4,777,127; GB Patent No. 2,200,651; and EP 0 345 242. Preferred recombinant retroviruses include those described in WO 91/02805.

Packaging cell lines suitable for use with the above-described retroviral vector constructs may be readily prepared (see PCT publications WO 95/30763 and WO 92/05266), and used to create producer cell lines (also termed vector cell lines) for the production of recombinant vector particles. Within particularly preferred embodiments of the invention, packaging cell lines are made from human (such as HT1O8O cells) or mink parent cell lines, thereby allowing production of recombinant retroviruses that can survive inactivation in human serum.

The present invention also employs alphavirus-based vectors that can function as gene delivery vehicles. Such vectors can be constructed from a wide variety of alphaviruses, including, for example, Sindbis virus vectors, Semliki forest virus (ATCC VR-67; ATCC VR-1247), Ross River virus (ATCC VR-373; ATCC VR1246) and Venezuelan equine encephalitis virus (ATCC VR-923; ATCC VR-1250; ATCC VR 1249; ATCC VR-532). Representative examples of such vector systems include those described in U.S. Patent Nos. 5,091,309; 5,217,879; and 5,185,440; and PCT Publication Nos. WO 92/10578; WO 94/21792; WO 95/27069; WO 95/27044; and WO 95/07994.

Gene delivery vehicles of the present invention can also employ parvovirus such as adeno-associated virus (AAV) vectors. Representative examples include the AAV vectors disclosed by Srivastava in WO 93/09239, Samulski et al., *J*. *Vir*. (1989) 63:3822-3828; Mendelson et *al*., *Virol*. (1988) *166:154-165;* and Flotte et al., *PNAS* (1993) *90:10613-10617*.

Representative examples of adenoviral vectors include those described by Berkner, *Biotechniques* (1988) 6:616-627; Rosenfeld et al., *Science* (1991) *252*:431-434; WO 93/19191; Kolls et al., *PNAS* (1994) 91:215-219; Kass-Eisler et al., *PNAS* (1993) *90*:11498-11502; Guzman et al., *Circulation* (1993) 88:2838-2848; Guzman et at, Cir. Res. (1993) 73:1202-1207; Zabner et al., *Cell* (1993) 75:207-216; Li Ct et al., *Hum. Gene Ther.* (1993) *4*:403-409; Cailaud et al., Eur. J Neurosci. (1993) 5:1287-1291; Vincent et al., *Nat*. *Genet*. (1993) *5*:130-134; Jaffe et al., Nat. Genet. (1992) 1:372-378; and Levrero et al., *Gene* (1991) *101:195-202.* Exemplary adenoviral gene therapy vectors employable in this invention also include those described in WO 94/12649, WO 93/03769; WO 93/19191; WO 94/28938; WO 95/11984 and WO 95/00655. Administration of DNA linked to killed adenovirus as described in Curiel, Hum. Gene Ther. (1992) 3:147-154 may be employed.

Other gene delivery vehicles and methods may be employed, including polycationic condensed DNA linked or unlinked to killed adenovirus alone, for example Curiel, *Hum. Gene Ther. (1992)3:147-154;* ligand linked DNA, for example see Wu, *J*. *Biol*. *Chem*. (1989) *264*:16985-16987; eukaryotic cell delivery vehicles cells, for example see U.S. Serial No. 08/240,030, filed May 9, 1994, and U.S. Serial No. 08/404,796; deposition of photopolymerized hydrogel materials; hand-held gene transfer particle gun, as described in U.S. Patent No. 5,149,655; ionizing radiation as described in U.S. Patent No. 5,206,152 and in W092111033; nucleic charge neutralization or fusion with cell membranes. Additional approaches are described in Philip, *Mol*. *Cell Biol*. (1994) 14:2411-2418, and in Woffendin, *Proc. Natl. Acad. Sci.* (1994) *91*:1581-1585.

Naked DNA may also be employed. Exemplary naked DNA introduction methods are described in WO 90111092 and U.S. Patent No. 5,580,859. Uptake efficiency may be improved using biodegradable latex beads. DNA coated latex beads are efficiently transported into cells after endocytosis initiation by the beads. The method may be improved further by treatment of the beads to increase hydrophobicity and thereby facilitate disruption of the endosome and release of the DNA into the cytoplasm. Liposomes that can act as gene delivery vehicles are described in U.S. Patent No. 5,422,120, PCT Nos. WO 95/13796, WO 94/23697, and WO 91/14445, and EP No. 0524 968.

Further non-viral delivery suitable for use includes mechanical delivery systems such as the approach described in Woffendin *et al*., *Proc. Natl*. *Acad. Sci. USA* (1994) *91*(24):11581-11585. Moreover, the coding sequence and the product of expression of such can be delivered through deposition of photopolymerized hydrogel materials. Other conventional methods for gene delivery that can be used for delivery of the coding sequence include, for example, use of hand-held gene transfer particle gun, as described in U.S. Patent No. 5,149,655; use of ionizing radiation for activating transferred gene, as described in U.S. Patent No. 5,206,152 and PCT No. WO 92/11033.

### G. Transgenic Animals

One aspect of the present invention relates to transgenic non-human animals having germline and/or somatic cells in which the biological activity of one or more genes are altered by a chromosomally incorporated transgene.

In a preferred embodiments, the transgene encodes a mutant protein, such as dominant negative protein which antagonizes at least a portion of the biological function of a wild-type protein.

Yet another preferred transgenic animal includes a transgene encoding an antisense transcript which, when transcribed from the transgene, hybridizes with a gene or a mRNA transcript thereof, and inhibits expression of the gene.

In one embodiment, the present invention provides a desired non-human animal or an animal (including human) cell which contains a predefined, specific and desired alteration rendering the non-human animal or animal cell predisposed to cancer. Specifically, the invention pertains to a genetically altered non-human animal (most preferably, a mouse), or a cell (either non-human animal or human) in culture, that is defective in at least one of two alleles of a tumor-suppressor gene. The inactivation of at least one of these tumor suppressor alleles results in an animal with a higher susceptibility to tumor induction or other proliferative or differentiative disorders, or disorders marked by aberrant signal transduction, e.g., from a cytokine or growth factor. A genetically altered mouse of this type is able to serve as a useful model for hereditary cancers and as a test animal for carcinogen studies. The invention additionally pertains to the use of such non-human animals or animal cells, and their progeny in research and medicine.

Furthermore, it is contemplated that cells of the transgenic animals of the present invention can include other transgenes, e.g., which alter the biological activity of a second tumor suppressor gene or an oncogene. For instance, the second transgene can functionally disrupt the biological activity of a second tumor suppressor gene, such as p53, p73, DCC, p21^{cip1}, p27^{kip1}, Rb, Mad or E2F. Alternatively, the second transgene can cause overexpression or loss of regulation of an oncogene, such as ras, myc, a cdc25 phosphatase, Bcl-2, Bcl-6, a transforming growth factor, neu, int-3, polyoma virus middle T antigen, SV4O large T antigen, a papillomaviral E6 protein, a papillomaviral E7 protein, CDK4, or cyclin Dl.

A preferred transgenic non-human animal of the present invention has germline and/or somatic cells in which one or more alleles of a gene are disrupted by a chromosomally incorporated transgene, wherein the transgene includes a marker sequence providing a detectable signal for identifying the presence of the transgene in cells of the transgenic animal, and replaces at least a portion of the gene or is inserted into the gene or disrupts expression of a wild-type protein.

Still another aspect of the present invention relates to methods for generating non-human animals and stem cells having a functionally disrupted endogenous gene. In a preferred embodiment, the method comprises the steps of:
(i) constructing a transgene construct including (a) a recombination region having at least a portion of the gene, which recombination region directs recombination of the transgene with the gene, and (b) a marker sequence which provides a detectable signal for identifying the presence of the transgene in a cell;
(ii) transferring the transgene into stem cells of a non-human animal;
(iii) selecting stem cells having a correctly targeted homologous recombination between the transgene and the gene;
(iv) transferring cells identified in step (iii) into a non-human blastocyst and implanting the resulting chimeric blastocyst into a non-human female; and
(v) collecting offspring harboring an endogenous gene allele having the correctly targeted recombination.

Yet another aspect of the invention provides a method for evaluating the carcinogenic potential of an agent by (i) contacting a transgenic animal of the present invention with a test agent, and (ii) comparing the number of transformed cells in a sample from the treated animal with the number of transformed cells in a sample from an untreated transgenic animal or transgenic animal treated with a control agent. The difference in the number of transformed cells in the treated animal, relative to the number of transformed cells in the absence of treatment with a control agent, indicates the carcinogenic potential of the test compound.

Another aspect of the invention provides a method of evaluating an anti-proliferative activity of a test compound. In preferred embodiments, the method includes contacting a transgenic animal of the present invention, or a sample of cells from such animal, with a test agent, and determining the number of transformed cells in a specimen from the transgenic animal or in the sample of cells. A statistically significant decrease in the number of transformed cells, relative to the number of transformed cells in the absence of the test agent, indicates the test compound is a potential anti-proliferative agent.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, *Molecular Cloning A Laboratory Manual*, 2nd Ed., ed. by Sambrook, Fritsch and Maniatis (Cold Spring Harbor Laboratory Press: 1989); *DNA Cloning,* Volumes I and II (D.N. Glover ed., 1985); *Oligonucleotide Synthesis* (M. J. Gait ed., 1984); Mullis *et al*. U.S. Patent No. *4,683,195; Nucleic Acid Hybridization* (B.D. Hames & S. J. Higgins eds. 1984); *Transcription And Translation* (B. D. Hames & S. J. Higgins eds. 1984); *Culture Of Animal Cells* (R. I. Freshney, Alan R. Liss, Inc., 1987); *Immobilized Cells And Enzymes* (IRL Press, 1986); B. Perbal, *A Practical Guide To Molecular Cloning* (1984); the treatise, *Methods in Enzymology* (Academic Press, Inc., N.Y.); *Gene Transfer Vectors For Mammalian Cells* (J. H. Miller and M.P. Calos eds., 1987, Cold Spring Harbor Laboratory); *Methods In Enzymology,* Vols. 154 and 155 (Wu et al. eds.), *Immunochemical Methods In Cell And Molecular Biology* (Mayer and Walker, eds., Academic Press, London, 1987); *Handbook Of Experimental Immunology,* Volumes I-IV (D. M. Weir and C.C. Blackwell, eds., 1986); *Manipulating the Mouse Embryo,* (Cold Spring Harbor Laboratory Press, Cold Spring Harbor, N.Y., 1986).

As mentioned above, the sequences described herein are believed to have particular utility in regards to colon cancer. However, they may also be useful with other types of cancers and other disease states.

The present invention will now be illustrated by reference to the following examples which set forth particularly advantageous embodiments. However, it should be noted that these embodiments are illustrative and are not to be construed as restricting the invention in any way.

### Examples

### A. Identification of differentially expressed sequences

### Description of the Libraries

SEQ ID Nos: 9-16 were derived from libraries designated as DE and PA as described below, and as described above, were truncated during the cloning process to produce the sequences of SEQ ID Nos 1-8. The DE library is a normalized, colon cancer specific, subtracted cDNA library. The DE library is specific for sequences expressed in colon cancer [proximal and distal Dukes' B, microsatellite stable (MSS)] but not expressed in normal tissues, including normal colon tissue. The PA library is a normalized, colon specific, subtracted cDNA library. The PA library is specific for sequences expressed in normal colon tissue but not expressed in other normal tissues.

### Construction of a colon cancer specific library

A subtracted colon cancer specific library was made by subtracting pooled proximal, stage B, MSS and distal, Stage B, MSS tumor tissue cDNA against a combination of pooled driver normal cDNA made from colon, peripheral blood leukocytes (PBL), liver, spleen, lung, kidney, heart, small intestine, skeletal muscle, and prostate tissue cDNAs. The following RNA samples were obtained from Origene Technologies, Inc., Rockville, Maryland, and were used to synthesize the pooled driver cDNA: #HT-1015 normal colon total RNA, #HT-1005 liver total RNA, #HT-1004 spleen total RNA, #HT-1009 lung total RNA, #HT-1003 kidney total RNA, #HT-1006 peripheral blood leukocyte total RNA, #HT-prostate total RNA, #HM-1002 heart muscle poly A+ RNA, #HM-1007 intestine poly A+ RNA, and #HM-1 008 skeletal muscle poly A+ RNA. First-strand cDNA was prepared for each using 1 microgram of RNA. A biased pool of first-strand cDNA was prepared containing 50% normal colon first-strand cDNA reaction and 5.56% of each of the remaining tissue first-strand cDNA reactions by volume. Eight individual amplification reactions, each containing 1 microliter of the biased first-strand cDNA reaction pool, were performed for 18 cycles. The double stranded cDNA product from all eight amplification reactions were pooled and purified for subsequent use in subtractive hybridization.

Briefly, double-stranded cDNA was generated using the Clontech SMART PCR cDNA Synthesis Kit (purchased from Clontech Laboratories Inc., Palo Alto, Calif.) following the manufacturer's instructions. Subtraction hybridization steps were performed in accordance with the manufacturer's instructions for the Clontech PCR-Select kit (purchased from Clontech Laboratories Inc, Palo Alto, Calif., and described in, for example, Daitchenko et al., 1996 PNAS 93:6025-6030, Gurskaya et al., 1996 Analytical Biochemistry 240:90-97). A forty-five fold mass excess of driver cDNA (450 nanograms) was used for each subtraction experiment. Subtractive hybridization of tester with driver cDNAs was performed twice, each time for about 8-12 hours. Subtracted cancer specific DE cDNA was ligated into the pCR2.1-TOPO plasmid vector (Invitrogen Corporation, Carlsbad CA) and chemically transformed into ultracompetent Epicurian E. coli XL1O-Gold cells (Stratagene, La Jolla, CA). A reverse library was also constructed wherein the tester and driver samples were switched; this library was designated as MD.

### Construction of a normal colon specific library

This normal colon tissue specific library was made using Clontech Laboratories Inc PCR-Select kit, K1804-1, following instructions from the users manual (PT1117-1).

Four, 100 1, SMART PCR cDNA amplification reactions for each normal, non-cancerous, patient sample, were performed, starting with 1 1 from their respective first strand cDNA reactions. Each sample was amplified for only 18 cycles using the following PCR conditions; 95 C-b sec, 68 C 5 mm. using a 9600 Perkin Elmer instrument. The following are Bayer Diagnostic sample identification numbers for the cDNA samples that were amplified: NPB(-) 27347, NPB(-)27859, NPB(-)28 147, NPB(-)28 162, NDB(-)28800, NDB(-)29243, NDB(-)29244 and NDB(-)42472. These are normal colon tissue samples obtained from the same patients providing the proximal stage B MSSand distal stage B MSS cancer samples, which were used to prepare the DE library described above. Equal volumes of the eight normal colon cDNAs were pooled. A subtracted normal colon tissue specific library was made by subtracting the normal colon cDNA pool against a combination of pooled driver normal cDNA made from peripheral blood leukocytes (PBL), liver, spleen, lung, kidney, heart, small intestine, skeletal muscle, and prostate tissue cDNAs. The following are the RNA samples that were used to synthesize the pooled driver cDNA: #HT-1005 liver total RNA, #HT-b 004 spleen total RNA, #HT1009 lung total RNA, #HT-1003 kidney total RNA, #HT-1 006 peripheral blood leukocyte total RNA, #HT-prostate total RNA, #HM-1 002 heart muscle poly A+ RNA, #HM-1007 intestine poly A+ RNA, and #HM-b008 skeletal muscle poly A+ RNA. First-strand cDNA was prepared for each using 1 microgram of RNA. A pool of first strand cDNA reactions was then made consisting of equal volumes of the nine driver tissue first-strand cDNA reactions. Eight individual amplification reactions, each containing 1 microliter of the first- strand cDNA reaction pool, were performed for 18 cycles. The double stranded cDNA product from all eight amplification reactions was pooled and purified for subsequent use in subtractive hybridization. The normal colon tissue specific subtracted library was called PA and individual clones derived from this library were referred to with a number prefixed by PA.

The normalized subtracted PA normal colon specific cDNA library and a subtracted normal human tissue specific cDNA library, consisting of the human tissues listed above were generated according published procedures (Daitchenko et al., 1996 PNAS 93:6025-6030, Gurskaya et al., 1996 Analytical Biochemistry 240:90-97) using Clontech Laboratories, Inc., PCR-Select cDNA subtraction kit, P11117-1. Library construction and cloning were carried out as described above for the colon cancer specific library. Out of the 1152 clones that were analyzed for differential expression, approximately 69% were differentially expressed, as described in the co-pending application USSN: 09/385,982.

Each EST isolated from each of the above libraries represents a sequence from a partial mRNA transcript, since the cDNA used for making the subtracted library was restricted with *Rsa*I, a four base cutter restriction endonuclease that generates fragments with an average size of about 600 base pairs. That is, the SMART™ system (BD Biosciences) described above may be used to generate full length mRNA from total cellular RNA sample, which is then used to generate a full length cDNA library. Subsequently, the full length cDNA is digested with a restriction enzyme such as *RsaI* to generate short fragments useful for subractive hybridization (see PCR-Select™ manual; BD Biosciences Clontech). According to this method, the present invention has identified the sequences of SEQ ID Nos 1-8, which are the *RsaI* digested products of the full length sequences (SEQ ID Nos 9-16) as being differentially expressed in colon cancer.

### Validation of differential expression in colon cancer

To validate that the differentially expressed sequences found in this library were specific to colon cancer, the clones were screened with cDNAs prepared from a colon cancer specific library, Delaware (DE), and a normal tissue specific library Maryland (MD).

cDNA clones were analyzed for differential expression following the procedure developed by von Stein et al., 1997, Nucleic Acids Research 25(13):2598-2602 and using probes synthesized according to a published method (Jin et al., 1997, Biotechniques 23:1083-1086). Out of the 1248 clones that were analyzed for differential expression approximately 83% were differentially expressed, as described in the co-pending application USSN: 09/385,982.

### Sequencing and analysis of differentially expressed clones

The nucleotide sequence of the inserts from clones shown to be differentially expressed was determined by single-pass sequencing from either the T7 or M13 promoter sites using fluorescently labeled dideoxynucleotides via the Sanger sequencing method. Sequences were analyzed according to methods described in the text (XI., Examples; B. Results of Public Database Search).

Each nucleic acid represents sequence from at least a partial mRNA transcript. As described above, the method of the invention for the identification of differentially expressed sequences comprises a step in which the full length cDNA molecule is cleaved to a partial mRNA transcript which is, in turn, used to assess differential expression of the partial sequence.

The differentially expressed full length genes and partial sequences of the invention were assigned a sequence identification number (see figures). The nucleic - acid sequences are provided in the attached Sequence Listing.

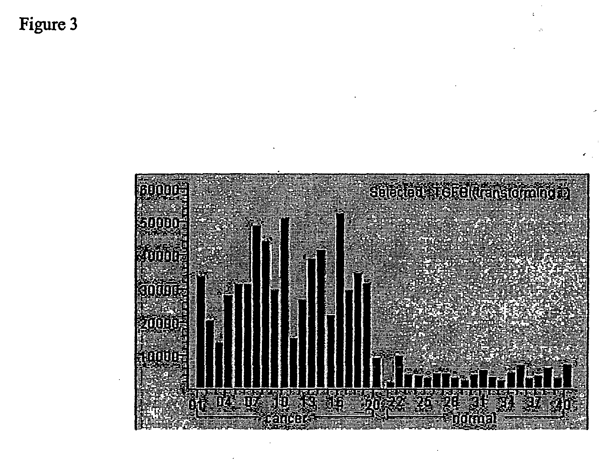

An example of the confirmation of a differentially-expressed sequence is shown in Figure 3. Differential expression of the sequence was determined by probing an Affymetrix human gene chip with RNA isolated from colon cancer tissue. Briefly, total RNA was extracted from twenty microdissected colon cancer tissue samples and twenty corresponding normal adjacent tissues. The RNA samples were labeled and processed on Affymetrix U133A chips according to the manufacturer's instructions. Raw signals are plotted on the y-axis; individual tissue samples are indicated on the x-axis; tumor and normal samples are grouped, as labeled. The raw signals for the probe set corresponding to TGFBi (transforming growth factor, beta-induced protein) are shown in the graph.

### B. Results of Public Database Searches

The full length cDNA sequence of SEQ ID Nos. 9-16 were obtained through a BLAST2 search of GenBank using the partial sequences of SEQ ID Nos 1-8 described herein and in the co-pending application USSN: 09/871,161.

A total of 8 sequences were analyzed. The sequences were first masked to identify vector-derived sequences, which were subsequently removed. The remaining sequence information was used to create the Sequence Listing (SEQ ID Nos. 1-8). Each of these sequences was used as the query sequence to perform a BLAST 2 search against the databases listed above. The BLAST 2 search differs from the traditional Blast search in that it allows for the introduction of gaps in order to produce an optimal alignment of two sequences. From the BLAST2 search, we identified the full length sequence (SEQ ID Nos 9-16) corresponding to each of the partial sequences (SEQ ID Nos 1-8), and which as been originally used, as described above, to obtain the partial sequences (SEQ ID Nos 1-8). The GenBank record of each full length sequence identified in the BLAST2 search was also utilized to obtain the amino acid sequence encoded by each cDNA.

Those skilled in the art will recognize, or be able to ascertain, using not more than routine experimentation, many equivalents to the specific embodiments of the invention described herein. Such specific embodiments and equivalents are intended to be encompassed by the following claims.

All patents, published patent applications, and publications cited herein are incorporated by reference as if set forth fully herein.

## Claims

1. A method of detecting colon cancer in an individual comprising:
(a) detecting the presence of a nucleic acid sequence consisting of a nucleic acid sequence selected from the group consisting of any one or more of SEQ ID Nos 9-16; or
(b) detecting the presence in said sample of a polypeptide sequence comprising a polypeptide sequence selected from the group consisting of any one or more of SEQ ID Nos 17-24; or
(c) detecting the presence of a nucleic acid sequence comprising a nucleic acid sequence selected from the group consisting of any one or more of SEQ ID Nos 1-8;
in a sample obtained from said individual.

2. The method of claim 1 wherein the nucleic acid sequence consists of a nucleic acid sequence selected from the group consisting of SEQ ID Nos 9-16.

3. The method of claim 1 wherein the polypeptide sequences comprises a polypeptide sequence selected from the group consisting of SEQ ID Nos 17-24.

4. The method of claim 1 wherein the nucleic acid sequence comprises a nucleic acid sequence selected from the group consisting of SEQ ID Nos 1-8.

5. The method of claim 1 or 3, wherein said step of detecting comprises:
(a) contacting said sample with a polypeptide ligand which is capable of binding to one or more of SEQ ID Nos 17-24; and
(b) detecting the binding of said polypeptide ligand to said one or more of SEQ ID Nos 17-24, wherein detection of binding is indicative or the presence of a polypeptide sequence comprising a polypeptide sequence selected from the group consisting of SEQ ID Nos 17-24 in said sample.

6. The method of claim 5, wherein said polypeptide ligand is an antibody.

7. The method of claim 5, wherein said polypeptide ligand comprises a detectable label.

8. The method of any one of the preceding claims, wherein said individual is a human.

9. The method of any one of claims 1, 2, 3 and 8, wherein said step of detecting comprises:
(a) contacting said sample with a polynucleotide probe comprising at least 12 contiguous nucleotides of a sequence selected from the group consisting of a sequence comprising SEQ ID Nos 9-16 or SEQ ID Nos 1-8 which is capable of hybridizing to a sequence selected from the group of a sequence comprising SEQ ID Nos 9-16 or SEQ ID Nos 1-8;
(b) detecting the hybridization of said polynucleotide probe to said sequence selected from the group of a sequence comprising SEQ ID Nos 9-16 or SEQ ID Nos 1-8, wherein detection of hybridization is indicative of the presence of a nucleic acid sequence comprising a nucleic acid sequence selected from the group consisting of SEQ ID Nos 9-16 or SEQ ID Nos 1-8.

10. The method of claim 9, wherein said probe comprises a detectable label.
